# EUROPEAN PATENT SPECIFICATION

(11) **EP 2 096 120 B1**
(45) Date of publication and mention of the grant of the patent: **14.03.2012**
(21) Application number: 09163270.3
(22) Date of filing: 18.02.2005
(51) Int. Cl.: C07K 14/705, A61K 38/17, A61K 38/55, A61K 38/43, G01N 33/50, A01K 67/027, A61P 1/18, A61P 3/04, A61P 3/10

(54) **Use of secreted protein products for preventing and treating pancreatic diseases and/or obesity and/or metabolic syndrome**
Verwendung sekretierter Proteinprodukte zur Prävention und Behandlung von Erkrankungen der Bauchspeicheldrüse und/oder Adipositas und/oder des metabolischen Syndroms
Utilisation de produits à protéines sécrétées pour empêcher et traiter les maladies pancréatiques et/ou l'obésité et/ou un syndrome métabolique

(30) Priority: 20.02.2004 EP 04003914
(43) Date of publication of application: 02.09.2009
(62) Divisional of application: 05701416.9
(73) Proprietor: DeveloGen Aktiengesellschaft, 37079 Göttingen (DE)
(72) Inventor: Onichtchouk, Daria, 79100, Freiburg (DE)
(74) Representative: Weiss, Wolfgang

(56) References cited:
- US-A1- 2003 077 720
- US-A1- 2003 194 766

### SEQUENCE LISTING

<110> DeveloGen Aktiengesellschaft für entwicklungsbiologische Forschung
<120> Use of secreted protein products for preventing and treating pancreatic diseases and/or obesity and/or metabolic syndrome
<130> 32600PWO
<140> PCT/EP2005/001711
   <141> 2005-02-18
<150> EP04003914.1
   <151> 2004-02-20
<160> 61
<170> PatentIn version 3.3
<210> 1
   <211> 19
   <212> DNA
   <213> Artificial
<220>
   <223> Artificial Sequence
<220>
   <221> misc_feature
   <223> Primer: Mouse SF02 forward primer
<400> 1
   cggacagcat cagccttga 19
<210> 2
   <211> 20
   <212> DNA
   <213> Artificial
<220>
   <223> Artificial Sequence
<220>
   <221> misc_feature
   <223> Primer: Mouse SF02 reverse primer
<400> 2
   ccgcgatgaa ggagatgaga 20
<210> 3
   <211> 19
   <212> DNA
   <213> Artificial
<220>
   <223> Artificial Sequence
<220>
   <221> misc_feature
   <223> Taqman probe: Mouse SF02 Taqman probe
<400> 3
   ctgcgcaaac ccgacggca 19
<210> 4
   <211> 19
   <212> DNA
   <213> Artificial
<220>
   <223> Artificial Sequence
<220>
   <221> misc_feature
   <223> Primer: Mouse SF03 forward primer
<400> 4
   gttgttcgcc atgccaaga 19
<210> 5
   <211> 22
   <212> DNA
   <213> Artificial
<220>
   <223> Artificial Sequence
<220>
   <221> misc_feature
   <223> Primer: Mouse SF03 reverse primer
<400> 5
   caaaagcttg tggagtcagg ct 22
<210> 6
   <211> 31
   <212> DNA
   <213> Artificial
<220>
   <223> Artificial Sequence
<220>
   <221> misc_feature
   <223> Taqman probe: Mouse SF03 Taqman probe
<400> 6
   acaccaacgc catgttcaag aataactacc c 31
<210> 7
   <211> 18
   <212> DNA
   <213> Artificial
<220>
   <223> Artificial Sequence
<220>
   <221> misc_feature
   <223> Primer: Mouse SF04 forward primer
<400> 7
   ggtacaacct ggcggtgg 18
<210> 8
   <211> 23
   <212> DNA
   <213> Artificial
<220>
   <223> Artificial Sequence
<220>
   <221> misc_feature
   <223> Primer: Mouse SF04 reverse primer
<400> 8
   gctcacgctc atcttgtcct taa 23
<210> 9
   <211> 18
   <212> DNA
   <213> Artificial
<220>
   <223> Artificial Sequence
<220>
   <221> misc_feature
   <223> Taqman probe: Mouse SF04 Taqman probe
<400> 9
   tgcccgtggc tgagccgc 18
<210> 10
   <211> 1285
   <212> DNA
   <213> Mus musculus
<220>
   <221> CDS
   <222> (514)..(1131)
   <223> SF01, cDNA: NM_026161, Protein: NP_080437
<400> 10
   ggtggtcccc actcgtcgcc ggctaaaccc ccgccacccc tggaggcccg gtcaagccgc     60
   agcgcgggcg accagtgtgt agcccggcgt cctcaccgag caggatagcc ggctgggacc    120
   gaagccgacc cgcccgccac cagccaggtg ccatgctgct gctcttgctg ggcttcctag    180
   gcccggcggc ctgctgggca ctgggcccgg ctggccctgg ctcctcggag ctgcggtcag    240
   ccttctcggc ggctcgcacc accccgctgg agggcacgtc ggagatggcg gtgaccttcg    300
   acaaggtgta cgtgaacatc gggggtgact tcgacgcagc caccgggcgg ttccgctgtc    360
   gcgtgccggg cgcctacttc ttctccttca cggccggcaa ggcccgcaca agagcctgtc    420
   ggtgatgctg gtgcgcaacc gcgacgaggt gcaggcgctg gctttcgacg agcagcgacg    480
   gccaggcgcg cggcgcgcgc cagccagagc gcc atg ctg cag ctc gac tac ggc     534
                                        Met Leu Gln Leu Asp Tyr Gly
                                        1               5
   gac acg gtg tgg ctg cgg ctg cac ggc gct ccg cag tac gcg ctc ggc      582
   Asp Thr Val Trp Leu Arg Leu His Gly Ala Pro Gln Tyr Ala Leu Gly
           10                  15                  20
   gcg ccg ggc gcc acc ttc agc ggc tac ctg gtg tac gcg gac gcc gac      630
   Ala Pro Gly Ala Thr Phe Ser Gly Tyr Leu Val Tyr Ala Asp Ala Asp
       25                  30                  35
   gcc gac gcg cct gcg cgc ggc ccc gcg gcc ccg gag ccg cgc tcg gcc      678
   Ala Asp Ala Pro Ala Arg Gly Pro Ala Ala Pro Glu Pro Arg Ser Ala
   40                  45                  50                  55
   ttc tcc gcg gcg cgc acg cgc agc ctg gtg ggc tcg gac gcc gcc ccc      726
   Phe Ser Ala Ala Arg Thr Arg Ser Leu Val Gly Ser Asp Ala Ala Pro
                   60                  65                  70
   ggc ccg cgc cac cgg ccg ttg gcc ttc gac acc gag ctg gta aac ata      774
   Gly Pro Arg His Arg Pro Leu Ala Phe Asp Thr Glu Leu Val Asn Ile
               75                  80                  85
   ggt ggc gac ttc gac gcg gcg gcc ggc gtg ttc cgc tgc cgc ctg ccg      822
   Gly Gly Asp Phe Asp Ala Ala Ala Gly Val Phe Arg Cys Arg Leu Pro
           90                  95                  100
   gga gcc tat ttc ttc tcc ttc acg ctg ggc aag ctg ccg cgc aag acg      870
   Gly Ala Tyr Phe Phe Ser Phe Thr Leu Gly Lys Leu Pro Arg Lys Thr
       105                 110                 115
   ctg tcg gtg aag ctg atg aag aac cgc gac gag gtg cag gcc atg att      918
   Leu Ser Val Lys Leu Met Lys Asn Arg Asp Glu Val Gln Ala Met Ile
   120                 125                 130                 135
   tac gac gac ggc gct tcg agg cgc cgt gag atg cag agt cag agc gtg      966
   Tyr Asp Asp Gly Ala Ser Arg Arg Arg Glu Met Gln Ser Gln Ser Val
                   140                 145                 150
   agg ctg ccg ctg cgg cgc ggc gac gcc gtc tgg cta ctt agc cac gat     1014
   Arg Leu Pro Leu Arg Arg Gly Asp Ala Val Trp Leu Leu Ser His Asp
               155                 160                 165
   cac gat ggc tat ggc gcc tac agc aac cac ggc aag tac atc act ttc     1062
   His Asp Gly Tyr Gly Ala Tyr Ser Asn His Gly Lys Tyr Ile Thr Phe
           170                 175                 180
   tca ggc ttc ctg gtg tac cct gac ctc gcc gcc gcc ggc ccg ccg gcc     1110
   Ser Gly Phe Leu Val Tyr Pro Asp Leu Ala Ala Ala Gly Pro Pro Ala
       185                 190                 195
   ctc aag ccc cca gag ctc tga gcctctgctt ggaggagccc gggagagccg        1161
   Leu Lys Pro Pro Glu Leu
   200                 205
   tggggcatgc atgccgagcc gggaccgcgg cccgaacgcc ccaccggtcc gagcatgact   1221
   gcctgctcag cacgcctgga ctctgccaat aaagtggggc tgcctgtcag ccttatggtc   1281
   ctgc                                                                1285
<210> 11
   <211> 205
   <212> PRT
   <213> Mus musculus
<400> 11
   Met Leu Gln Leu Asp Tyr Gly Asp Thr Val Trp Leu Arg Leu His Gly
   1               5                   10                  15
   Ala Pro Gln Tyr Ala Leu Gly Ala Pro Gly Ala Thr Phe Ser Gly Tyr
               20                  25                  30
   Leu Val Tyr Ala Asp Ala Asp Ala Asp Ala Pro Ala Arg Gly Pro Ala
           35                  40                  45
   Ala Pro Glu Pro Arg Ser Ala Phe Ser Ala Ala Arg Thr Arg Ser Leu
       50                  55                  60
   Val Gly Ser Asp Ala Ala Pro Gly Pro Arg His Arg Pro Leu Ala Phe
   65                  70                  75                  80
   Asp Thr Glu Leu Val Asn Ile Gly Gly Asp Phe Asp Ala Ala Ala Gly
                   85                  90                  95
   Val Phe Arg Cys Arg Leu Pro Gly Ala Tyr Phe Phe Ser Phe Thr Leu
               100                 105                 110
   Gly Lys Leu Pro Arg Lys Thr Leu Ser Val Lys Leu Met Lys Asn Arg
           115                 120                 125
   Asp Glu Val Gln Ala Met Ile Tyr Asp Asp Gly Ala Ser Arg Arg Arg
       130                 135                 140
   Glu Met Gln Ser Gln Ser Val Arg Leu Pro Leu Arg Arg Gly Asp Ala
   145                 150                 155                 160
   Val Trp Leu Leu Ser His Asp His Asp Gly Tyr Gly Ala Tyr Ser Asn
                   165                 170                 175
   His Gly Lys Tyr Ile Thr Phe Ser Gly Phe Leu Val Tyr Pro Asp Leu
               180                 185                 190
   Ala Ala Ala Gly Pro Pro Ala Leu Lys Pro Pro Glu Leu
           195                 200                 205
<210> 12
   <211> 1393
   <212> DNA
   <213> Homo sapiens
<220>
   <221> CDS
   <222> (225)..(1214)
   <223> SF01, cDNA: NM_031909, Protein: NP_114115
<400> 12
<210> 13
   <211> 329
   <212> PRT
   <213> Homo sapiens
<400> 13
   Met Leu Pro Leu Leu Leu Gly Leu Leu Gly Pro Ala Ala Cys Trp Ala
   1               5                   10                  15
   Leu Gly Pro Thr Pro Gly Pro Gly Ser Ser Glu Leu Arg Ser Ala Phe
               20                  25                  30
   Ser Ala Ala Arg Thr Thr Pro Leu Glu Gly Thr Ser Glu Met Ala Val
           35                  40                  45
   Thr Phe Asp Lys Val Tyr Val Asn Ile Gly Gly Asp Phe Asp Val Ala
       50                  55                  60
   Thr Gly Gln Phe Arg Cys Arg Val Pro Gly Ala Tyr Phe Phe Ser Phe
   65                  70                  75                  80
   Thr Ala Gly Lys Ala Pro His Lys Ser Leu Ser Val Met Leu Val Arg
                   85                  90                  95
   Asn Arg Asp Glu Val Gln Ala Leu Ala Phe Asp Glu Gln Arg Arg Pro
               100                 105                 110
   Gly Ala Arg Arg Ala Ala Ser Gln Ser Ala Met Leu Gln Leu Asp Tyr
           115                 120                 125
   Gly Asp Thr Val Trp Leu Arg Leu His Gly Ala Pro His Tyr Ala Leu
   130 135 140
   Gly Ala Pro Gly Ala Thr Phe Ser Gly Tyr Leu Val Tyr Ala Asp Ala
   145 150 155 160
   Asp Ala Asp Ala Pro Ala Arg Gly Pro Pro Ala Pro Pro Glu Pro Arg
   165 170 175
   Ser Ala Phe Ser Ala Ala Arg Thr Arg Ser Leu Val Gly Ser Asp Ala
   180 185 190
   Gly Pro Gly Pro Arg His Gln Pro Leu Ala Phe Asp Thr Glu Phe Val
   195 200 205
   Asn Ile Gly Gly Asp Phe Asp Ala Ala Ala Gly Val Phe Arg Cys Arg
   210 215 220
   Leu Pro Gly Ala Tyr Phe Phe Ser Phe Thr Leu Gly Lys Leu Pro Arg
   225 230 235 240
   Lys Thr Leu Ser Val Lys Leu Met Lys Asn Arg Asp Glu Val Gln Ala
   245 250 255
   Met Ile Tyr Asp Asp Gly Ala Ser Arg Arg Arg Glu Met Gln Ser Gln
   260 265 270
   Ser Val Met Leu Ala Leu Arg Arg Gly Asp Ala Val Trp Leu Leu Ser
   275 280 285
   His Asp His Asp Gly Tyr Gly Ala Tyr Ser Asn His Gly Lys Tyr Ile
   290 295 300
   Thr Phe Ser Gly Phe Leu Val Tyr Pro Asp Leu Ala Pro Ala Ala Pro
   305 310 315 320
   Pro Gly Leu Gly Ala Ser Glu Leu Leu
   325
<210> 14
   <211> 2388
   <212> DNA
   <213> Mus musculus
<220>
   <221> CDS
   <222> (244)..(1092)
   <223> SF02, cDNA: NM_178644, Protein: NP_848759
<400> 14
   ggagtccgga gctcaggaat cggggcggct aggggacacg cacggggggc gccgtccggg     60
   actcggggtg cgtggctgta cgcggcccac gtgaattcgc tgcacgtcgt ggggaacctc    120
   cgggttgagt ctcccctcta gaagtagggg actgccggga atccccgaac cggaccccca    180
   acttcgagca aactttgtag gcgcgtctcc cctcccccac gcggcgcgcc gggggccccg      240
   ggg atg cgg ccc cct ggc tgc cgc gac gtc ccc tcg gcg cgc ccc gcg        288
       Met Arg Pro Pro Gly Cys Arg Asp Val Pro Ser Ala Arg Pro Ala
       1               5                   10                  15
   ctg ccg ctg ctg ctg ctg ctg ctg tcg ccg ctg ctg ctt ggg gcg ctg        336
   Leu Pro Leu Leu Leu Leu Leu Leu Ser Pro Leu Leu Leu Gly Ala Leu
                   20                  25                  30
   cac ggc gtg ggc gcg ggc agc ggc gct ccg gcc gag ctg cgg gtc cga        384
   His Gly Val Gly Ala Gly Ser Gly Ala Pro Ala Glu Leu Arg Val Arg
               35                  40                  45
   gtg aga ctg ccc gac agc cag gtg atc gag gag agt cta cag gcg gac        432
   Val Arg Leu Pro Asp Ser Gln Val Ile Glu Glu Ser Leu Gln Ala Asp
           50                  55                  60
   agc gac gcg gac agc atc agc ctt gat ctg cgc aaa ccc gac ggc act        480
   Ser Asp Ala Asp Ser Ile Ser Leu Asp Leu Arg Lys Pro Asp Gly Thr
       65                  70                  75
   ctc atc tcc ttc atc gcg gat ttc aag aag gac gtg aag atc ttc cga        528
   Leu Ile Ser Phe Ile Ala Asp Phe Lys Lys Asp Val Lys Ile Phe Arg
   80                  85                  90                  95
   gcc ctg atc ctc ggg gag ctg gag aag ggg cag agt cag ttc cag gca        576
   Ala Leu Ile Leu Gly Glu Leu Glu Lys Gly Gln Ser Gln Phe Gln Ala
                   100                 105                 110
   ctt tgc ttt gtc aca agg ctg cac cac aat gac atc atc ccc agt gag        624
   Leu Cys Phe Val Thr Arg Leu His His Asn Asp Ile Ile Pro Ser Glu
               115                 120                 125
   gcc atg gcc aag ctc cgg cag aaa aac ccc cgc gca gtg cgg cag gct        672
   Ala Met Ala Lys Leu Arg Gln Lys Asn Pro Arg Ala Val Arg Gln Ala
           130                 135                 140
   gag gaa gtg agg ggt ctg gaa cag tta cat atg gat atc gct gtt aac        720
   Glu Glu Val Arg Gly Leu Glu Gln Leu His Met Asp Ile Ala Val Asn
       145                 150                 155
   ttc agc cag ggg ggc ctg ctg agt ccc cat ctc cac aac gta tgt gct        768
   Phe Ser Gln Gly Gly Leu Leu Ser Pro His Leu His Asn Val Cys Ala
   160                 165                 170                 175
   gag gcc aca gat gcc atc tac acc cgc cag gag gat gtc cag ttc tgg        816
   Glu Ala Thr Asp Ala Ile Tyr Thr Arg Gln Glu Asp Val Gln Phe Trp
                   180                 185                 190
   aca gag cga ggt gtg gac agt tct gtt ttc gag gct ctg ccc aag gca        864
   Thr Glu Arg Gly Val Asp Ser Ser Val Phe Glu Ala Leu Pro Lys Ala
               195                 200                 205
   tta gaa cag gcg gaa tta cct cgt tgt gga cga gtt ggg gat cga gga        912
   Leu Glu Gln Ala Glu Leu Pro Arg Cys Gly Arg Val Gly Asp Arg Gly
           210                 215                 220
   aag ccc tgt act tgc cac tac agt ctg agc ctg gcc tgg tac cca tgc        960
   Lys Pro Cys Thr Cys His Tyr Ser Leu Ser Leu Ala Trp Tyr Pro Cys
       225                 230                 235
   atg ctc aag tat tgc cac agt cgt gat cgg cca gcg ccc tac aag tgt       1008
   Met Leu Lys Tyr Cys His Ser Arg Asp Arg Pro Ala Pro Tyr Lys Cys
   240                 245                 250                 255
   ggc atc cga agc tgc agg aaa agc tac acc ttt gac ttc tat gta cct       1056
   Gly Ile Arg Ser Cys Arg Lys Ser Tyr Thr Phe Asp Phe Tyr Val Pro
                   260                 265                 270
   cag aag caa ctg tgc ctc tgg gat gag gac ccc taa caacaggaga       1102
   Gln Lys Gln Leu Cys Leu Trp Asp Glu Asp Pro
               275                 280
   cagaggacca agaacggata cttgggcctg caaattcttc gctgaccacc agagagcgtg   1162
   gcaacccaat caggtcttag gtctcttcct gagccgctgg ccctgaagcc acaacctcat   1222
   ctgcatgact gtgaaagggt gtcaccgcaa ccttgaggca gccctgatgc ccacctgtgc   1282
   ccatcttgag gcattggggg gtgggggggg gaggcagttc tccagacgga taccctcccc   1342
   tcttcctttc acctgaaatg tcccgcgaag gtggaatcaa aatgcaccgg cctaggcctt   1402
   tatggaactt gttccggagg catggccctg tagcacacta tctcaccagc aagggaacca   1462
   gagagggaca gctggggact cgtgccccag ctcctgtgtc tggttacagt gccttctctc   1522
   taccctgggg gcaatgggga gtagggatgc tgcctccaag accaccgcgt gtgcctttcc   1582
   tgagacccaa tttggatact tcagcgggca ccgattcttc ctgcccctgg actgatgtac   1642
   tttggtcagg ttctggggca gggagggagc atgaagtaca aggaaaactt gaattccaga   1702
   tttttaatgc aaaatattta tcatttgtac cagaaataaa agtcttttaa gttttcactc   1762
   accctatggc gccagcttag gctttcggag agaactttgt cccgtgctgc cacctagtgt   1822
   cagaaatgtg ccttacatgg ttagtgccag tctgggcaaa gcaacttcta aaggtcctat   1882
   tgtgtgatct gtgagaggct ctgtgcttaa gatgatggaa caagggagct gggaaggact   1942
   cagtagttaa gagcacgtgc tgctttttta cagaggaccc aagttcgatt ccttacatcc    2002
   gcttccagtg tggggagtgt cacggacacc tgtaacatca acataatgga gatcggccac   2062
   cttttatggc ctccaagtgc tctaacacat ataataaaat taaattaatt taaaattaaa   2122
   aactagggac ttcaggatgg ctcagtgatt aagaacactg gctgctcttc cagagatccc   2182
   atgttccatg ctcaagaccc acatggtaaa tctgatgccc tcttctggct tgcaagaggc   2242
   cttgtacgtg gagctcatac atgcaagcaa aacaccagtg gaaacacaag taagttatat   2302
   aaatgatgag gcaaggaccg aagacatagc tcatttatag cgaatttgct caggatgggc   2362
   aaggccatgg tcctaatgtc ctgccc                                        2388
<210> 15
   <211> 282
   <212> PRT
   <213> Mus musculus
<400> 15
   Met Arg Pro Pro Gly Cys Arg Asp Val Pro Ser Ala Arg Pro Ala Leu
   1 5 10 15
   Pro Leu Leu Leu Leu Leu Leu Ser Pro Leu Leu Leu Gly Ala Leu His
   20 25 30
   Gly Val Gly Ala Gly Ser Gly Ala Pro Ala Glu Leu Arg Val Arg Val
   35 40 45
   Arg Leu Pro Asp Ser Gln Val Ile Glu Glu Ser Leu Gln Ala Asp Ser
   50 55 60
   Asp Ala Asp Ser Ile Ser Leu Asp Leu Arg Lys Pro Asp Gly Thr Leu
   65 70 75 80
   Ile Ser Phe Ile Ala Asp Phe Lys Lys Asp Val Lys Ile Phe Arg Ala
   85 90 95
   Leu Ile Leu Gly Glu Leu Glu Lys Gly Gln Ser Gln Phe Gln Ala Leu
   100 105 110
   Cys Phe Val Thr Arg Leu His His Asn Asp Ile Ile Pro Ser Glu Ala
   115 120 125
   Met Ala Lys Leu Arg Gln Lys Asn Pro Arg Ala Val Arg Gln Ala Glu
   130 135 140
   Glu Val Arg Gly Leu Glu Gln Leu His Met Asp Ile Ala Val Asn Phe
   145 150 155 160
   Ser Gln Gly Gly Leu Leu Ser Pro His Leu His Asn Val Cys Ala Glu
   165 170 175
   Ala Thr Asp Ala Ile Tyr Thr Arg Gln Glu Asp Val Gln Phe Trp Thr
   180 185 190
   Glu Arg Gly Val Asp Ser Ser Val Phe Glu Ala Leu Pro Lys Ala Leu
   195 200 205
   Glu Gln Ala Glu Leu Pro Arg Cys Gly Arg Val Gly Asp Arg Gly Lys
   210 215 220
   Pro Cys Thr Cys His Tyr Ser Leu Ser Leu Ala Trp Tyr Pro Cys Met
   225 230 235 240
   Leu Lys Tyr Cys His Ser Arg Asp Arg Pro Ala Pro Tyr Lys Cys Gly
   245 250 255
   Ile Arg Ser Cys Arg Lys Ser Tyr Thr Phe Asp Phe Tyr Val Pro Gln
   260 265 270
   Lys Gln Leu Cys Leu Trp Asp Glu Asp Pro
   275 280
<210> 16
   <211> 1957
   <212> DNA
   <213> Homo sapiens
<220>
   <221> CDS
   <222> (242)..(1063)
   <223> SF02, cDNA: NM_178507, Protein: NP_848602
<400> 16
   cccggggccg cggagccggg ccggggcagc gccgtctccg cctcggggcc gccgggggcg      60
   ccctgctgag cgctacccac gtgcgtccgc gccacctcgc gggcgacccc gcggccaagg     120
   cccccggcgg agcggctccc gggcgccccg aactagcccc caactttggg cgaagtttgc     180
   ctgcgcctct ccccgccccc acgcggcgcg ccggggccgc ggacggcagc ggcccccggg     240
   g atg cgc ctt ccc ggg gta ccc ctg gcg cgc cct gcg ctg ctg ctg ctg     289
     Met Arg Leu Pro Gly Val Pro Leu Ala Arg Pro Ala Leu Leu Leu Leu
     1               5                   10                  15
   ctg ccg ctg ctc gcg ccg ctg ctg gga acg ggt gcg ccg gcc gag ctg       337
   Leu Pro Leu Leu Ala Pro Leu Leu Gly Thr Gly Ala Pro Ala Glu Leu
               20                  25                  30
   cgg gtc cgc gtg cgg ctg ccg gac ggc cag gtg acc gag gag agc ctg       385
   Arg Val Arg Val Arg Leu Pro Asp Gly Gln Val Thr Glu Glu Ser Leu
           35                  40                  45
   cag gcg gac agc gac gcg gac agc atc agc ctc gag ctg cgc aag ccc       433
   Gln Ala Asp Ser Asp Ala Asp Ser Ile Ser Leu Glu Leu Arg Lys Pro
       50                  55                  60
   gac ggc acc ctc gtc tcc ttc acc gcc gac ttc aag aag gat gtg aag       481
   Asp Gly Thr Leu Val Ser Phe Thr Ala Asp Phe Lys Lys Asp Val Lys
   65                  70                  75                  80
   gtc ttc cgg gcc ctg atc ctg ggg gag ctg gag aag ggg cag agt cag       529
   Val Phe Arg Ala Leu Ile Leu Gly Glu Leu Glu Lys Gly Gln Ser Gln
                   85                  90                  95
   ttc cag gcc ctc tgc ttt gtc acc cag ctg cag cac aat gag atc atc       577
   Phe Gln Ala Leu Cys Phe Val Thr Gln Leu Gln His Asn Glu Ile Ile
               100                 105                 110
   ccc agt gag gcc atg gcc aag ctc cgg cag aaa aat ccc cgg gca gtg       625
   Pro Ser Glu Ala Met Ala Lys Leu Arg Gln Lys Asn Pro Arg Ala Val
           115                 120                 125
   cgg cag gcg gag gag gtt cgg ggt ctg gag cat ctg cac atg gat gtc       673
   Arg Gln Ala Glu Glu Val Arg Gly Leu Glu His Leu His Met Asp Val
       130                 135                 140
   gct gtc aac ttc agc cag ggg gcc ctg ctg agc ccc cat ctc cac aac       721
   Ala Val Asn Phe Ser Gln Gly Ala Leu Leu Ser Pro His Leu His Asn
   145                 150                 155                 160
   gtg tgt gcc gag gcc gtg gat gcc atc tac acc cgc cag gag gat gtc       769
   Val Cys Ala Glu Ala Val Asp Ala Ile Tyr Thr Arg Gln Glu Asp Val
                   165                 170                 175
   cgg ttc tgg ctg gag caa ggt gtg gac agt tct gtg ttc gag gct ctg       817
   Arg Phe Trp Leu Glu Gln Gly Val Asp Ser Ser Val Phe Glu Ala Leu
               180                 185                 190
   ccc aag gcc tca gag cag gcg gag ctg cct cgc tgc agg cag gtg ggg       865
   Pro Lys Ala Ser Glu Gln Ala Glu Leu Pro Arg Cys Arg Gln Val Gly
           195                 200                 205
   gac cac ggg aag ccc tgc gtc tgc cgc tat ggc ctg agc ctg gcc tgg       913
   Asp His Gly Lys Pro Cys Val Cys Arg Tyr Gly Leu Ser Leu Ala Trp
       210                 215                 220
   tac ccc tgc atg ctc aag tac tgc cac agc cgc gac cgg ccc acg ccc       961
   Tyr Pro Cys Met Leu Lys Tyr Cys His Ser Arg Asp Arg Pro Thr Pro
   225                 230                 235                 240
   tac aag tgt ggc atc cgc agc tgc cag aag agc tac agc ttc gac ttc      1009
   Tyr Lys Cys Gly Ile Arg Ser Cys Gln Lys Ser Tyr Ser Phe Asp Phe
                   245                 250                 255
   tac gtg ccc cag agg cag ctg tgt ctc tgg gat gag gat ccc tac cca      1057
   Tyr Val Pro Gln Arg Gln Leu Cys Leu Trp Asp Glu Asp Pro Tyr Pro
               260                 265                 270
   ggc tag ggtgggagca acctggcggg tggctgctct gggcccactg ctcttcacca      1113
   Gly
   gccactagag ggggtggcaa cccccacctg aggccttatt tccctccctc cccactcccc   1173
   tggccctaga gcctgggccc ctctggcccc atctcacatg actgtgaagg gggtgtggca   1233
   tggcaggggg tctcatgaag gcacccccat tcccaccctg tgccttcctt gcgggcagag   1293
   agggagagaa gggctcccca gatctacacc cctccctcct gcatctcccc tggagtgttc   1353
   acttgcaagc tgccaaaaca tgatggcctc tggttgttct gttgaactcc ttgaacgttt   1413
   agaccctaaa aggagtctat acctggacac ccacctcccc agacacaact cccttcccca   1473
   tgcacacatc tggaaggagc tggcccctca gtcccttcct actccccaac aaggggctca   1533
   ctatccccaa agaaggagct gttggggacc cacgacgcag cccctgtact ggattacagc   1593
   atattctcat ctctggcccc gaggctgcct gtggggcgag tggagacctc ccatcactga   1653
   gacagatcac agaccacgag tgcctttccc ggacctggac gttgcctcca gagcaggcac   1713
   cagctctttc cctctctaca cagaaatatt tttgtaaggt tctggggcag ggagggagca   1773
   tgaagtacga ggaaaacttg aattccagat ttttagtgca aagtatttat catttctacc   1833
   agaaataaac gttttaagtt tttacttgaa aaaaaaaaaa aaaaaaaaaa aaaaaaaaaa   1893
   aaaaaaaaaa aaaaaaaaaa aaaaaaaaaa aaaaaaaaaa aaaaaaaaaa aaaaaaaaaa   1953
   aaaa                                                                 1957
<210> 17
   <211> 273
   <212> PRT
   <213> Homo sapiens
<400> 17
   Met Arg Leu Pro Gly Val Pro Leu Ala Arg Pro Ala Leu Leu Leu Leu
   1               5                   10                  15
   Leu Pro Leu Leu Ala Pro Leu Leu Gly Thr Gly Ala Pro Ala Glu Leu
               20                  25                  30
   Arg Val Arg Val Arg Leu Pro Asp Gly Gln Val Thr Glu Glu Ser Leu
           35                  40                  45
   Gln Ala Asp Ser Asp Ala Asp Ser Ile Ser Leu Glu Leu Arg Lys Pro
   50 55 60
   Asp Gly Thr Leu Val Ser Phe Thr Ala Asp Phe Lys Lys Asp Val Lys
   65 70 75 80
   Val Phe Arg Ala Leu Ile Leu Gly Glu Leu Glu Lys Gly Gln Ser Gln
   85 90 95
   Phe Gln Ala Leu Cys Phe Val Thr Gln Leu Gln His Asn Glu Ile Ile
   100 105 110
   Pro Ser Glu Ala Met Ala Lys Leu Arg Gln Lys Asn Pro Arg Ala Val
   115 120 125
   Arg Gln Ala Glu Glu Val Arg Gly Leu Glu His Leu His Met Asp Val
   130 135 140
   Ala Val Asn Phe Ser Gln Gly Ala Leu Leu Ser Pro His Leu His Asn
   145 150 155 160
   Val Cys Ala Glu Ala Val Asp Ala Ile Tyr Thr Arg Gln Glu Asp Val
   165 170 175
   Arg Phe Trp Leu Glu Gln Gly Val Asp Ser Ser Val Phe Glu Ala Leu
   180 185 190
   Pro Lys Ala Ser Glu Gln Ala Glu Leu Pro Arg Cys Arg Gln Val Gly
   195 200 205
   Asp His Gly Lys Pro Cys Val Cys Arg Tyr Gly Leu Ser Leu Ala Trp
   210 215 220
   Tyr Pro Cys Met Leu Lys Tyr Cys His Ser Arg Asp Arg Pro Thr Pro
   225 230 235 240
   Tyr Lys Cys Gly Ile Arg Ser Cys Gln Lys Ser Tyr Ser Phe Asp Phe
   245 250 255
   Tyr Val Pro Gln Arg Gln Leu Cys Leu Trp Asp Glu Asp Pro Tyr Pro
   260 265 270
   Gly
<210> 18
   <211> 2312
   <212> DNA
   <213> Mus musculus
<220>
   <221> CDS
   <222> (118)..(1857)
   <223> SF03, cDNA: NM_016697, Protein: NP_057906
<400> 18
   ctgggtagcg gctcctctct tgctctgtcg ggctactgcc agacttgctg agtctcggga      60
   ccgctccggc tcttattgcc actctctcgt gctctcctcg ctcccccaag aagcagg      117
   atg gcc ggg acc gtg cgc acc gcg tgc ttg ctg gtg gcg atg ctg cta      165
   Met Ala Gly Thr Val Arg Thr Ala Cys Leu Leu Val Ala Met Leu Leu
   1               5                   10                  15
   ggc ttg ggc tgc ctg gga cag gcg cag ccc ccg ccg cct cca gac gcc      213
   Gly Leu Gly Cys Leu Gly Gln Ala Gln Pro Pro Pro Pro Pro Asp Ala
               20                  25                  30
   acc tgt cac cag gtc cgt tct ttc ttc cag aga ctg cag ccc gga ctc      261
   Thr Cys His Gln Val Arg Ser Phe Phe Gln Arg Leu Gln Pro Gly Leu
           35                  40                  45
   aaa tgg gtt cca gaa acc cct gta cca gga tca gat ttg caa gta tgt      309
   Lys Trp Val Pro Glu Thr Pro Val Pro Gly Ser Asp Leu Gln Val Cys
       50                  55                  60
   ctc ccc aag ggc cca aca tgc tgc tca aga aag atg gaa gaa aaa tac      357
   Leu Pro Lys Gly Pro Thr Cys Cys Ser Arg Lys Met Glu Glu Lys Tyr
   65                  70                  75                  80
   caa cta aca gca cgg ctg aac atg gaa caa ctg ctc cag tct gcg agt      405
   Gln Leu Thr Ala Arg Leu Asn Met Glu Gln Leu Leu Gln Ser Ala Ser
                   85                  90                  95
   atg gaa ctc aag ttc tta att att cag aat gct gcg gtt ttc caa gag      453
   Met Glu Leu Lys Phe Leu Ile Ile Gln Asn Ala Ala Val Phe Gln Glu
               100                 105                 110
   gcc ttt gaa att gtt gtt cgc cat gcc aag aac tac acc aac gcc atg      501
   Ala Phe Glu Ile Val Val Arg His Ala Lys Asn Tyr Thr Asn Ala Met
           115                 120                 125
   ttc aag aat aac tac ccc agc ctg act cca caa gct ttt gag ttt gtc      549
   Phe Lys Asn Asn Tyr Pro Ser Leu Thr Pro Gln Ala Phe Glu Phe Val
       130                 135                 140
   ggt gaa ttt ttc aca gat gtg tct ctc tac atc ttg ggt tct gat atc      597
   Gly Glu Phe Phe Thr Asp Val Ser Leu Tyr Ile Leu Gly Ser Asp Ile
   145                 150                 155                 160
   aac gtg gat gat atg gtc aat gaa ttg ttc gac agc ctc ttt cca gtc      645
   Asn Val Asp Asp Met Val Asn Glu Leu Phe Asp Ser Leu Phe Pro Val
                   165                 170                 175
   atc tac acc cag atg atg aac cca ggc ctg cct gag tca gtc tta gac      693
   Ile Tyr Thr Gln Met Met Asn Pro Gly Leu Pro Glu Ser Val Leu Asp
               180                 185                 190
   atc aac gag tgc ctc cga gga gca aga cgt gac ctg aaa gta ttt ggc      741
   Ile Asn Glu Cys Leu Arg Gly Ala Arg Arg Asp Leu Lys Val Phe Gly
           195                 200                 205
   agt ttc ccc aag ctt att atg acc cag gtt tcc aag tca ctg caa gtc      789
   Ser Phe Pro Lys Leu Ile Met Thr Gln Val Ser Lys Ser Leu Gln Val
       210                 215                 220
   act cga atc ttc ctt caa gcc ctg aat ctc gga att gaa gtc atc aac      837
   Thr Arg Ile Phe Leu Gln Ala Leu Asn Leu Gly Ile Glu Val Ile Asn
   225                 230                 235                 240
   act acc gac cac ctc aag ttt agt aag gac tgt ggc cgt atg ctc acc      885
   Thr Thr Asp His Leu Lys Phe Ser Lys Asp Cys Gly Arg Met Leu Thr
                   245                     250                     255
   cga atg tgg tat tgc tct tac tgc cag gga ctg atg atg gtt aag cct      933
   Arg Met Trp Tyr Cys Ser Tyr Cys Gln Gly Leu Met Met Val Lys Pro
               260                     265                     270
   tgc ggt ggt tat tgc aat gtg gtc atg caa ggc tgt atg gct ggt gtg      981
   Cys Gly Gly Tyr Cys Asn Val Val Met Gln Gly Cys Met Ala Gly Val
           275                     280                     285
   gtg gag atc gac aag tac tgg aga gaa tac att ctg tct ctt gaa gag     1029
   Val Glu Ile Asp Lys Tyr Trp Arg Glu Tyr Ile Leu Ser Leu Glu Glu
       290                     295                     300
   ctc gtg aat ggc atg tac aga atc tac gac atg gag aat gtg ctg ctc     1077
   Leu Val Asn Gly Met Tyr Arg Ile Tyr Asp Met Glu Asn Val Leu Leu
   305                     310                     315                     320
   ggc ctc ttt tct acc atc cat gat tcc atc cag tat gtg cag aag aac     1125
   Gly Leu Phe Ser Thr Ile His Asp Ser Ile Gln Tyr Val Gln Lys Asn
                   325                     330                     335
   gga ggc aag ctg acc acc acc att ggc aag ttg tgt gcc cac tcc cag     1173
   Gly Gly Lys Leu Thr Thr Thr Ile Gly Lys Leu Cys Ala His Ser Gln
               340                     345                     350
   caa cgc caa tat aga tct gct tat tac cct gaa gat ctg ttt att gac     1221
   Gln Arg Gln Tyr Arg Ser Ala Tyr Tyr Pro Glu Asp Leu Phe Ile Asp
           355                     360                     365
   aag aag ata tta aaa gtc gct cat gtc gaa cat gaa gaa acc tta tcc     1269
   Lys Lys Ile Leu Lys Val Ala His Val Glu His Glu Glu Thr Leu Ser
       370                     375                     380
   agc cga aga agg gaa ctg att cag aaa ctg aag tct ttc atc aac ttc     1317
   Ser Arg Arg Arg Glu Leu Ile Gln Lys Leu Lys Ser Phe Ile Asn Phe
   385                     390                     395                     400
   tat agc gct ttg ccg ggc tac atc tgc agc cat agc ccc gtg gcc gaa     1365
   Tyr Ser Ala Leu Pro Gly Tyr Ile Cys Ser His Ser Pro Val Ala Glu
                   405                     410                     415
   aat gat acc ctg tgc tgg aac gga caa gaa ctt gtg gag aga tac agc     1413
   Asn Asp Thr Leu Cys Trp Asn Gly Gln Glu Leu Val Glu Arg Tyr Ser
               420                     425                     430
   cag aag gcg gca agg aac ggg atg aag aat cag ttt aac ctc cat gag     1461
   Gln Lys Ala Ala Arg Asn Gly Met Lys Asn Gln Phe Asn Leu His Glu
           435                     440                     445
   ctg aaa atg aag ggc cct gag ccg gtg gtt agc cag atc att gac aaa     1509
   Leu Lys Met Lys Gly Pro Glu Pro Val Val Ser Gln Ile Ile Asp Lys
       450                     455                     460
   ctg aag cac att aac cag ctc ctg aga acc atg tct gtg ccc aag ggt     1557
   Leu Lys His Ile Asn Gln Leu Leu Arg Thr Met Ser Val Pro Lys Gly
   465                     470                     475                     480
   aaa gtt ctg gat aaa agc ctg gat gaa gaa gga ctt gaa agt gga gac     1605
   Lys Val Leu Asp Lys Ser Leu Asp Glu Glu Gly Leu Glu Ser Gly Asp
                   485                     490                     495
   tgc ggt gat gat gaa gat gaa tgc att gga agc tct ggt gac ggg atg     1653
   Cys Gly Asp Asp Glu Asp Glu Cys Ile Gly Ser Ser Gly Asp Gly Met
               500                     505                     510
   gtg aaa gtg aag aat caa ctg cgc ttc ctt gca gaa ctg gcc tat gat      1701
   Val Lys Val Lys Asn Gln Leu Arg Phe Leu Ala Glu Leu Ala Tyr Asp
           515                 520                 525
   ctg gat gtg gac gat gct ccg ggg aac aag cag cat gga aat cag aag      1749
   Leu Asp Val Asp Asp Ala Pro Gly Asn Lys Gln His Gly Asn Gln Lys
       530                 535                 540
   gac aac gag atc acc acc tct cac agc gtg ggg aac atg ccg tcc cca      1797
   Asp Asn Glu Ile Thr Thr Ser His Ser Val Gly Asn Met Pro Ser Pro
   545                 550                 555                 560
   ctg aag atc ctc atc agt gtg gcc atc tat gtg gcg tgc ttt ttt ttc      1845
   Leu Lys Ile Leu Ile Ser Val Ala Ile Tyr Val Ala Cys Phe Phe Phe
                   565                 570                 575
   ctg gtg cac tga cttgccagcg tccagtgcct gtgctgccct gcagcacctg          1897
   Leu Val His
   tggtccctac agaaagggag ccaccttctt ttttttttct tttttttttt ttttttatct      1957
   tttatgcctc ctcccaccac cattaagtag gagactaacc gcgtgttatg ttttcgaaaa    2017
   tcaaatggta tctttatgag gatggtaaat tttagtggta ggatagattg tctttttgca    2077
   aagaaaaaaa aaaccttcaa gttgtgccaa attattttct tacatttgac tgttggaaca    2137
   tggttgtcat gtttccctct tttctctttc tctgcatgga tttctttgac aaaaaaaaaa    2197
   taaataaaca ttcaaataaa aaaaaaaaaa aaaaaaaaaa aaaaaaaaaa aaaaaaaaaa    2257
   aaaaaaaaaa aaaaaaaaaa aaaaaaaaaa aaaaaaaaaa aaaaaaaaaa aaaaa         2312
<210> 19
   <211> 579
   <212> PRT
   <213> Mus musculus
<400> 19
   Met Ala Gly Thr Val Arg Thr Ala Cys Leu Leu Val Ala Met Leu Leu
   1               5                   10                  15
   Gly Leu Gly Cys Leu Gly Gln Ala Gln Pro Pro Pro Pro Pro Asp Ala
               20                  25                  30
   Thr Cys His Gln Val Arg Ser Phe Phe Gln Arg Leu Gln Pro Gly Leu
           35                  40                  45
   Lys Trp Val Pro Glu Thr Pro Val Pro Gly Ser Asp Leu Gln Val Cys
       50                  55                  60
   Leu Pro Lys Gly Pro Thr Cys Cys Ser Arg Lys Met Glu Glu Lys Tyr
   65                  70                  75                  80
   Gln Leu Thr Ala Arg Leu Asn Met Glu Gln Leu Leu Gln Ser Ala Ser
                   85                  90                  95
   Met Glu Leu Lys Phe Leu Ile Ile Gln Asn Ala Ala Val Phe Gln Glu
   100 105 110
   Ala Phe Glu Ile Val Val Arg His Ala Lys Asn Tyr Thr Asn Ala Met
   115 120 125
   Phe Lys Asn Asn Tyr Pro Ser Leu Thr Pro Gln Ala Phe Glu Phe Val
   130 135 140
   Gly Glu Phe Phe Thr Asp Val Ser Leu Tyr Ile Leu Gly Ser Asp Ile
   145 150 155 160
   Asn Val Asp Asp Met Val Asn Glu Leu Phe Asp Ser Leu Phe Pro Val
   165 170 175
   Ile Tyr Thr Gln Met Met Asn Pro Gly Leu Pro Glu Ser Val Leu Asp
   180 185 190
   Ile Asn Glu Cys Leu Arg Gly Ala Arg Arg Asp Leu Lys Val Phe Gly
   195 200 205
   Ser Phe Pro Lys Leu Ile Met Thr Gln Val Ser Lys Ser Leu Gln Val
   210 215 220
   Thr Arg Ile Phe Leu Gln Ala Leu Asn Leu Gly Ile Glu Val Ile Asn
   225 230 235 240
   Thr Thr Asp His Leu Lys Phe Ser Lys Asp Cys Gly Arg Met Leu Thr
   245 250 255
   Arg Met Trp Tyr Cys Ser Tyr Cys Gln Gly Leu Met Met Val Lys Pro
   260 265 270
   Cys Gly Gly Tyr Cys Asn Val Val Met Gln Gly Cys Met Ala Gly Val
   275 280 285
   Val Glu Ile Asp Lys Tyr Trp Arg Glu Tyr Ile Leu Ser Leu Glu Glu
   290 295 300
   Leu Val Asn Gly Met Tyr Arg Ile Tyr Asp Met Glu Asn Val Leu Leu
   305 310 315 320
   Gly Leu Phe Ser Thr Ile His Asp Ser Ile Gln Tyr Val Gln Lys Asn
   325 330 335
   Gly Gly Lys Leu Thr Thr Thr Ile Gly Lys Leu Cys Ala His Ser Gln
   340 345 350
   Gln Arg Gln Tyr Arg Ser Ala Tyr Tyr Pro Glu Asp Leu Phe Ile Asp
   355 360 365
   Lys Lys Ile Leu Lys Val Ala His Val Glu His Glu Glu Thr Leu Ser
   370 375 380
   Ser Arg Arg Arg Glu Leu Ile Gln Lys Leu Lys Ser Phe Ile Asn Phe
   385 390 395 400
   Tyr Ser Ala Leu Pro Gly Tyr Ile Cys Ser His Ser Pro Val Ala Glu
   405 410 415
   Asn Asp Thr Leu Cys Trp Asn Gly Gln Glu Leu Val Glu Arg Tyr Ser
   420 425 430
   Gln Lys Ala Ala Arg Asn Gly Met Lys Asn Gln Phe Asn Leu His Glu
   435 440 445
   Leu Lys Met Lys Gly Pro Glu Pro Val Val Ser Gln Ile Ile Asp Lys
   450 455 460
   Leu Lys His Ile Asn Gln Leu Leu Arg Thr Met Ser Val Pro Lys Gly
   465 470 475 480
   Lys Val Leu Asp Lys Ser Leu Asp Glu Glu Gly Leu Glu Ser Gly Asp
   485 490 495
   Cys Gly Asp Asp Glu Asp Glu Cys Ile Gly Ser Ser Gly Asp Gly Met
   500 505 510
   Val Lys Val Lys Asn Gln Leu Arg Phe Leu Ala Glu Leu Ala Tyr Asp
   515 520 525
   Leu Asp Val Asp Asp Ala Pro Gly Asn Lys Gln His Gly Asn Gln Lys
   530 535 540
   Asp Asn Glu Ile Thr Thr Ser His Ser Val Gly Asn Met Pro Ser Pro
   545 550 555 560
   Leu Lys Ile Leu Ile Ser Val Ala Ile Tyr Val Ala Cys Phe Phe Phe
   565 570 575
   Leu Val His
<210> 20
   <211> 2382
   <212> DNA
   <213> Homo sapiens
<220>
   <221> CDS
   <222> (191)..(1933)
   <223> SF03, cDNA: NM_004484, Protein: NP_004475
<400> 20
ccctgccccg cgccgccaag cggttcccgc cctcgcccag cgcccaggta gctgcgagga 60
   aacttttgca gcggctgggt agcagcacgt ctcttgctcc tcagggccac tgccaggctt     120
   gccgagtcct gggactgctc tcgctccggc tgccactctc ccgcgctctc ctagctccct     180
   gcgaagcagg atg gcc ggg acc gtg cgc acc gcg tgc ttg gtg gtg gcg        229
   atg ctg ctc agc ttg gac ttc ccg gga cag gcg cag ccc ccg ccg ccg       277
   ccg ccg gac gcc acc tgt cac caa gtc cgc tcc ttc ttc cag aga ctg       325
   cag ccc gga ctc aag tgg gtg cca gaa act ccc gtg cca gga tca gat       373
   ttg caa gta tgt ctc cct aag ggc cca aca tgc tgc tca aga aag atg       421
   gaa gaa aaa tac caa cta aca gca cga ttg aac atg gaa cag ctg ctt       469
   cag tct gca agt atg gag ctc aag ttc tta att att cag aat gct gcg       517
   gtt ttc caa gag gcc ttt gaa att gtt gtt cgc cat gcc aag aac tac       565
   acc aat gcc atg ttc aag aac aac tac cca agc ctg act cca caa gct       613
   ttt gag ttt gtg ggt gaa ttt ttc aca gat gtg tct ctc tac atc ttg       661
   ggt tct gac atc aat gta gat gac atg gtc aat gaa ttg ttt gac agc       709
   ctg ttt cca gtc atc tat acc cag cta atg aac cca ggc ctg cct gat       757
   tca gcc ttg gac atc aat gag tgc ctc cga gga gca aga cgt gac ctg       805
   aaa gta ttt ggg aat ttc ccc aag ctt att atg acc cag gtt tcc aag       853
   tca ctg caa gtc act agg atc ttc ctt cag gct ctg aat ctt gga att       901
   gaa gtg atc aac aca act gat cac ctg aag ttc agt aag gac tgt ggc       949
   cga atg ctc acc aga atg tgg tac tgc tct tac tgc cag gga ctg atg 997
   atg gtt aaa ccc tgt ggc ggt tac tgc aat gtg gtc atg caa ggc tgt 1045
   atg gca ggt gtg gtg gag att gac aag tac tgg aga gaa tac att ctg 1093
   tcc ctt gaa gaa ctt gtg aat ggc atg tac aga atc tat gac atg gag 1141
   aac gta ctg ctt ggt ctc ttt tca aca atc cat gat tct atc cag tat 1189
   gtc cag aag aat gca gga aag ctg acc acc act att ggc aag tta tgt 1237
   gcc cat tct caa caa cgc caa tat aga tct gct tat tat cct gaa gat 1285
   ctc ttt att gac aag aaa gta tta aaa gtt gct cat gta gaa cat gaa 1333
   gaa acc tta tcc agc cga aga agg gaa cta att cag aag ttg aag tct 1381
   ttc atc agc ttc tat agt gct ttg cct ggc tac atc tgc agc cat agc 1429
   cct gtg gcg gaa aac gac acc ctt tgc tgg aat gga caa gaa ctc gtg 1477
   gag aga tac agc caa aag gca gca agg aat gga atg aaa aac cag ttc 1525
   aat ctc cat gag ctg aaa atg aag ggc cct gag cca gtg gtc agt caa 1573
   att att gac aaa ctg aag cac att aac cag ctc ctg aga acc atg tct 1621
   atg ccc aaa ggt aga gtt ctg gat aaa aac ctg gat gag gaa ggg ttt 1669
   gaa agt gga gac tgc ggt gat gat gaa gat gag tgc att gga ggc tct 1717
   ggt gat gga atg ata aaa gtg aag aat cag ctc cgc ttc ctt gca gaa 1765
   ctg gcc tat gat ctg gat gtg gat gat gcg cct gga aac agt cag cag      1813
   gca act ccg aag gac aac gag ata agc acc ttt cac aac ctc ggg aac      1861
   gtt cat tcc ccg ctg aag ctt ctc acc agc atg gcc atc tcg gtg gtg      1909
   tgc ttc ttc ttc ctg gtg cac tga ctgcctggtg cccagcacat gtgctgccct     1963
   acagcaccct gtggtcttcc tcgataaagg gaaccacttt cttatttttt tctatttttt     2023
   tttttttgtt atcctgtata cctcctccag ccatgaagta gaggactaac catgtgttat     2083
   gttttcgaaa atcaaatggt atcttttgga ggaagataca ttttagtggt agcatataga     2143
   ttgtcctttt gcaaagaaag aaaaaaaacc atcaagttgt gccaaattat tctcctatgt     2203
   ttggctgcta gaacatggtt accatgtctt tctctctcac tccctccctt tctatcgttc     2263
   tctctttgca tggatttctt tgaaaaaaaa taaattgctc aaataaaaaa aaaaaaaaaa     2323
   aaaaaaaaaa aaaaaaaaaa aaaaaaaaaa aaaaaaaaaa aaaaaaaaaa aaaaaaaaa      2382
<210> 21
   <211> 580
   <212> PRT
   <213> Homo sapiens
<400> 21
<210> 22
   <211> 1234
   <212> DNA
   <213> Mus musculus
<220>
   <221> CDS
   <222> (89)..(1138)
   <223> SF04, cDNA: NM_007443, Protein: NP_031469
<400> 22
<210> 23
   <211> 349
   <212> PRT
   <213> Mus musculus
<400> 23
   Met Gln Gly Leu Arg Thr Leu Phe Leu Leu Leu Thr Ala Cys Leu Ala
   1               5                   10                  15
   Ser Arg Ala Asp Pro Ala Ser Thr Leu Pro Asp Ile Gln Val Gln Glu
               20                  25                  30
   Asn Phe Ser Glu Ser Arg Ile Tyr Gly Lys Trp Tyr Asn Leu Ala Val
           35                  40                  45
   Gly Ser Thr Cys Pro Trp Leu Ser Arg Ile Lys Asp Lys Met Ser Val
       50                  55                  60
   Ser Thr Leu Val Leu Gln Glu Gly Ala Thr Glu Thr Glu Ile Ser Met
   65                  70                  75                  80
   Thr Ser Thr Arg Trp Arg Arg Gly Val Cys Glu Glu Ile Thr Gly Ala
                   85                  90                  95
   Tyr Gln Lys Thr Asp Ile Asp Gly Lys Phe Leu Tyr His Lys Ser Lys
               100                 105                 110
   Trp Asn Ile Thr Leu Glu Ser Tyr Val Val His Thr Asn Tyr Asp Glu
           115                 120                 125
   Tyr Ala Ile Phe Leu Thr Lys Lys Ser Ser His His His Gly Leu Thr
       130                 135                 140
   Ile Thr Ala Lys Leu Tyr Gly Arg Glu Pro Gln Leu Arg Asp Ser Leu
   145 150 155 160
   Leu Gln Glu Phe Lys Asp Val Ala Leu Asn Val Gly Ile Ser Glu Asn
   165 170 175
   Ser Ile Ile Phe Met Pro Asp Arg Gly Glu Cys Val Pro Gly Asp Arg
   180 185 190
   Glu Val Glu Pro Thr Ser Ile Ala Arg Ala Arg Arg Ala Val Leu Pro
   195 200 205
   Gln Glu Ser Glu Gly Ser Gly Thr Glu Pro Leu Ile Thr Gly Thr Leu
   210 215 220
   Lys Lys Glu Asp Ser Cys Gln Leu Asn Tyr Ser Glu Gly Pro Cys Leu
   225 230 235 240
   Gly Met Gln Glu Arg Tyr Tyr Tyr Asn Gly Ala Ser Met Ala Cys Glu
   245 250 255
   Thr Phe Gln Tyr Gly Gly Cys Leu Gly Asn Gly Asn Asn Phe Ile Ser
   260 265 270
   Glu Lys Asp Cys Leu Gln Thr Cys Arg Thr Ile Ala Ala Cys Asn Leu
   275 280 285
   Pro Ile Val Gln Gly Pro Cys Arg Ala Phe Ile Lys Leu Trp Ala Phe
   290 295 300
   Asp Ala Ala Gln Gly Lys Cys Ile Gln Phe His Tyr Gly Gly Cys Lys
   305 310 315 320
   Gly Asn Gly Asn Lys Phe Tyr Ser Glu Lys Glu Cys Lys Glu Tyr Cys
   325 330 335
   Gly Val Pro Gly Asp Gly Tyr Glu Glu Leu Ile Arg Ser
   340 345
<210> 24
   <211> 1413
   <212> DNA
   <213> Homo sapiens
<220>
   <221> CDS
   <222> (227)..(1285)
   <223> SF04, cDNA: NM_001633, Protein: NP_001624
<400> 24
<210> 25
   <211> 352
   <212> PRT
   <213> Homo sapiens
<400> 25
   Met Arg Ser Leu Gly Ala Leu Leu Leu Leu Leu Ser Ala Cys Leu Ala
   1               5                   10                  15
   Val Ser Ala Gly Pro Val Pro Thr Pro Pro Asp Asn Ile Gln Val Gln
               20                  25                  30
   Glu Asn Phe Asn Ile Ser Arg Ile Tyr Gly Lys Trp Tyr Asn Leu Ala
           35                  40                  45
   Ile Gly Ser Thr Cys Pro Trp Leu Lys Lys Ile Met Asp Arg Met Thr
       50                  55                  60
   Val Ser Thr Leu Val Leu Gly Glu Gly Ala Thr Glu Ala Glu Ile Ser
   65                  70                  75                  80
   Met Thr Ser Thr Arg Trp Arg Lys Gly Val Cys Glu Glu Thr Ser Gly
                   85                  90                  95
   Ala Tyr Glu Lys Thr Asp Thr Asp Gly Lys Phe Leu Tyr His Lys Ser
               100                 105                 110
   Lys Trp Asn Ile Thr Met Glu Ser Tyr Val Val His Thr Asn Tyr Asp
   115 120 125
   Glu Tyr Ala Ile Phe Leu Thr Lys Lys Phe Ser Arg His His Gly Pro
   130 135 140
   Thr Ile Thr Ala Lys Leu Tyr Gly Arg Ala Pro Gln Leu Arg Glu Thr
   145 150 155 160
   Leu Leu Gln Asp Phe Arg Val Val Ala Gln Gly Val Gly Ile Pro Glu
   165 170 175
   Asp Ser Ile Phe Thr Met Ala Asp Arg Gly Glu Cys Val Pro Gly Glu
   180 185 190
   Gln Glu Pro Glu Pro Ile Leu Ile Pro Arg Val Arg Arg Ala Val Leu
   195 200 205
   Pro Gln Glu Glu Glu Gly Ser Gly Gly Gly Gln Leu Val Thr Glu Val
   210 215 220
   Thr Lys Lys Glu Asp Ser Cys Gln Leu Gly Tyr Ser Ala Gly Pro Cys
   225 230 235 240
   Met Gly Met Thr Ser Arg Tyr Phe Tyr Asn Gly Thr Ser Met Ala Cys
   245 250 255
   Glu Thr Phe Gln Tyr Gly Gly Cys Met Gly Asn Gly Asn Asn Phe Val
   260 265 270
   Thr Glu Lys Glu Cys Leu Gln Thr Cys Arg Thr Val Ala Ala Cys Asn
   275 280 285
   Leu Pro Ile Val Arg Gly Pro Cys Arg Ala Phe Ile Gln Leu Trp Ala
   290 295 300
   Phe Asp Ala Val Lys Gly Lys Cys Val Leu Phe Pro Tyr Gly Gly Cys
   305 310 315 320
   Gln Gly Asn Gly Asn Lys Phe Tyr Ser Glu Lys Glu Cys Arg Glu Tyr
   325 330 335
   Cys Gly Val Pro Gly Asp Gly Asp Glu Glu Leu Leu Arg Phe Ser Asn
   340 345 350
<210> 26
   <211> 2944
   <212> DNA
   <213> Mus musculus
<220>
   <221> CDS
   <222> (98)..(1330)
   <223> SF05, cDNA: NM_009250, Protein: NP_033276
<400> 26
   cggcacgaga tccggagcag tctcagcctg cccagcatcc tctccagcat cccgagcggg      60
   gattgcaggt gtgaaggaga cttgaaacca tcccatc atg act tac ctt gaa ctg      115
                                            Met Thr Tyr Leu Glu Leu
                                            1               5
   ctt gct ttg ctg gcc ttg caa agt gtg gtg aca ggg gca acg ttc cca      163
   Leu Ala Leu Leu Ala Leu Gln Ser Val Val Thr Gly Ala Thr Phe Pro
               10                  15                  20
   gat gaa acc ata act gag tgg tca gtg aac atg tat aac cac ctt cga      211
   Asp Glu Thr Ile Thr Glu Trp Ser Val Asn Met Tyr Asn His Leu Arg
           25                  30                  35
   ggc acc ggg gaa gat gaa aac att ctc ttc tct cca cta agc att gcc      259
   Gly Thr Gly Glu Asp Glu Asn Ile Leu Phe Ser Pro Leu Ser Ile Ala
       40                  45                  50
   ctt gcg atg gga atg atg gag ctt ggg gct caa gga tct act agg aaa      307
   Leu Ala Met Gly Met Met Glu Leu Gly Ala Gln Gly Ser Thr Arg Lys
   55                  60                  65                  70
   gaa atc cgc cat tca atg gga tat gag ggt ctg aaa ggt ggt gaa gaa      355
   Glu Ile Arg His Ser Met Gly Tyr Glu Gly Leu Lys Gly Gly Glu Glu
                   75                  80                  85
   ttt tct ttc ctg agg gat ttt tct aat atg gcc tct gcc gaa gaa aac      403
   Phe Ser Phe Leu Arg Asp Phe Ser Asn Met Ala Ser Ala Glu Glu Asn
               90                  95                  100
   caa tat gtg atg aaa ctt gcc aat tcg ctc ttt gta caa aat gga ttt      451
   Gln Tyr Val Met Lys Leu Ala Asn Ser Leu Phe Val Gln Asn Gly Phe
           105                 110                 115
   cat gtc aat gag gaa ttc ttg caa atg ctg aaa atg tac ttt aat gca      499
   His Val Asn Glu Glu Phe Leu Gln Met Leu Lys Met Tyr Phe Asn Ala
       120                 125                 130
   gaa gtc aac cat gtg gac ttc agt caa aat gtg gct gtg gct aac tcc      547
   Glu Val Asn His Val Asp Phe Ser Gln Asn Val Ala Val Ala Asn Ser
   135                 140                 145                 150
   atc aat aaa tgg gtg gag aat tat aca aac agt ctg ttg aaa gat ctg      595
   Ile Asn Lys Trp Val Glu Asn Tyr Thr Asn Ser Leu Leu Lys Asp Leu
                   155                 160                 165
   gtg tct ccg gag gac ttt gat ggt gtc act aat ttg gcc ctc atc aat      643
   Val Ser Pro Glu Asp Phe Asp Gly Val Thr Asn Leu Ala Leu Ile Asn
               170                 175                 180
   gct gta tat ttc aaa gga aac tgg aag tct cag ttt aga cct gaa aat      691
   Ala Val Tyr Phe Lys Gly Asn Trp Lys Ser Gln Phe Arg Pro Glu Asn
           185                 190                 195
   acc aga act ttc tcc ttc acg aaa gat gat gaa agt gaa gtg cag att      739
   Thr Arg Thr Phe Ser Phe Thr Lys Asp Asp Glu Ser Glu Val Gln Ile
       200                 205                 210
   cca atg atg tat caa caa gga gaa ttt tat tat ggt gaa ttt agt gat      787
   Pro Met Met Tyr Gln Gln Gly Glu Phe Tyr Tyr Gly Glu Phe Ser Asp
   215                 220                 225                 230
   gga tcc aat gag gct ggt ggt atc tac caa gtc ctt gag ata ccc tat      835
   Gly Ser Asn Glu Ala Gly Gly Ile Tyr Gln Val Leu Glu Ile Pro Tyr
                   235                     240                 245
   gag gga gat gag atc agc atg atg ctg gca ctg tcc aga cag gaa gtc        883
   Glu Gly Asp Glu Ile Ser Met Met Leu Ala Leu Ser Arg Gln Glu Val
               250                 255                     260
   cca ctg gcc aca ctg gag cct ctg ctc aaa gca cag ctg atc gaa gaa        931
   Pro Leu Ala Thr Leu Glu Pro Leu Leu Lys Ala Gln Leu Ile Glu Glu
           265                     270                 275
   tgg gca aac tct gtg aag aaa caa aag gtg gaa gtg tac ttg ccc agg        979
   Trp Ala Asn Ser Val Lys Lys Gln Lys Val Glu Val Tyr Leu Pro Arg
       280                 285                     290
   ttc act gtg gaa cag gaa att gat tta aaa gac atc ttg aaa gcc ctt       1027
   Phe Thr Val Glu Gln Glu Ile Asp Leu Lys Asp Ile Leu Lys Ala Leu
   295                     300                 305                 310
   ggg gtc act gaa att ttc atc aaa gat gca aat ttg act gcc atg tca       1075
   Gly Val Thr Glu Ile Phe Ile Lys Asp Ala Asn Leu Thr Ala Met Ser
                   315                     320                 325
   gat aag aaa gag ctg ttc ctc tcc aaa gct gtt cac aag tcc tgc att       1123
   Asp Lys Lys Glu Leu Phe Leu Ser Lys Ala Val His Lys Ser Cys Ile
               330                 335                     340
   gag gtt aat gaa gaa ggg tca gaa gcc gct gca gcc tcc gga atg att       1171
   Glu Val Asn Glu Glu Gly Ser Glu Ala Ala Ala Ala Ser Gly Met Ile
           345                     350                 355
   gcg att agt agg atg gct gtg ctg tac cct cag gtt att gtc gac cat       1219
   Ala Ile Ser Arg Met Ala Val Leu Tyr Pro Gln Val Ile Val Asp His
       360                 365                     370
   cca ttt ctc tat ctt atc agg aac agg aaa tct ggc ata atc tta ttc       1267
   Pro Phe Leu Tyr Leu Ile Arg Asn Arg Lys Ser Gly Ile Ile Leu Phe
   375                     380                 385                 390
   atg gga cga gtc atg aac cct gaa aca atg aat aca agt ggc cat gac       1315
   Met Gly Arg Val Met Asn Pro Glu Thr Met Asn Thr Ser Gly His Asp
                   395                     400                 405
   ttt gag gaa ctt taa atgacgacgt ttgagtacaa agaaagcagg aacaaagcac       1370
   Phe Glu Glu Leu
               410
   attatgtttg caagtggtat atatttagga tttctgtttt atagtgttac ttagggaaat    1430
   atttaaatag ttctggatag tagtaatcca tgtgacctat aagttagcct gtcaaaagct    1490
   gttatcagta taaagagtat ggtcccattg tgtcattgtg tctggtgtgc tgctgtttaa    1550
   aataaaagta catattgaaa ctgtgaacca ctttttttca ttttgaaagt agttgtagtc    1610
   tatacaatac tatgtctgag atttgaaacc tatgctgttt ctttaggaat tgtagtaaaa    1670
   tgatcctaca aggcaaaatg tagaaactgt tgtttctgag tttcttcata atcatgcaga    1730
   atcaaacacc aaagtaagca acatacatat atatatataa taagcaatac tgtgaagggg    1790
   aggccaaaag gcagagaaat tgagattgtt atttagtgtg gcattccatg acaaaagatt    1850
   taggaggaaa tgtgggatat gtaagaccca tagatgtata ttttgtatat ctgtagtatt    1910
   atacttttaa tttattaaag tataactctt ttatttattt ttaaaagttt cctgtgaacc    1970
   aatatgccac atgactctac tagcaagttc agatatctca ttagctattc tggatgacat    2030
   caagaggcct catggaggga atcccgtgta ccatttacgt tttagtgatt ttttgtgatg 2090
   ttcacacaaa gatgaaatca cattgttgca cactctctag actatatcca agaaaggcat 2150
   caagtggtac attggtgtgc caggaaaata gatgtaatta ctttattaaa aaagttcctg 2210
   gtattgtgca tcatatggaa tcagtgctgc ttaaacttag tacgtcctgc tgacacctgg 2270
   tcacttatta caaatatagg ttcttatcca ggatgtctaa ggtagagtgg gaaccacagc 2330
   tttctatcat tactgacatc caaatgatgc cgcagatatc tgaccatagc ctttgctgag 2390
   agtcccttgg gttgcaatgt cgtacttgaa gtcagcctca cattttcaca gactgagatt 2450
   ggagagatga gggtgcaggg aggagataat ctacactagt gatacgatgc ctttgtcaag 2510
   cactggtgtg atctcgaagt attctagtac acactctaga taaattcttc tgtacattac 2570
   aacacttgaa atgcagtcgt taaaaatatg gagacattta taggcaatac ccatgaaaga 2630
   atttatgact atccgaggac acagtactta acaatgaatc ttttacagct tatattttca 2690
   gaggacttgt agtttattca taaatcttca tgttattgta caatagtgct cttgttttca 2750
   tttataattt atgaagctga gatgctggtg ttaattcagt gttcacattc tctgctaaga 2810
   acagtcttta tctctgtatc cttcttgtta atatgacatc tatagctata tctatatgtt 2870
   cattagttaa acaaatgtat ggcctgtaag gaagaataaa cattattatg caatcatgta 2930
   aaaaaaaaaa aaaa 2944
<210> 27
   <211> 410
   <212> PRT
   <213> Mus musculus
<400> 27
   Met Thr Tyr Leu Glu Leu Leu Ala Leu Leu Ala Leu Gln Ser Val Val
   1 5 10 15
   Thr Gly Ala Thr Phe Pro Asp Glu Thr Ile Thr Glu Trp Ser Val Asn
   20 25 30
   Met Tyr Asn His Leu Arg Gly Thr Gly Glu Asp Glu Asn Ile Leu Phe
   35 40 45
   Ser Pro Leu Ser Ile Ala Leu Ala Met Gly Met Met Glu Leu Gly Ala
   50 55 60
   Gln Gly Ser Thr Arg Lys Glu Ile Arg His Ser Met Gly Tyr Glu Gly
   65 70 75 80
   Leu Lys Gly Gly Glu Glu Phe Ser Phe Leu Arg Asp Phe Ser Asn Met
   85 90 95
   Ala Ser Ala Glu Glu Asn Gln Tyr Val Met Lys Leu Ala Asn Ser Leu
   100 105 110
   Phe Val Gln Asn Gly Phe His Val Asn Glu Glu Phe Leu Gln Met Leu
   115 120 125
   Lys Met Tyr Phe Asn Ala Glu Val Asn His Val Asp Phe Ser Gln Asn
   130 135 140
   Val Ala Val Ala Asn Ser Ile Asn Lys Trp Val Glu Asn Tyr Thr Asn
   145 150 155 160
   Ser Leu Leu Lys Asp Leu Val Ser Pro Glu Asp Phe Asp Gly Val Thr
   165 170 175
   Asn Leu Ala Leu Ile Asn Ala Val Tyr Phe Lys Gly Asn Trp Lys Ser
   180 185 190
   Gln Phe Arg Pro Glu Asn Thr Arg Thr Phe Ser Phe Thr Lys Asp Asp
   195 200 205
   Glu Ser Glu Val Gln Ile Pro Met Met Tyr Gln Gln Gly Glu Phe Tyr
   210 215 220
   Tyr Gly Glu Phe Ser Asp Gly Ser Asn Glu Ala Gly Gly Ile Tyr Gln
   225 230 235 240
   Val Leu Glu Ile Pro Tyr Glu Gly Asp Glu Ile Ser Met Met Leu Ala
   245 250 255
   Leu Ser Arg Gln Glu Val Pro Leu Ala Thr Leu Glu Pro Leu Leu Lys
   260 265 270
   Ala Gln Leu Ile Glu Glu Trp Ala Asn Ser Val Lys Lys Gln Lys Val
   275 280 285
   Glu Val Tyr Leu Pro Arg Phe Thr Val Glu Gln Glu Ile Asp Leu Lys
   290 295 300
   Asp Ile Leu Lys Ala Leu Gly Val Thr Glu Ile Phe Ile Lys Asp Ala
   305 310 315 320
   Asn Leu Thr Ala Met Ser Asp Lys Lys Glu Leu Phe Leu Ser Lys Ala
   325 330 335
   Val His Lys Ser Cys Ile Glu Val Asn Glu Glu Gly Ser Glu Ala Ala
   340 345 350
   Ala Ala Ser Gly Met Ile Ala Ile Ser Arg Met Ala Val Leu Tyr Pro
   355 360 365
   Gln Val Ile Val Asp His Pro Phe Leu Tyr Leu Ile Arg Asn Arg Lys
   370 375 380
   Ser Gly Ile Ile Leu Phe Met Gly Arg Val Met Asn Pro Glu Thr Met
   385 390 395 400
   Asn Thr Ser Gly His Asp Phe Glu Glu Leu
   405 410
<210> 28
   <211> 1910
   <212> DNA
   <213> Homo sapiens
<220>
   <221> CDS
   <222> (421)..(1653)
   <223> SF05, cDNA: NM_005025, Protein: NP_005016
<400> 28
ggagacgaaa gcaggaacga gagcggagcg gagcacagtc cgccgagcac aagctccagc      60
   atcccgtcag gggttgcagg tgtgtgggag gtaagcgtgg gtccttcttc ggggcgtctg     120
   cactgccctg gagaaaacgt cttccaatag gaattacatc aagacacagc taaaagagtc     180
   cgcgttgggt taggaaaccg gtgcaaaacc tctcgggtga aagatttaca tttttcgact     240
   ttaagggcaa aaaagcaaaa ggaagcttga ctctgggtat aaagggcgtg gttagtgttt     300
   ttggtttgag ttgcaccagt aaaactgttc tgggagatct gaagattgag aaaaatcctg     360
   ctaattgagg acgaggtgga tgttatctgg tggatgttat aggcttgaaa ctgttacaat     420
   atg gct ttc ctt gga ctc ttc tct ttg ctg gtt ctg caa agt atg gct      468
   aca ggg gcc act ttc cct gag gaa gcc att gct gac ttg tca gtg aat      516
   atg tac aat cgt ctt aga gcc act ggt gaa gat gaa aat att ctc ttc      564
   tct cca ttg agt att gct ctt gca atg gga atg atg gaa ctt ggg gcc      612
   caa gga tct acc cag aaa gaa atc cgc cac tca atg gga tat gac agc      660
   cta aaa aat ggt gaa gaa ttt tct ttc ttg aag gag ttt tca aac atg      708
   gta act gct aaa gag agc caa tat gtg atg aaa att gcc aat tcc ttg      756
   ttt gtg caa aat gga ttt cat gtc aat gag gag ttt ttg caa atg atg      804
   aaa aaa tat ttt aat gca gca gta aat cat gtg gac ttc agt caa aat      852
   gta gcc gtg gcc aac tac atc aat aag tgg gtg gag aat aac aca aac      900
   aat ctg gtg aaa gat ttg gta tcc cca agg gat ttt gat gct gcc act      948
   tat ctg gcc ctc att aat gct gtc tat ttc aag ggg aac tgg aag tcg      996
   cag ttt agg cct gaa aat act aga acc ttt tct ttc act aaa gat gat      1044
   gaa agt gaa gtc caa att cca atg atg tat cag caa gga gaa ttt tat      1092
   tat ggg gaa ttt agt gat ggc tcc aat gaa gct ggt ggt atc tac caa      1140
   gtc cta gaa ata cca tat gaa gga gat gaa ata agc atg atg ctg gtg      1188
   ctg tcc aga cag gaa gtt cct ctt gct act ctg gag cca tta gtc aaa      1236
   gca cag ctg gtt gaa gaa tgg gca aac tct gtg aag aag caa aaa gta      1284
   gaa gta tac ctg ccc agg ttc aca gtg gaa cag gaa att gat tta aaa      1332
   gat gtt ttg aag gct ctt gga ata act gaa att ttc atc aaa gat gca      1380
   aat ttg aca ggc ctc tct gat aat aag gag att ttt ctt tcc aaa gca      1428
   att cac aag tcc ttc cta gag gtt aat gaa gaa ggc tca gaa gct gct      1476
   gct gtc tca gga atg att gca att agt agg atg gct gtg ctg tat cct      1524
   caa gtt att gtc gac cat cca ttt ttc ttt ctt atc aga aac agg aga      1572
   act ggt aca att cta ttc atg gga cga gtc atg cat cct gaa aca atg      1620
   aac aca agt gga cat gat ttc gaa gaa ctt taa gttactttat ttgaataaca    1673
   aggaaaacag taactaagca cattatgttt gcaactggta tatatttagg atttgtgttt 1733
   tacagtatat cttaagataa tatttaaaat agttccagat aaaaacaata tatgtaaatt 1793
   ataagtaact tgtcaaggaa tgttatcagt attaagctaa tggtcctgtt atgtcattgt 1853
   gtttgtgtgc tgttgtttaa aataaaagta cctattgaac atgaaaaaaa aaaaaaa 1910
<210> 29
   <211> 410
   <212> PRT
   <213> Homo sapiens
<400> 29
<210> 30
   <211> 2202
   <212> DNA
   <213> Mus musculus
<220>
   <221> CDS
   <222> (23)..(697)
   <223> SF06, cDNA: NM_172633, Protein: NP_766221
<400> 30
   gacagggcgc cccccagccc ag atg ccc gcg cct ggc cag ggc ccc aga ggg 52
                                 Met Pro Ala Pro Gly Gln Gly Pro Arg Gly
                                 1               5                   10
   ccg ctg ctg agc atg ccc ggg cgc cgg ggg gcg ctg cgt gag cca gcc       100
   Pro Leu Leu Ser Met Pro Gly Arg Arg Gly Ala Leu Arg Glu Pro Ala
                   15                  20                  25
   gac ttt ggc tcc agc ctg ggg gcg gtg ctg gcc ctg ctg ttg ctg ctg       148
   Asp Phe Gly Ser Ser Leu Gly Ala Val Leu Ala Leu Leu Leu Leu Leu
               30                  35                  40
   ctg ccc gcc tgc tgc ccc gta agg gct cag aac gac acg gag ccc atc       196
   Leu Pro Ala Cys Cys Pro Val Arg Ala Gln Asn Asp Thr Glu Pro Ile
           45                  50                  55
   gtg cta gag ggc aag tgc ctg gta gtg tgc gat tcc agc cca tcg ggg       244
   Val Leu Glu Gly Lys Cys Leu Val Val Cys Asp Ser Ser Pro Ser Gly
       60                  65                  70
   gat ggc gcc gtc act tct tcc ctg ggc att tct gtg cgc tca ggc agt       292
   Asp Gly Ala Val Thr Ser Ser Leu Gly Ile Ser Val Arg Ser Gly Ser
   75                  80                  85                  90
   gcc aag gtg gcc ttc tcc gct act cgg agc acc aac cac gag ccg tca       340
   Ala Lys Val Ala Phe Ser Ala Thr Arg Ser Thr Asn His Glu Pro Ser
                   95                  100                 105
   gag atg agc aac cgt acc atg acc atc tac ttc gac cag gtc tta gta       388
   Glu Met Ser Asn Arg Thr Met Thr Ile Tyr Phe Asp Gln Val Leu Val
               110                 115                 120
   aac att ggc aac cac ttt gac ctt gcc tcc agt ata ttt gta gca cca       436
   Asn Ile Gly Asn His Phe Asp Leu Ala Ser Ser Ile Phe Val Ala Pro
           125                 130                 135
   aga aag gga att tat agc ttc agc ttc cac gtg gtc aaa gtg tac aac       484
   Arg Lys Gly Ile Tyr Ser Phe Ser Phe His Val Val Lys Val Tyr Asn
       140                 145                 150
   aga caa act atc cag gtc agc tta atg cag aat ggc tac ccg gtg atc       532
   Arg Gln Thr Ile Gln Val Ser Leu Met Gln Asn Gly Tyr Pro Val Ile
   155                 160                 165                 170
   tct gca ttt gcc gga gac cag gat gtt acc agg gaa gca gcc agc aat       580
   Ser Ala Phe Ala Gly Asp Gln Asp Val Thr Arg Glu Ala Ala Ser Asn
                   175                 180                 185
   ggt gtt ctg ctg ctc atg gaa aga gaa gac aaa gtt cat ctc aaa cta       628
   Gly Val Leu Leu Leu Met Glu Arg Glu Asp Lys Val His Leu Lys Leu
               190                 195                 200
   gag aga ggc aac ctc atg gga ggc tgg aaa tac tcc aca ttc tcg ggc       676
   Glu Arg Gly Asn Leu Met Gly Gly Trp Lys Tyr Ser Thr Phe Ser Gly
           205                 210                 215
   ttc ttg gtt ttt cct cta tag actcagagcc accaggatga tgggaaggtt          727
   Phe Leu Val Phe Pro Leu
       220
   ttgatcagga cccagggatc tgccccctgt aacaccttga acttgtctgg ataggatggc     787
   ttgggcccac ctccatcaga ttattgctgt agaagaatga ctttcttcta aagctccagt     847
   attttctttg actattgaca attcctcggg aacctggcct ctaattagtt taagaagaca      907
   aggtcttaag gagaaatgaa attatcgatt tgagcaattt gtacccgtga ttgtaaagtc     967
   gatatcggat tttattgttg gaaccattgg cttaacttct catgtttgta cggtgtatct    1027
   tgtcctgatg acatagatgc tgctgaccct cagatggatt gcacgcttca gtcagggctt    1087
   aaagcaagag cccagcagag gaccacctaa ccagacagtc tttgacctgt gttctgtgtg    1147
   tgtgtagcct taagaaaaag aatggcatca ttttcattcc gtagcttttc cctagggtct    1207
   tgggggtctt gggagggagc tgggcattgg taacctgtcg aaaagtgctt tatcctgaga    1267
   agcaaatttt gcacgattgg actgcagttt ctgttttgta ccgtctgtga ttttcttttt    1327
   tcctcgggaa gctttccttt tcttcctcag gtttcactcc tcaaacctac ttagttttca    1387
   tgctgggggc tcggagagaa aaacaaaaca aaacaaaaca acaaaactta tgttcagtcc    1447
   ttgtatgaga ccaaacaaaa cagaacaaaa tctgcatact ttgttttgga taaaggaaac    1507
   caggaaaaaa aaaaaaagaa agcctatatt tcaaagtaag gacagcaaat agaacatcct    1567
   gcataacctg cataaccttc cctggagttc tctctcagtg cagcttcaaa cttgcaacgt    1627
   ggatttcatt tttttttttt tttaatttat tctctgggat cccttagtaa cctggcattg    1687
   ctcattgctc atgaatccct taacaatggc ggagctgacg cggcctaatc ctctctttgc    1747
   ttttcctctg gatatgtctc agtagtgagt tagatctgcc tctgagtggc cacacaatta    1807
   caagatgctt caaagaaaca ggaccagtgc ctcttcctcg agatcttcct ttgatctgtc    1867
   aaggagaggg ttgtgttgac tgatgctctg tggatgaaca tctggccaga agccacctgg    1927
   actggaaatc tcagcagtta aaggtactgg ctgctgtacc ccatgggtgg ttttttcaaa    1987
   ccctgcttct tttggcatct tgtgcaacag cactattaag tagcaactga gagacctggt    2047
   gcagtatttg tgtgccaagg aaacgtcact aatcccaaag caatcaaaga tgtgacctca    2107
   aactggaagt taatttgtcc tttgtgtaac aatgtaacca aaatattgat gataaaactc    2167
   ataatttaag attcagaata aatggatttg atgtc                               2202
<210> 31
   <211> 224
   <212> PRT
   <213> Mus musculus
<400> 31
   Met Pro Ala Pro Gly Gln Gly Pro Arg Gly Pro Leu Leu Ser Met Pro
   1               5                   10                  15
   Gly Arg Arg Gly Ala Leu Arg Glu Pro Ala Asp Phe Gly Ser Ser Leu
               20                  25                  30
   Gly Ala Val Leu Ala Leu Leu Leu Leu Leu Leu Pro Ala Cys Cys Pro
           35                  40                  45
   Val Arg Ala Gln Asn Asp Thr Glu Pro Ile Val Leu Glu Gly Lys Cys
       50                  55                  60
   Leu Val Val Cys Asp Ser Ser Pro Ser Gly Asp Gly Ala Val Thr Ser
   65                  70                  75                  80
   Ser Leu Gly Ile Ser Val Arg Ser Gly Ser Ala Lys Val Ala Phe Ser
                   85                  90                  95
   Ala Thr Arg Ser Thr Asn His Glu Pro Ser Glu Met Ser Asn Arg Thr
               100                 105                 110
   Met Thr Ile Tyr Phe Asp Gln Val Leu Val Asn Ile Gly Asn His Phe
           115                 120                 125
   Asp Leu Ala Ser Ser Ile Phe Val Ala Pro Arg Lys Gly Ile Tyr Ser
       130                 135                 140
   Phe Ser Phe His Val Val Lys Val Tyr Asn Arg Gln Thr Ile Gln Val
   145                 150                 155                 160
   Ser Leu Met Gln Asn Gly Tyr Pro Val Ile Ser Ala Phe Ala Gly Asp
                   165                 170                 175
   Gln Asp Val Thr Arg Glu Ala Ala Ser Asn Gly Val Leu Leu Leu Met
               180                 185                 190
   Glu Arg Glu Asp Lys Val His Leu Lys Leu Glu Arg Gly Asn Leu Met
           195                 200                 205
   Gly Gly Trp Lys Tyr Ser Thr Phe Ser Gly Phe Leu Val Phe Pro Leu
       210                 215                 220
<210> 32
   <211> 2750
   <212> DNA
   <213> Homo sapiens
<220>
   <221> CDS
   <222> (564)..(1238)
   <223> SF06, cDNA: NM_182511, Protein: NP_872317
<400> 32
   ggagcagaca cacagacccg ggccggaggc ccctcttcta gccctgcggg aaccggacag      60
   ttccccaact ggggactctg gaaccacagc tcctaaatca tcaaattctc aagctttttt     120
   tttccctctc ttcgtcccag ccatcccagt cttcttcttc tttttttttt ttttaactta     180
   ttgttttttt cgctcctgtc attatgaaag tggtcacgcc attcaatatt aagacttgga     240
   gggaattggg gaaagaaaag aaagaatcta aaagaagaga agcgaccggt gcttttaagg     300
   gtgtctaatt ttcaaaagag acgtctggga gtattttgct ctgggcgttt ggagcaactt     360
   cgcggacagc ggagctcgcc cagcatggat gttccaggtt cacaggcgcc tttcttctga     420
   gaacgaccct ggccttgaac gtcagagccg gggacgaagg cccccggagg ctgctgcgag     480
   ctccgcgcgt tccttcgcgc ccttccgcgc cgctcgcgcc ggcgccggcc tccacccccg     540
   cgcgccgcct cccaccagtc ccg atg cag gcg ccc ggc cgg ggg cca ctc ggg     593
                                 Met Gln Ala Pro Gly Arg Gly Pro Leu Gly
                                 1               5                   10
   ctg cgg ctg atg atg ccc ggg cgc cgg ggg gcg ctg cgc gag cct ggc      641
   Leu Arg Leu Met Met Pro Gly Arg Arg Gly Ala Leu Arg Glu Pro Gly
                   15                  20                  25
   ggc tgc gga tcc tgc ctg ggg gtg gcg ctg gcc ctg ctg ttg ctg cta      689
   Gly Cys Gly Ser Cys Leu Gly Val Ala Leu Ala Leu Leu Leu Leu Leu
               30                  35                  40
   ctg ccc gcc tgc tgc ccc gtg cgg gcg cag aac gac acg gag ccc atc      737
   Leu Pro Ala Cys Cys Pro Val Arg Ala Gln Asn Asp Thr Glu Pro Ile
           45                  50                  55
   gtg ctg gag ggc aag tgc ctg gtg gtg tgc gac tcc agc ccg tcg gcg      785
   Val Leu Glu Gly Lys Cys Leu Val Val Cys Asp Ser Ser Pro Ser Ala
       60                  65                  70
   gac ggc gcc gtc acc tcc tcc cta ggc atc tcc gtg cgc tcc ggc agc      833
   Asp Gly Ala Val Thr Ser Ser Leu Gly Ile Ser Val Arg Ser Gly Ser
   75                  80                  85                  90
   gcc aag gtg gcc ttc tcc gcc acg cgg agc acc aac cac gag ccg tcc      881
   Ala Lys Val Ala Phe Ser Ala Thr Arg Ser Thr Asn His Glu Pro Ser
                   95                  100                 105
   gag atg agc aac cgc acc atg acc atc tat ttc gac cag gta tta gta      929
   Glu Met Ser Asn Arg Thr Met Thr Ile Tyr Phe Asp Gln Val Leu Val
               110                 115                 120
   aat att ggc aac cac ttt gat ctt gct tcc agt ata ttt gta gca ccg      977
   Asn Ile Gly Asn His Phe Asp Leu Ala Ser Ser Ile Phe Val Ala Pro
           125                 130                 135
   aga aaa ggg att tat agc ttc agc ttc cac gtg gtc aaa gtg tat aac     1025
   Arg Lys Gly Ile Tyr Ser Phe Ser Phe His Val Val Lys Val Tyr Asn
       140                 145                 150
   aga caa acc atc cag gtc agt tta atg cag aat ggc tac cca gtg atc     1073
   Arg Gln Thr Ile Gln Val Ser Leu Met Gln Asn Gly Tyr Pro Val Ile
   155                 160                 165                 170
   tcg gcc ttt gca gga gac cag gat gtc acc aga gaa gct gct agc aat     1121
   Ser Ala Phe Ala Gly Asp Gln Asp Val Thr Arg Glu Ala Ala Ser Asn
                   175                 180                 185
   ggc gtg ctg ctg ctc atg gaa agg gaa gac aaa gtg cat ctc aaa ctt     1169
   Gly Val Leu Leu Leu Met Glu Arg Glu Asp Lys Val His Leu Lys Leu
               190                 195                 200
   gag aga ggc aac ctc atg ggg ggc tgg aaa tac tcc aca ttc tcg ggc     1217
   Glu Arg Gly Asn Leu Met Gly Gly Trp Lys Tyr Ser Thr Phe Ser Gly
           205                 210                 215
   ttc ttg gtg ttt cct cta taa acacagagcc ccctagatgg tgggggaatg        1268
   Phe Leu Val Phe Pro Leu
       220
   gcaaactgga cccaggactc cgccctttaa aacaccctga acttactgga attggacacc   1328
   ttgtttccaa cctccgtcag actgttgcag tagaagaatg atttcctttg aaacctccag   1388
   tacttttgtt tttgtttttt ggaatactga caattcctcg ggaacctggc ctctaattag   1448
   ttttagatga caaggtctta aggagaaatg aaattatcga tttgagcaat ttgtacctgt   1508
   gattgtaaag tcaatatcgg attttattgt tgggaccatg gacctctttt gtttgtatgt   1568
   tgtattgtcg tcccaacgga aggagagctc ctgactccag gatgggctgc aggttgcagt 1628
   cagggcttga agtaggagcc cagcaaagaa ccacctgctg gacagtcctt gacatgtgtt 1688
   ctgtgtgtgt ctgtatagcc ttaagaaaaa gaatggcttc actttcattc tgtattcttc 1748
   cccccaccat gtggctggga ggacttggga gggggatggg gacattggga acctgtcaag 1808
   aagtgcttta tccagagaag caaattttgc acgattggac tgcaattttt gttttgtatt 1868
   gtttgtgttt tttcttgaaa agctttactt ttctttccac actcagctct ccctcctcaa 1928
   ccccactttt atttttcttg ctggggttga ggagagaaaa tatagaattc ctggataaga 1988
   ccaaacaaaa caaaacatta aaatacctgt atgttttgtt ttagacgaga ccaaactaaa 2048
   caaaaagtat ctgtttatca aagtaaaagt aacacaatgg acaattctgc ttattctctc 2108
   aaagagattc taagatgcac ctttagaact attaatagca acctgcattt ttttttaatt 2168
   tatacttcag aatcctttaa gaacctggtg ttcctgagtg gtcctgaatc atataagttg 2228
   gtaatggaag ctgtaatgac caagtcccct aaacatacta tgtctttgcc acgtgtgctg 2288
   tgacttctct gtgggtgatt taatttattt ggatccacct ctgagtgagc gcacagtgat 2348
   caggtgcttc aaagccaaca gaccagctcc tcttcctccg gatcctcttt tgatctgccc 2408
   aggaaaggga tgcattgaca ctctcctgca tgcacctggc gagaagccac ctgaaagtca 2468
   ctgtggttaa agatattggt ggaggtaccc caggagcact gttacaaatc cttcttgttt 2528
   tggcatctcg tacaacatta ttaagacaca gctgagagtt gatgggtgtg taatgcatat 2588
   gccaaggaaa tgtcactaat cccaaagcaa tcaaaaagga gacctcaaac cagatgttaa 2648
   tttgttcttt gtgtaacaat gtaaccaaaa tattgatgat aaaagtcata atttaagatt 2708
   cagaataaat gggtttgatg tctggcaaaa aaaaaaaaaa aa 2750
<210> 33
   <211> 224
   <212> PRT
   <213> Homo sapiens
<400> 33
   Met Gln Ala Pro Gly Arg Gly Pro Leu Gly Leu Arg Leu Met Met Pro
   1               5                   10                  15
   Gly Arg Arg Gly Ala Leu Arg Glu Pro Gly Gly Cys Gly Ser Cys Leu
               20                  25                  30
   Gly Val Ala Leu Ala Leu Leu Leu Leu Leu Leu Pro Ala Cys Cys Pro
           35                  40                  45
   Val Arg Ala Gln Asn Asp Thr Glu Pro Ile Val Leu Glu Gly Lys Cys
       50                  55                  60
   Leu Val Val Cys Asp Ser Ser Pro Ser Ala Asp Gly Ala Val Thr Ser
   65                  70                  75                      80
   Ser Leu Gly Ile Ser Val Arg Ser Gly Ser Ala Lys Val Ala Phe Ser
                   85                  90                  95
   Ala Thr Arg Ser Thr Asn His Glu Pro Ser Glu Met Ser Asn Arg Thr
               100                 105                 110
   Met Thr Ile Tyr Phe Asp Gln Val Leu Val Asn Ile Gly Asn His Phe
           115                 120                 125
   Asp Leu Ala Ser Ser Ile Phe Val Ala Pro Arg Lys Gly Ile Tyr Ser
       130                 135                 140
   Phe Ser Phe His Val Val Lys Val Tyr Asn Arg Gln Thr Ile Gln Val
   145                 150                 155                     160
   Ser Leu Met Gln Asn Gly Tyr Pro Val Ile Ser Ala Phe Ala Gly Asp
                   165                 170                 175
   Gln Asp Val Thr Arg Glu Ala Ala Ser Asn Gly Val Leu Leu Leu Met
               180                 185                 190
   Glu Arg Glu Asp Lys Val His Leu Lys Leu Glu Arg Gly Asn Leu Met
           195                 200                 205
   Gly Gly Trp Lys Tyr Ser Thr Phe Ser Gly Phe Leu Val Phe Pro Leu
       210                 215                 220
<210> 34
   <211> 1542
   <212> DNA
   <213> Mus musculus
<220>
   <221> CDS
   <222> (131)..(1258)
   <223> SF07, cDNA: NM_026840, Protein: NP_081116
<400> 34
   cccagtgaaa ccgaatcctc cagctctggt gtcccaggcg ccgcctcccc tctgcgcccc      60
   tgccctgccc tggacagcct gtcgcgccgt ctgcagtcct gaactattct gcaggtcacc     120
   agccctgaag atg aag ttt tgg ctg ctg ctc gga ctt ctg ttg cta cac        169
              Met Lys Phe Trp Leu Leu Leu Gly Leu Leu Leu Leu His
              1               5                   10
   gaa gcg ctg gaa gat gtt gct ggc cag cat tct cct aag aac aag cgt       217
   Glu Ala Leu Glu Asp Val Ala Gly Gln His Ser Pro Lys Asn Lys Arg
       15                  20                  25
   cca aag gag caa gga gaa aac aga atc aaa cca acc aac aaa aag gcc       265
   Pro Lys Glu Gln Gly Glu Asn Arg Ile Lys Pro Thr Asn Lys Lys Ala
   30                  35                  40                  45
   aaa ccc aag att cct aag gta aag gac agg gac tcc act gac tca acc       313
   Lys Pro Lys Ile Pro Lys Val Lys Asp Arg Asp Ser Thr Asp Ser Thr
                   50                  55                  60
   gca aag agc cag tcc atc atg atg caa gcg atg ggc aac ggt cgc ttc      361
   Ala Lys Ser Gln Ser Ile Met Met Gln Ala Met Gly Asn Gly Arg Phe
               65                  70                  75
   cag agg cct gct gcc aca gtg agt ctc ctg gca ggg caa act cta gag      409
   Gln Arg Pro Ala Ala Thr Val Ser Leu Leu Ala Gly Gln Thr Leu Glu
           80                  85                  90
   cta cga tgt aag gga agc aaa gtg gag tgg agt tac ccc gcc tac ttg      457
   Leu Arg Cys Lys Gly Ser Lys Val Glu Trp Ser Tyr Pro Ala Tyr Leu
       95                  100                 105
   gac acc ttc aag gac tcc cgc ctc act gtg aag cag agt gaa cgc tat      505
   Asp Thr Phe Lys Asp Ser Arg Leu Thr Val Lys Gln Ser Glu Arg Tyr
   110                 115                 120                 125
   ggg cag ttg act ctg gtc aac tcc acc gcg gcc gac acc ggt gaa ttc      553
   Gly Gln Leu Thr Leu Val Asn Ser Thr Ala Ala Asp Thr Gly Glu Phe
                   130                 135                 140
   agc tgc tgg gag caa ctg tgc aat ggc tac atc tgc aga cgg gat gaa      601
   Ser Cys Trp Glu Gln Leu Cys Asn Gly Tyr Ile Cys Arg Arg Asp Glu
               145                 150                 155
   gcc aaa aca ggc tcc acc tat atc ttc ttc aca gag aaa gga gag ctg      649
   Ala Lys Thr Gly Ser Thr Tyr Ile Phe Phe Thr Glu Lys Gly Glu Leu
           160                 165                 170
   ttt gtg cct tct ccc agt tac ttt gat gtt gtc tac ctg aac ccg gac      697
   Phe Val Pro Ser Pro Ser Tyr Phe Asp Val Val Tyr Leu Asn Pro Asp
       175                 180                 185
   aga caa gct gtg gtt cct tgt cga gtg aca gcc cca tca gcc aaa gtc      745
   Arg Gln Ala Val Val Pro Cys Arg Val Thr Ala Pro Ser Ala Lys Val
   190                 195                 200                 205
   acg ctc cac agg gag ttt ccc gcc aaa gaa atc cct gcc aat ggc acg      793
   Thr Leu His Arg Glu Phe Pro Ala Lys Glu Ile Pro Ala Asn Gly Thr
                   210                 215                 220
   gac att gtg tac gac atg aag aga ggt ttc gtg tac ctt cag cct cat      841
   Asp Ile Val Tyr Asp Met Lys Arg Gly Phe Val Tyr Leu Gln Pro His
               225                 230                 235
   tcc gat cac cag ggt gtg gtc tac tgc aaa gcg gaa gcc ggg ggc aag      889
   Ser Asp His Gln Gly Val Val Tyr Cys Lys Ala Glu Ala Gly Gly Lys
           240                 245                 250
   tct cag atc tca gtc aag tat cag ctg ctc tat gta gag gtt cct agt      937
   Ser Gln Ile Ser Val Lys Tyr Gln Leu Leu Tyr Val Glu Val Pro Ser
       255                 260                 265
   ggc cct cca tca aca acc atc ttg gcc tcc tct aac aaa gtg agg ggc      985
   Gly Pro Pro Ser Thr Thr Ile Leu Ala Ser Ser Asn Lys Val Arg Gly
   270                 275                 280                 285
   ggt gat gac atc agc gtg ctc tgc act gtc ctc ggg gag cct gat gtg     1033
   Gly Asp Asp Ile Ser Val Leu Cys Thr Val Leu Gly Glu Pro Asp Val
                   290                 295                 300
   gag gtt gaa ttc agg tgg ctc ttt cct ggg cag aag gac gaa agg cct     1081
   Glu Val Glu Phe Arg Trp Leu Phe Pro Gly Gln Lys Asp Glu Arg Pro
               305                 310                 315
   gtg acc atc cag gac acc tgg aga ctg att cac aga gga ctg gga cac     1129
   Val Thr Ile Gln Asp Thr Trp Arg Leu Ile His Arg Gly Leu Gly His
           320                 325                 330
   acc aca aga atc tcc cag agt gtc att atc gtg gaa gac ttt gag acc     1177
   Thr Thr Arg Ile Ser Gln Ser Val Ile Ile Val Glu Asp Phe Glu Thr
       335                 340                 345
   att gat gcg ggc tac tac ata tgc aca gct cag aat ctc cga gga cag     1225
   Ile Asp Ala Gly Tyr Tyr Ile Cys Thr Ala Gln Asn Leu Arg Gly Gln
   350                 355                 360                 365
   acc aca gta gcg acc act gtt gag ttt tcc tga ttggaaagtg aagagtagtg   1278
   Thr Thr Val Ala Thr Thr Val Glu Phe Ser
                   370                 375
   gaccaatggg atgcccatct gcacacacag cttccaggtg ctttatagga ggccaagggc   1338
   caacccctgc cagtgggtca gacagacatc cgaattaaaa ggaagtcact agtctattaa  1398
   tagaagtata aactttccta actaaagtat gtattttgac tcagccatgt ttctactttt   1458
   tatactgaga aaacatgtca acaactttgt atcaatcgtt tctattaaat gagcaagatt   1518
   ttataaaaaa aaaaaaaaaa aaaa                                          1542
<210> 35
   <211> 375
   <212> PRT
   <213> Mus musculus
<400> 35
   Met Lys Phe Trp Leu Leu Leu Gly Leu Leu Leu Leu His Glu Ala Leu
   1 5 10 15
   Glu Asp Val Ala Gly Gln His Ser Pro Lys Asn Lys Arg Pro Lys Glu
   20 25 30
   Gln Gly Glu Asn Arg Ile Lys Pro Thr Asn Lys Lys Ala Lys Pro Lys
   35 40 45
   Ile Pro Lys Val Lys Asp Arg Asp Ser Thr Asp Ser Thr Ala Lys Ser
   50 55 60
   Gln Ser Ile Met Met Gln Ala Met Gly Asn Gly Arg Phe Gln Arg Pro
   65 70 75 80
   Ala Ala Thr Val Ser Leu Leu Ala Gly Gln Thr Leu Glu Leu Arg Cys
   85 90 95
   Lys Gly Ser Lys Val Glu Trp Ser Tyr Pro Ala Tyr Leu Asp Thr Phe
   100 105 110
   Lys Asp Ser Arg Leu Thr Val Lys Gln Ser Glu Arg Tyr Gly Gln Leu
   115 120 125
   Thr Leu Val Asn Ser Thr Ala Ala Asp Thr Gly Glu Phe Ser Cys Trp
   130 135 140
   Glu Gln Leu Cys Asn Gly Tyr Ile Cys Arg Arg Asp Glu Ala Lys Thr
   145 150 155 160
   Gly Ser Thr Tyr Ile Phe Phe Thr Glu Lys Gly Glu Leu Phe Val Pro
   165 170 175
   Ser Pro Ser Tyr Phe Asp Val Val Tyr Leu Asn Pro Asp Arg Gln Ala
   180 185 190
   Val Val Pro Cys Arg Val Thr Ala Pro Ser Ala Lys Val Thr Leu His
   195 200 205
   Arg Glu Phe Pro Ala Lys Glu Ile Pro Ala Asn Gly Thr Asp Ile Val
   210 215 220
   Tyr Asp Met Lys Arg Gly Phe Val Tyr Leu Gln Pro His Ser Asp His
   225 230 235 240
   Gln Gly Val Val Tyr Cys Lys Ala Glu Ala Gly Gly Lys Ser Gln Ile
   245 250 255
   Ser Val Lys Tyr Gln Leu Leu Tyr Val Glu Val Pro Ser Gly Pro Pro
   260 265 270
   Ser Thr Thr Ile Leu Ala Ser Ser Asn Lys Val Arg Gly Gly Asp Asp
   275 280 285
   Ile Ser Val Leu Cys Thr Val Leu Gly Glu Pro Asp Val Glu Val Glu
   290 295 300
   Phe Arg Trp Leu Phe Pro Gly Gln Lys Asp Glu Arg Pro Val Thr Ile
   305 310 315 320
   Gln Asp Thr Trp Arg Leu Ile His Arg Gly Leu Gly His Thr Thr Arg
   325 330 335
   Ile Ser Gln Ser Val Ile Ile Val Glu Asp Phe Glu Thr Ile Asp Ala
   340 345 350
   Gly Tyr Tyr Ile Cys Thr Ala Gln Asn Leu Arg Gly Gln Thr Thr Val
   355 360 365
   Ala Thr Thr Val Glu Phe Ser
   370 375
<210> 36
   <211> 1502
   <212> DNA
   <213> Homo sapiens
<220>
   <221> CDS
   <222> (62)..(1189)
   <223> SF07, cDNA: NM_006207, Protein: NP_006198
<400> 36
   cctgcgtccc cgccccgcgc agccgccgcg ctcctgcgct ccgaggtccg aggttcccga       60
   g atg aag gtc tgg ctg ctg ctt ggt ctt ctg ctg gtg cac gaa gcg ctg     109
     Met Lys Val Trp Leu Leu Leu Gly Leu Leu Leu Val His Glu Ala Leu
     1               5                   10                  15
   gag gat gtt act ggc caa cac ctt ccc aag aac aag cgt cca aaa gaa       157
   Glu Asp Val Thr Gly Gln His Leu Pro Lys Asn Lys Arg Pro Lys Glu
               20                  25                  30
   cca gga gag aat aga atc aaa cct acc aac aag aag gtg aag ccc aaa       205
   Pro Gly Glu Asn Arg Ile Lys Pro Thr Asn Lys Lys Val Lys Pro Lys
           35                  40                  45
   att cct aaa atg aag gac agg gac tca gcc aat tca gca cca aag acg       253
   Ile Pro Lys Met Lys Asp Arg Asp Ser Ala Asn Ser Ala Pro Lys Thr
       50                  55                  60
   cag tct atc atg atg caa gtg ctg gat aaa ggt cgc ttc cag aaa ccc       301
   Gln Ser Ile Met Met Gln Val Leu Asp Lys Gly Arg Phe Gln Lys Pro
   65                  70                  75                  80
   gcc gct acc ctg agt ctg ctg gcg ggg caa act gta gag ctt cga tgt       349
   Ala Ala Thr Leu Ser Leu Leu Ala Gly Gln Thr Val Glu Leu Arg Cys
                   85                  90                  95
   aaa ggg agt aga att ggg tgg agc tac cct gcg tat ctg gac acc ttt       397
   Lys Gly Ser Arg Ile Gly Trp Ser Tyr Pro Ala Tyr Leu Asp Thr Phe
               100                 105                 110
   aag gat tct cgc ctc agc gtc aag cag aat gag cgc tac ggc cag ttg       445
   Lys Asp Ser Arg Leu Ser Val Lys Gln Asn Glu Arg Tyr Gly Gln Leu
           115                 120                 125
   act ctg gtc aac tcc acc tcg gca gac aca ggt gaa ttc agc tgc tgg       493
   Thr Leu Val Asn Ser Thr Ser Ala Asp Thr Gly Glu Phe Ser Cys Trp
       130                 135                 140
   gtg cag ctc tgc agc ggc tac atc tgc agg aag gac gag gcc aaa acg       541
   Val Gln Leu Cys Ser Gly Tyr Ile Cys Arg Lys Asp Glu Ala Lys Thr
   145                 150                 155                 160
   ggc tcc acc tac atc ttt ttt aca gag aaa gga gaa ctc ttt gta cct       589
   Gly Ser Thr Tyr Ile Phe Phe Thr Glu Lys Gly Glu Leu Phe Val Pro
                   165                 170                 175
   tct ccc agc tac ttc gat gtt gtc tac ttg aac ccg gac aga cag gct       637
   Ser Pro Ser Tyr Phe Asp Val Val Tyr Leu Asn Pro Asp Arg Gln Ala
               180                 185                 190
   gtg gtt cct tgt cgg gtg acc gtg ctg tcg gcc aaa gtc acg ctc cac       685
   Val Val Pro Cys Arg Val Thr Val Leu Ser Ala Lys Val Thr Leu His
           195                 200                 205
   agg gaa ttc cca gcc aag gag atc cca gcc aat gga acg gac att gtt       733
   Arg Glu Phe Pro Ala Lys Glu Ile Pro Ala Asn Gly Thr Asp Ile Val
       210                 215                 220
   tat gac atg aag cgg ggc ttt gtg tat ctg caa cct cat tcc gag cac       781
   Tyr Asp Met Lys Arg Gly Phe Val Tyr Leu Gln Pro His Ser Glu His
   225                 230                 235                 240
   cag ggt gtg gtt tac tgc agg gcg gag gcc ggg ggc aga tct cag atc       829
   Gln Gly Val Val Tyr Cys Arg Ala Glu Ala Gly Gly Arg Ser Gln Ile
                   245                 250                 255
   tcc gtc aag tac cag ctg ctc tac gtg gcg gtt ccc agt ggc cct ccc      877
   Ser Val Lys Tyr Gln Leu Leu Tyr Val Ala Val Pro Ser Gly Pro Pro
               260                 265                 270
   tca aca acc atc ttg gct tct tca aac aaa gtg aaa agt ggg gac gac      925
   Ser Thr Thr Ile Leu Ala Ser Ser Asn Lys Val Lys Ser Gly Asp Asp
           275                 280                 285
   atc agt gtg ctc tgc act gtc ctg ggg gag ccc gat gtg gag gtg gag      973
   Ile Ser Val Leu Cys Thr Val Leu Gly Glu Pro Asp Val Glu Val Glu
       290                 295                 300
   ttc acc tgg atc ttc cca ggg cag aag gat gaa agg cct gtg acg atc     1021
   Phe Thr Trp Ile Phe Pro Gly Gln Lys Asp Glu Arg Pro Val Thr Ile
   305                 310                 315                 320
   caa gac act tgg agg ttg atc cac aga gga ctg gga cac acc acg aga     1069
   Gln Asp Thr Trp Arg Leu Ile His Arg Gly Leu Gly His Thr Thr Arg
                   325                 330                 335
   atc tcc cag agt gtc att aca gtg gaa gac ttc gag acg att gat gca     1117
   Ile Ser Gln Ser Val Ile Thr Val Glu Asp Phe Glu Thr Ile Asp Ala
               340                 345                 350
   gga tat tac att tgc act gct cag aat ctt caa gga cag acc aca gta     1165
   Gly Tyr Tyr Ile Cys Thr Ala Gln Asn Leu Gln Gly Gln Thr Thr Val
           355                 360                 365
   gct acc act gtt gag ttt tcc tga cttggaaaag gaaatgtaat gaacttatgg    1219
   Ala Thr Thr Val Glu Phe Ser
       370                 375
   aaagcccatt tgtgtacaca gtcagctttg gggttccttt tattagtgct ttgccagagg   1279
   ctgatgtcaa gcaccacacc ccaaccccag cgtctcgtga gtccgaccca gacatccaaa   1339
   ctaaaaggaa gtcatccagt ctattcacag aagtgttaac ttttctaaca gaaagcatga   1399
   ttttgattgc ttacctacat acgtgttcct agtttttata catgtgtaaa caattttata   1459
   taatcaatca tttctattaa atgagcacgt ttttgtaaaa aat                      1502
<210> 37
   <211> 375
   <212> PRT
   <213> Homo sapiens
<400> 37
   Met Lys Val Trp Leu Leu Leu Gly Leu Leu Leu Val His Glu Ala Leu
   1 5 10 15
   Glu Asp Val Thr Gly Gln His Leu Pro Lys Asn Lys Arg Pro Lys Glu
   20 25 30
   Pro Gly Glu Asn Arg Ile Lys Pro Thr Asn Lys Lys Val Lys Pro Lys
   35 40 45
   Ile Pro Lys Met Lys Asp Arg Asp Ser Ala Asn Ser Ala Pro Lys Thr
   50 55 60
   Gln Ser Ile Met Met Gln Val Leu Asp Lys Gly Arg Phe Gln Lys Pro
   65 70 75 80
   Ala Ala Thr Leu Ser Leu Leu Ala Gly Gln Thr Val Glu Leu Arg Cys
   85 90 95
   Lys Gly Ser Arg Ile Gly Trp Ser Tyr Pro Ala Tyr Leu Asp Thr Phe
   100 105 110
   Lys Asp Ser Arg Leu Ser Val Lys Gln Asn Glu Arg Tyr Gly Gln Leu
   115 120 125
   Thr Leu Val Asn Ser Thr Ser Ala Asp Thr Gly Glu Phe Ser Cys Trp
   130 135 140
   Val Gln Leu Cys Ser Gly Tyr Ile Cys Arg Lys Asp Glu Ala Lys Thr
   145 150 155 160
   Gly Ser Thr Tyr Ile Phe Phe Thr Glu Lys Gly Glu Leu Phe Val Pro
   165 170 175
   Ser Pro Ser Tyr Phe Asp Val Val Tyr Leu Asn Pro Asp Arg Gln Ala
   180 185 190
   Val Val Pro Cys Arg Val Thr Val Leu Ser Ala Lys Val Thr Leu His
   195 200 205
   Arg Glu Phe Pro Ala Lys Glu Ile Pro Ala Asn Gly Thr Asp Ile Val
   210 215 220
   Tyr Asp Met Lys Arg Gly Phe Val Tyr Leu Gln Pro His Ser Glu His
   225 230 235 240
   Gln Gly Val Val Tyr Cys Arg Ala Glu Ala Gly Gly Arg Ser Gln Ile
   245 250 255
   Ser Val Lys Tyr Gln Leu Leu Tyr Val Ala Val Pro Ser Gly Pro Pro
   260 265 270
   Ser Thr Thr Ile Leu Ala Ser Ser Asn Lys Val Lys Ser Gly Asp Asp
   275 280 285
   Ile Ser Val Leu Cys Thr Val Leu Gly Glu Pro Asp Val Glu Val Glu
   290 295 300
   Phe Thr Trp Ile Phe Pro Gly Gln Lys Asp Glu Arg Pro Val Thr Ile
   305 310 315 320
   Gln Asp Thr Trp Arg Leu Ile His Arg Gly Leu Gly His Thr Thr Arg
   325 330 335
   Ile Ser Gln Ser Val Ile Thr Val Glu Asp Phe Glu Thr Ile Asp Ala
   340 345 350
   Gly Tyr Tyr Ile Cys Thr Ala Gln Asn Leu Gln Gly Gln Thr Thr Val
   355 360 365
   Ala Thr Thr Val Glu Phe Ser
   370 375
<210> 38
   <211> 2379
   <212> DNA
   <213> Mus musculus
<220>
   <221> CDS
   <222> (69)..(2237)
   <223> SF08, cDNA: NM_019696, Protein: NP_062670
<400> 38
   agacgcccgc aggaagaaga gaacccacca agaacctcgg cacagcgcgc tcgtcgaccc      60
   ttcccgct atg tgg ggt ctc ttg ctc gca gtg acc gcc ttt gcg cct tcc      110
            Met Trp Gly Leu Leu Leu Ala Val Thr Ala Phe Ala Pro Ser
            1               5                   10
   gtc ggt ctg ggg ctg ggg gct ccc agc gcc tca gtg ccg ggc ctg gcg      158
   Val Gly Leu Gly Leu Gly Ala Pro Ser Ala Ser Val Pro Gly Leu Ala
   15                  20                  25                  30
   ccg ggc tca acc ctg gct cca cac agc agc gtt gca cag ccg tcc aca      206
   Pro Gly Ser Thr Leu Ala Pro His Ser Ser Val Ala Gln Pro Ser Thr
                   35                  40                  45
   aag gca aat gag acc tca gaa cgg cat gtc cgg ctt cga gtc atc aag      254
   Lys Ala Asn Glu Thr Ser Glu Arg His Val Arg Leu Arg Val Ile Lys
               50                  55                  60
   aaa aaa aag atc gtt gtc aag aag cga aag aag cta agg cac cct ggt      302
   Lys Lys Lys Ile Val Val Lys Lys Arg Lys Lys Leu Arg His Pro Gly
           65                  70                  75
   ccc ttg ggg act gct agg cct gtg gtg ccc act cac cca gca aag acc      350
   Pro Leu Gly Thr Ala Arg Pro Val Val Pro Thr His Pro Ala Lys Thr
       80                  85                  90
   ctc act ctc cct gag aaa caa gaa cca gga tgt ccc cct cta ggc ttg      398
   Leu Thr Leu Pro Glu Lys Gln Glu Pro Gly Cys Pro Pro Leu Gly Leu
   95                  100                 105                 110
   gag tcc ttg agg gtt tca gat agc cag ctg gag gcc tcc agc agc cag      446
   Glu Ser Leu Arg Val Ser Asp Ser Gln Leu Glu Ala Ser Ser Ser Gln
                   115                 120                 125
   tcc ttt ggt ctt gga gca cac cga gga cgg ctc aat atc cag tca ggt      494
   Ser Phe Gly Leu Gly Ala His Arg Gly Arg Leu Asn Ile Gln Ser Gly
               130                 135                 140
   ctg gag gac ggt gac ctg tat gat ggc gct tgg tgc gct gag caa caa      542
   Leu Glu Asp Gly Asp Leu Tyr Asp Gly Ala Trp Cys Ala Glu Gln Gln
           145                 150                 155
   gac act gaa cct tgg ctt cag gtg gat gca aag aat ccc gtc cgc ttc      590
   Asp Thr Glu Pro Trp Leu Gln Val Asp Ala Lys Asn Pro Val Arg Phe
       160                 165                 170
   gca gga att gtt aca cag ggc aga aac tct gtg tgg agg tat gac tgg      638
   Ala Gly Ile Val Thr Gln Gly Arg Asn Ser Val Trp Arg Tyr Asp Trp
   175                 180                 185                 190
   gtc aca tca ttc aag gtc cag ttc agc aat gac agc cag acc tgg tgg      686
   Val Thr Ser Phe Lys Val Gln Phe Ser Asn Asp Ser Gln Thr Trp Trp
                   195                 200                 205
   aag agt agg aac agt act gga atg gac ata gta ttt cct gcc aat tca      734
   Lys Ser Arg Asn Ser Thr Gly Met Asp Ile Val Phe Pro Ala Asn Ser
               210                 215                 220
   gac gca gag acc cca gtg ttg aac ctt ctg cca gag cct cag gtg gct      782
   Asp Ala Glu Thr Pro Val Leu Asn Leu Leu Pro Glu Pro Gln Val Ala
           225                 230                 235
   cga ttc att cgc ctg ctg cct cag acc tgg ttt cag gga ggt gta cct      830
   Arg Phe Ile Arg Leu Leu Pro Gln Thr Trp Phe Gln Gly Gly Val Pro
       240                 245                 250
   tgc ctc cgg gca gag atc ctg gcc tgc cca gtc tca gat cct aat gac      878
   Cys Leu Arg Ala Glu Ile Leu Ala Cys Pro Val Ser Asp Pro Asn Asp
   255                 260                 265                 270
   ctg ttc cct gag gcc cac aca ctg gga tct tcg aac tct ttg gac ttc      926
   Leu Phe Pro Glu Ala His Thr Leu Gly Ser Ser Asn Ser Leu Asp Phe
                   275                 280                 285
   cgg cat cac aat tat aaa gct atg aga aag ctg atg aaa cag gtg aat      974
   Arg His His Asn Tyr Lys Ala Met Arg Lys Leu Met Lys Gln Val Asn
               290                 295                 300
   gag cag tgc ccc aac atc acg cgc atc tac agc atc ggg aag agc cac     1022
   Glu Gln Cys Pro Asn Ile Thr Arg Ile Tyr Ser Ile Gly Lys Ser His
           305                 310                 315
   cag ggt ttg aag ctg tat gtg atg gaa atg tca gac cat cct ggg gag     1070
   Gln Gly Leu Lys Leu Tyr Val Met Glu Met Ser Asp His Pro Gly Glu
       320                 325                 330
   cat gag ctg ggc gag ccc gag gtc cgc tac gtg gct gga atg cat ggg     1118
   His Glu Leu Gly Glu Pro Glu Val Arg Tyr Val Ala Gly Met His Gly
   335                 340                 345                 350
   aat gag gcc ctg ggg cgg gag ttg ctt ctg ctt ttg atg cag ttc tta     1166
   Asn Glu Ala Leu Gly Arg Glu Leu Leu Leu Leu Leu Met Gln Phe Leu
                   355                 360                 365
   tgc cat gag ttc ctg cga ggg gac ccg cga gtg act cgg ctg ctc act     1214
   Cys His Glu Phe Leu Arg Gly Asp Pro Arg Val Thr Arg Leu Leu Thr
               370                 375                 380
   gag aca cga atc cat cta ttg ccc tcc atg aat cct gat ggc tat gag     1262
   Glu Thr Arg Ile His Leu Leu Pro Ser Met Asn Pro Asp Gly Tyr Glu
           385                 390                 395
   act gcc tac cac agg ggc tca gag ctg gtg ggc tgg gca gag ggc cgc     1310
   Thr Ala Tyr His Arg Gly Ser Glu Leu Val Gly Trp Ala Glu Gly Arg
       400                 405                 410
   tgg acc cac cag ggc att gac ctt aac cac aat ttt gct gac ctc aac     1358
   Trp Thr His Gln Gly Ile Asp Leu Asn His Asn Phe Ala Asp Leu Asn
   415                 420                 425                 430
   aca caa ctg tgg tat gca gag gat gat gga ctg gta ccc gac act gtc     1406
   Thr Gln Leu Trp Tyr Ala Glu Asp Asp Gly Leu Val Pro Asp Thr Val
                   435                 440                 445
   ccc aac cat cac ctg cca ctg cct acc tac tat aca ttg ccc aat gcc      1454
   Pro Asn His His Leu Pro Leu Pro Thr Tyr Tyr Thr Leu Pro Asn Ala
               450                 455                 460
   acc gtg gct cct gaa acg tgg gca gtg atc aaa tgg atg aag cgc atc      1502
   Thr Val Ala Pro Glu Thr Trp Ala Val Ile Lys Trp Met Lys Arg Ile
           465                 470                 475
   ccg ttt gtg ctg agt gcc aac ctc cat ggg ggc gag ctt gtg gtg tcc      1550
   Pro Phe Val Leu Ser Ala Asn Leu His Gly Gly Glu Leu Val Val Ser
       480                 485                 490
   tat cct ttc gac atg act cgg act ccg tgg gct gct cgt gaa ctc act      1598
   Tyr Pro Phe Asp Met Thr Arg Thr Pro Trp Ala Ala Arg Glu Leu Thr
   495                 500                 505                 510
   ccc aca cca gat gat gct gtc ttc cgc tgg ctc agc act gtc tat gct      1646
   Pro Thr Pro Asp Asp Ala Val Phe Arg Trp Leu Ser Thr Val Tyr Ala
                   515                 520                 525
   ggc acg aat agg gcc atg cag gat acc gac cgc cga cct tgt cat agc      1694
   Gly Thr Asn Arg Ala Met Gln Asp Thr Asp Arg Arg Pro Cys His Ser
               530                 535                 540
   cag gac ttc tcc ttg cat ggc aac gtc atc aat gga gcc gac tgg cac      1742
   Gln Asp Phe Ser Leu His Gly Asn Val Ile Asn Gly Ala Asp Trp His
           545                 550                 555
   aca gtt cct ggg agc atg aac gac ttc agc tac cta cac acc aat tgc      1790
   Thr Val Pro Gly Ser Met Asn Asp Phe Ser Tyr Leu His Thr Asn Cys
       560                 565                 570
   ttt gag gtc aca gtg gag ctg tcc tgt gac aag ttc cct cat gag aag      1838
   Phe Glu Val Thr Val Glu Leu Ser Cys Asp Lys Phe Pro His Glu Lys
   575                 580                 585                 590
   gag ctg cct cag gag tgg gaa aac aac aaa gat gct ctt ctc acc tac      1886
   Glu Leu Pro Gln Glu Trp Glu Asn Asn Lys Asp Ala Leu Leu Thr Tyr
                   595                 600                 605
   ctg gag cag gtg cgc atg ggc att act gga gtt gtc cgg gat aaa gac      1934
   Leu Glu Gln Val Arg Met Gly Ile Thr Gly Val Val Arg Asp Lys Asp
               610                 615                 620
   aca gag ctc ggc att gcg gat gct gtc att gcc gtg gag ggc att aac      1982
   Thr Glu Leu Gly Ile Ala Asp Ala Val Ile Ala Val Glu Gly Ile Asn
           625                 630                 635
   cac gat gtt aca aca gct tgg ggc gga gat tac tgg cgg ctg ctg aca      2030
   His Asp Val Thr Thr Ala Trp Gly Gly Asp Tyr Trp Arg Leu Leu Thr
       640                 645                 650
   cct ggg gac tat gtg gtg aca gcc agt gct gag ggt tac cat aca gtc      2078
   Pro Gly Asp Tyr Val Val Thr Ala Ser Ala Glu Gly Tyr His Thr Val
   655                 660                 665                 670
   aga caa cac tgt cag gtc acc ttt gaa gag ggc cct gtt ccc tgc aat      2126
   Arg Gln His Cys Gln Val Thr Phe Glu Glu Gly Pro Val Pro Cys Asn
                   675                 680                 685
   ttc cta ctc acc aag act ccc aaa gag agg ctt cga gaa ctg ttg gca      2174
   Phe Leu Leu Thr Lys Thr Pro Lys Glu Arg Leu Arg Glu Leu Leu Ala
               690                 695                 700
   aca cga ggg aag ttg ccc cca gac ctt cgg agg aag ctg gag cgg ctg      2222
   Thr Arg Gly Lys Leu Pro Pro Asp Leu Arg Arg Lys Leu Glu Arg Leu
           705                 710                 715
   agg gga cag aag taa cgtcttcagc tgaagagagc cacatccttg gacaggctgg     2277
   Arg Gly Gln Lys
       720
   acctgtccag aactgaagga ggagggggaa gagagaggga cggggtagaa gaggtgctct   2337
   ggctcattaa agcttcgtgg tgcctgataa aaaaaaaaaa aa                      2379
<210> 39
   <211> 722
   <212> PRT
   <213> Mus musculus
<400> 39
   Met Trp Gly Leu Leu Leu Ala Val Thr Ala Phe Ala Pro Ser Val Gly
   1               5                   10                  15
   Leu Gly Leu Gly Ala Pro Ser Ala Ser Val Pro Gly Leu Ala Pro Gly
               20                  25                  30
   Ser Thr Leu Ala Pro His Ser Ser Val Ala Gln Pro Ser Thr Lys Ala
           35                  40                  45
   Asn Glu Thr Ser Glu Arg His Val Arg Leu Arg Val Ile Lys Lys Lys
       50                  55                  60
   Lys Ile Val Val Lys Lys Arg Lys Lys Leu Arg His Pro Gly Pro Leu
   65                  70                  75                  80
   Gly Thr Ala Arg Pro Val Val Pro Thr His Pro Ala Lys Thr Leu Thr
                   85                  90                  95
   Leu Pro Glu Lys Gln Glu Pro Gly Cys Pro Pro Leu Gly Leu Glu Ser
               100                 105                 110
   Leu Arg Val Ser Asp Ser Gln Leu Glu Ala Ser Ser Ser Gln Ser Phe
           115                 120                 125
   Gly Leu Gly Ala His Arg Gly Arg Leu Asn Ile Gln Ser Gly Leu Glu
       130                 135                 140
   Asp Gly Asp Leu Tyr Asp Gly Ala Trp Cys Ala Glu Gln Gln Asp Thr
   145                 150                 155                 160
   Glu Pro Trp Leu Gln Val Asp Ala Lys Asn Pro Val Arg Phe Ala Gly
                   165                 170                 175
   Ile Val Thr Gln Gly Arg Asn Ser Val Trp Arg Tyr Asp Trp Val Thr
               180                 185                 190
   Ser Phe Lys Val Gln Phe Ser Asn Asp Ser Gln Thr Trp Trp Lys Ser
           195                 200                 205
   Arg Asn Ser Thr Gly Met Asp Ile Val Phe Pro Ala Asn Ser Asp Ala
   210 215 220
   Glu Thr Pro Val Leu Asn Leu Leu Pro Glu Pro Gln Val Ala Arg Phe
   225 230 235 240
   Ile Arg Leu Leu Pro Gln Thr Trp Phe Gln Gly Gly Val Pro Cys Leu
   245 250 255
   Arg Ala Glu Ile Leu Ala Cys Pro Val Ser Asp Pro Asn Asp Leu Phe
   260 265 270
   Pro Glu Ala His Thr Leu Gly Ser Ser Asn Ser Leu Asp Phe Arg His
   275 280 285
   His Asn Tyr Lys Ala Met Arg Lys Leu Met Lys Gln Val Asn Glu Gln
   290 295 300
   Cys Pro Asn Ile Thr Arg Ile Tyr Ser Ile Gly Lys Ser His Gln Gly
   305 310 315 320
   Leu Lys Leu Tyr Val Met Glu Met Ser Asp His Pro Gly Glu His Glu
   325 330 335
   Leu Gly Glu Pro Glu Val Arg Tyr Val Ala Gly Met His Gly Asn Glu
   340 345 350
   Ala Leu Gly Arg Glu Leu Leu Leu Leu Leu Met Gln Phe Leu Cys His
   355 360 365
   Glu Phe Leu Arg Gly Asp Pro Arg Val Thr Arg Leu Leu Thr Glu Thr
   370 375 380
   Arg Ile His Leu Leu Pro Ser Met Asn Pro Asp Gly Tyr Glu Thr Ala
   385 390 395 400
   Tyr His Arg Gly Ser Glu Leu Val Gly Trp Ala Glu Gly Arg Trp Thr
   405 410 415
   His Gln Gly Ile Asp Leu Asn His Asn Phe Ala Asp Leu Asn Thr Gln
   420 425 430
   Leu Trp Tyr Ala Glu Asp Asp Gly Leu Val Pro Asp Thr Val Pro Asn
   435 440 445
   His His Leu Pro Leu Pro Thr Tyr Tyr Thr Leu Pro Asn Ala Thr Val
   450 455 460
   Ala Pro Glu Thr Trp Ala Val Ile Lys Trp Met Lys Arg Ile Pro Phe
   465 470 475 480
   Val Leu Ser Ala Asn Leu His Gly Gly Glu Leu Val Val Ser Tyr Pro
                   485                 490                 495
   Phe Asp Met Thr Arg Thr Pro Trp Ala Ala Arg Glu Leu Thr Pro Thr
               500                 505                 510
   Pro Asp Asp Ala Val Phe Arg Trp Leu Ser Thr Val Tyr Ala Gly Thr
           515                 520                 525
   Asn Arg Ala Met Gln Asp Thr Asp Arg Arg Pro Cys His Ser Gln Asp
       530                 535                 540
   Phe Ser Leu His Gly Asn Val Ile Asn Gly Ala Asp Trp His Thr Val
   545                 550                 555                 560
   Pro Gly Ser Met Asn Asp Phe Ser Tyr Leu His Thr Asn Cys Phe Glu
                   565                 570                 575
   Val Thr Val Glu Leu Ser Cys Asp Lys Phe Pro His Glu Lys Glu Leu
               580                 585                 590
   Pro Gln Glu Trp Glu Asn Asn Lys Asp Ala Leu Leu Thr Tyr Leu Glu
           595                 600                 605
   Gln Val Arg Met Gly Ile Thr Gly Val Val Arg Asp Lys Asp Thr Glu
       610                 615                 620
   Leu Gly Ile Ala Asp Ala Val Ile Ala Val Glu Gly Ile Asn His Asp
   625                 630                 635                 640
   Val Thr Thr Ala Trp Gly Gly Asp Tyr Trp Arg Leu Leu Thr Pro Gly
                   645                 650                 655
   Asp Tyr Val Val Thr Ala Ser Ala Glu Gly Tyr His Thr Val Arg Gln
               660                 665                 670
   His Cys Gln Val Thr Phe Glu Glu Gly Pro Val Pro Cys Asn Phe Leu
           675                 680                 685
   Leu Thr Lys Thr Pro Lys Glu Arg Leu Arg Glu Leu Leu Ala Thr Arg
       690                 695                 700
   Gly Lys Leu Pro Pro Asp Leu Arg Arg Lys Leu Glu Arg Leu Arg Gly
   705                 710                 715                 720
   Gln Lys
<210> 40
   <211> 2390
   <212> DNA
   <213> Homo sapiens
<220>
   <221> CDS
   <222> (65)..(2269)
   <223> SF08, cDNA: NM_019609, Protein: NP_062555
<400> 40
   cggcaggaag agaccgaccc gccacccgcc gtagcccgcg cgcccctggc actcaatccc      60
   cgcc atg tgg ggg ctc ctg ctc gcc ctg gcc gcc ttc gcg ccg gcc gtc      109
        Met Trp Gly Leu Leu Leu Ala Leu Ala Ala Phe Ala Pro Ala Val
        1               5                   10                  15
   ggc ccg gct ctg ggg gcg ccc agg aac tcg gtg ctg ggc ctc gcg cag      157
   Gly Pro Ala Leu Gly Ala Pro Arg Asn Ser Val Leu Gly Leu Ala Gln
                   20                  25                  30
   ccc ggg acc acc aag gtc cca ggc tcg acc ccg gcc ctg cat agc agc      205
   Pro Gly Thr Thr Lys Val Pro Gly Ser Thr Pro Ala Leu His Ser Ser
               35                  40                  45
   ccg gca cag ccg ccg gcg gag aca gct aac ggg acc tca gaa cag cat      253
   Pro Ala Gln Pro Pro Ala Glu Thr Ala Asn Gly Thr Ser Glu Gln His
           50                  55                  60
   gtc cgg att cga gtc atc aag aag aaa aag gtc att atg aag aag cgg      301
   Val Arg Ile Arg Val Ile Lys Lys Lys Lys Val Ile Met Lys Lys Arg
       65                  70                  75
   aag aag cta act cta act cgc ccc acc cca ctg gtg act gcc ggg ccc      349
   Lys Lys Leu Thr Leu Thr Arg Pro Thr Pro Leu Val Thr Ala Gly Pro
   80                  85                  90                  95
   ctt gtg acc ccc act cca gca ggg acc ctc gac ccc gct gag aaa caa      397
   Leu Val Thr Pro Thr Pro Ala Gly Thr Leu Asp Pro Ala Glu Lys Gln
                   100                 105                 110
   gaa aca ggc tgt cct cct ttg ggt ctg gag tcc ctg cga gtt tca gat      445
   Glu Thr Gly Cys Pro Pro Leu Gly Leu Glu Ser Leu Arg Val Ser Asp
               115                 120                 125
   agc cgg ctt gag gca tcc agc agc cag tcc ttt ggt ctt gga cca cac      493
   Ser Arg Leu Glu Ala Ser Ser Ser Gln Ser Phe Gly Leu Gly Pro His
           130                 135                 140
   cga gga cgg ctc aac att cag tca ggc ctg gag gac ggc gat cta tat      541
   Arg Gly Arg Leu Asn Ile Gln Ser Gly Leu Glu Asp Gly Asp Leu Tyr
       145                 150                 155
   gat gga gcc tgg tgt gct gag gag cag gac gcc gat cca tgg ttt cag      589
   Asp Gly Ala Trp Cys Ala Glu Glu Gln Asp Ala Asp Pro Trp Phe Gln
   160                 165                 170                 175
   gtg gac gct ggg cac ccc acc cgc ttc tcg ggt gtt atc aca cag ggc      637
   Val Asp Ala Gly His Pro Thr Arg Phe Ser Gly Val Ile Thr Gln Gly
                   180                 185                 190
   agg aac tct gtc tgg agg tat gac tgg gtc aca tca tac aag gtc cag      685
   Arg Asn Ser Val Trp Arg Tyr Asp Trp Val Thr Ser Tyr Lys Val Gln
               195                 200                 205
   ttc agc aat gac agt cgg acc tgg tgg gga agt agg aac cac agc agt      733
   Phe Ser Asn Asp Ser Arg Thr Trp Trp Gly Ser Arg Asn His Ser Ser
           210                 215                 220
   ggg atg gac gca gta ttt cct gcc aat tca gac cca gaa act cca gtg      781
   Gly Met Asp Ala Val Phe Pro Ala Asn Ser Asp Pro Glu Thr Pro Val
       225                 230                 235
   ctg aac ctc ctg ccg gag ccc cag gtg gcc cgc ttc att cgc ctg ctg      829
   Leu Asn Leu Leu Pro Glu Pro Gln Val Ala Arg Phe Ile Arg Leu Leu
   240                 245                 250                 255
   ccc cag acc tgg ctc cag gga ggc gcg cct tgc ctc cgg gca gag atc      877
   Pro Gln Thr Trp Leu Gln Gly Gly Ala Pro Cys Leu Arg Ala Glu Ile
                   260                 265                 270
   ctg gcc tgc cca gtc tca gac ccc aat gac cta ttc ctt gag gcc cct      925
   Leu Ala Cys Pro Val Ser Asp Pro Asn Asp Leu Phe Leu Glu Ala Pro
               275                 280                 285
   gcg tcg gga tcc tct gac cct cta gac ttt cag cat cac aat tac aag      973
   Ala Ser Gly Ser Ser Asp Pro Leu Asp Phe Gln His His Asn Tyr Lys
           290                 295                 300
   gcc atg agg aag ctg atg aag cag gta caa gag caa tgc ccc aac atc     1021
   Ala Met Arg Lys Leu Met Lys Gln Val Gln Glu Gln Cys Pro Asn Ile
       305                 310                 315
   acc cgc atc tac agc att ggg aag agc tac cag ggc ctg aag ctg tat     1069
   Thr Arg Ile Tyr Ser Ile Gly Lys Ser Tyr Gln Gly Leu Lys Leu Tyr
   320                 325                 330                 335
   gtg atg gaa atg tcg gac aag cct ggg gag cat gag ctg ggg gag cct     1117
   Val Met Glu Met Ser Asp Lys Pro Gly Glu His Glu Leu Gly Glu Pro
                   340                 345                 350
   gag gtg cgc tac gtg gct ggc atg cat ggg aac gag gcc ctg ggg cgg     1165
   Glu Val Arg Tyr Val Ala Gly Met His Gly Asn Glu Ala Leu Gly Arg
               355                 360                 365
   gag ttg ctt ctg ctc ctg atg cag ttc ctg tgc cat gag ttc ctg cga     1213
   Glu Leu Leu Leu Leu Leu Met Gln Phe Leu Cys His Glu Phe Leu Arg
           370                 375                 380
   ggg aac cca cgg gtg acc cgg ctg ctc tct gag atg cgc att cac ctg     1261
   Gly Asn Pro Arg Val Thr Arg Leu Leu Ser Glu Met Arg Ile His Leu
       385                 390                 395
   ctg ccc tcc atg aac cct gat ggc tat gag atc gcc tac cac cgg ggt     1309
   Leu Pro Ser Met Asn Pro Asp Gly Tyr Glu Ile Ala Tyr His Arg Gly
   400                 405                 410                 415
   tca gag ctg gtg ggc tgg gcc gag ggc cgc tgg aac aac cag agc atc     1357
   Ser Glu Leu Val Gly Trp Ala Glu Gly Arg Trp Asn Asn Gln Ser Ile
                   420                 425                 430
   gat ctt aac cat aat ttt gct gac ctc aac aca cca ctg tgg gaa gca     1405
   Asp Leu Asn His Asn Phe Ala Asp Leu Asn Thr Pro Leu Trp Glu Ala
               435                 440                 445
   cag gac gat ggg aag gtg ccc cac atc gtc ccc aac cat cac ctg cca     1453
   Gln Asp Asp Gly Lys Val Pro His Ile Val Pro Asn His His Leu Pro
           450                 455                 460
   ttg ccc act tac tac acc ctg ccc aat gcc acc gtg gct cct gaa acg     1501
   Leu Pro Thr Tyr Tyr Thr Leu Pro Asn Ala Thr Val Ala Pro Glu Thr
       465                 470                 475
   cgg gca gta atc aag tgg atg aag cgg atc ccc ttt gtg cta agt gcc     1549
   Arg Ala Val Ile Lys Trp Met Lys Arg Ile Pro Phe Val Leu Ser Ala
   480                 485                 490                 495
   aac ctc cac ggg ggt gag ctc gtg gtg tcc tac cca ttc gac atg act     1597
   Asn Leu His Gly Gly Glu Leu Val Val Ser Tyr Pro Phe Asp Met Thr
                   500                     505                 510
   cgc acc ccg tgg gct gcc cgc gag ctc acg ccc aca cca gat gat gct     1645
   Arg Thr Pro Trp Ala Ala Arg Glu Leu Thr Pro Thr Pro Asp Asp Ala
               515                     520                 525
   gtg ttt cgc tgg ctc agc act gtc tat gct ggc agt aat ctg gcc atg     1693
   Val Phe Arg Trp Leu Ser Thr Val Tyr Ala Gly Ser Asn Leu Ala Met
           530                     535                     540
   cag gac acc agc cgc cga ccc tgc cac agc cag gac ttc tcc gtg cac     1741
   Gln Asp Thr Ser Arg Arg Pro Cys His Ser Gln Asp Phe Ser Val His
       545                     550                     555
   ggc aac atc atc aac ggg gct gac tgg cac acg gtc ccc ggg agc atg     1789
   Gly Asn Ile Ile Asn Gly Ala Asp Trp His Thr Val Pro Gly Ser Met
   560                 565                     570                 575
   aat gac ttc agc tac cta cac acc aac tgc ttt gag gtc act gtg gag     1837
   Asn Asp Phe Ser Tyr Leu His Thr Asn Cys Phe Glu Val Thr Val Glu
                   580                     585                 590
   ctg tcc tgt gac aag ttc cct cac gag aat gaa ttg ccc cag gag tgg     1885
   Leu Ser Cys Asp Lys Phe Pro His Glu Asn Glu Leu Pro Gln Glu Trp
               595                     600                 605
   gag aac aac aaa gac gcc ctc ctc acc tac ctg gag cag gtg cgc atg     1933
   Glu Asn Asn Lys Asp Ala Leu Leu Thr Tyr Leu Glu Gln Val Arg Met
           610                     615                     620
   ggc att gca gga gtg gtg agg gac aag gac acg gag ctt ggg att gct     1981
   Gly Ile Ala Gly Val Val Arg Asp Lys Asp Thr Glu Leu Gly Ile Ala
       625                     630                     635
   gac gct gtc att gcc gtg gat ggg att aac cat gac gtg acc acg gcg     2029
   Asp Ala Val Ile Ala Val Asp Gly Ile Asn His Asp Val Thr Thr Ala
   640                 645                     650                 655
   tgg ggc ggg gat tat tgg cgt ctg ctg acc cca ggg gac tac atg gtg     2077
   Trp Gly Gly Asp Tyr Trp Arg Leu Leu Thr Pro Gly Asp Tyr Met Val
                   660                     665                 670
   act gcc agt gcc gag ggc tac cat tca gtg aca cgg aac tgt cgg gtc     2125
   Thr Ala Ser Ala Glu Gly Tyr His Ser Val Thr Arg Asn Cys Arg Val
               675                     680                 685
   acc ttt gaa gag ggc ccc ttc ccc tgc aat ttc gtg ctc acc aag act     2173
   Thr Phe Glu Glu Gly Pro Phe Pro Cys Asn Phe Val Leu Thr Lys Thr
           690                     695                 700
   ccc aaa cag agg ctg cgc gag ctg ctg gca gct ggg gcc aag gtg ccc     2221
   Pro Lys Gln Arg Leu Arg Glu Leu Leu Ala Ala Gly Ala Lys Val Pro
       705                     710                     715
   ccg gac ctt cgc agg cgc ctg gag cgg cta agg gga cag aag gat tga     2269
   Pro Asp Leu Arg Arg Arg Leu Glu Arg Leu Arg Gly Gln Lys Asp
   720                 725                     730
   tacctgcggt ttaagagccc tagggcaggc tggacctgtc aagacgggaa ggggaagagt   2329
   agagagggag ggacaaagtg aggaaaaggt gctcattaaa gctaccgggc accttagctc   2389
   a                                                                   2390
<210> 41
   <211> 734
   <212> PRT
   <213> Homo sapiens
<400> 41
   Met Trp Gly Leu Leu Leu Ala Leu Ala Ala Phe Ala Pro Ala Val Gly
   1               5                   10                  15
   Pro Ala Leu Gly Ala Pro Arg Asn Ser Val Leu Gly Leu Ala Gln Pro
               20                  25                  30
   Gly Thr Thr Lys Val Pro Gly Ser Thr Pro Ala Leu His Ser Ser Pro
           35                  40                  45
   Ala Gln Pro Pro Ala Glu Thr Ala Asn Gly Thr Ser Glu Gln His Val
       50                  55                  60
   Arg Ile Arg Val Ile Lys Lys Lys Lys Val Ile Met Lys Lys Arg Lys
   65                  70                  75                  80
   Lys Leu Thr Leu Thr Arg Pro Thr Pro Leu Val Thr Ala Gly Pro Leu
                   85                  90                  95
   Val Thr Pro Thr Pro Ala Gly Thr Leu Asp Pro Ala Glu Lys Gln Glu
               100                 105                 110
   Thr Gly Cys Pro Pro Leu Gly Leu Glu Ser Leu Arg Val Ser Asp Ser
           115                 120                 125
   Arg Leu Glu Ala Ser Ser Ser Gln Ser Phe Gly Leu Gly Pro His Arg
       130                 135                 140
   Gly Arg Leu Asn Ile Gln Ser Gly Leu Glu Asp Gly Asp Leu Tyr Asp
   145                 150                 155                 160
   Gly Ala Trp Cys Ala Glu Glu Gln Asp Ala Asp Pro Trp Phe Gln Val
                   165                 170                 175
   Asp Ala Gly His Pro Thr Arg Phe Ser Gly Val Ile Thr Gln Gly Arg
               180                 185                 190
   Asn Ser Val Trp Arg Tyr Asp Trp Val Thr Ser Tyr Lys Val Gln Phe
           195                 200                 205
   Ser Asn Asp Ser Arg Thr Trp Trp Gly Ser Arg Asn His Ser Ser Gly
       210                 215                 220
   Met Asp Ala Val Phe Pro Ala Asn Ser Asp Pro Glu Thr Pro Val Leu
   225                 230                 235                 240
   Asn Leu Leu Pro Glu Pro Gln Val Ala Arg Phe Ile Arg Leu Leu Pro
                   245                 250                 255
   Gln Thr Trp Leu Gln Gly Gly Ala Pro Cys Leu Arg Ala Glu Ile Leu
   260 265 270
   Ala Cys Pro Val Ser Asp Pro Asn Asp Leu Phe Leu Glu Ala Pro Ala
   275 280 285
   Ser Gly Ser Ser Asp Pro Leu Asp Phe Gln His His Asn Tyr Lys Ala
   290 295 300
   Met Arg Lys Leu Met Lys Gln Val Gln Glu Gln Cys Pro Asn Ile Thr
   305 310 315 320
   Arg Ile Tyr Ser Ile Gly Lys Ser Tyr Gln Gly Leu Lys Leu Tyr Val
   325 330 335
   Met Glu Met Ser Asp Lys Pro Gly Glu His Glu Leu Gly Glu Pro Glu
   340 345 350
   Val Arg Tyr Val Ala Gly Met His Gly Asn Glu Ala Leu Gly Arg Glu
   355 360 365
   Leu Leu Leu Leu Leu Met Gln Phe Leu Cys His Glu Phe Leu Arg Gly
   370 375 380
   Asn Pro Arg Val Thr Arg Leu Leu Ser Glu Met Arg Ile His Leu Leu
   385 390 395 400
   Pro Ser Met Asn Pro Asp Gly Tyr Glu Ile Ala Tyr His Arg Gly Ser
   405 410 415
   Glu Leu Val Gly Trp Ala Glu Gly Arg Trp Asn Asn Gln Ser Ile Asp
   420 425 430
   Leu Asn His Asn Phe Ala Asp Leu Asn Thr Pro Leu Trp Glu Ala Gln
   435 440 445
   Asp Asp Gly Lys Val Pro His Ile Val Pro Asn His His Leu Pro Leu
   450 455 460
   Pro Thr Tyr Tyr Thr Leu Pro Asn Ala Thr Val Ala Pro Glu Thr Arg
   465 470 475 480
   Ala Val Ile Lys Trp Met Lys Arg Ile Pro Phe Val Leu Ser Ala Asn
   485 490 495
   Leu His Gly Gly Glu Leu Val Val Ser Tyr Pro Phe Asp Met Thr Arg
   500 505 510
   Thr Pro Trp Ala Ala Arg Glu Leu Thr Pro Thr Pro Asp Asp Ala Val
   515 520 525
   Phe Arg Trp Leu Ser Thr Val Tyr Ala Gly Ser Asn Leu Ala Met Gln
   530 535 540
   Asp Thr Ser Arg Arg Pro Cys His Ser Gln Asp Phe Ser Val His Gly
   545 550 555 560
   Asn Ile Ile Asn Gly Ala Asp Trp His Thr Val Pro Gly Ser Met Asn
   565 570 575
   Asp Phe Ser Tyr Leu His Thr Asn Cys Phe Glu Val Thr Val Glu Leu
   580 585 590
   Ser Cys Asp Lys Phe Pro His Glu Asn Glu Leu Pro Gln Glu Trp Glu
   595 600 605
   Asn Asn Lys Asp Ala Leu Leu Thr Tyr Leu Glu Gln Val Arg Met Gly
   610 615 620
   Ile Ala Gly Val Val Arg Asp Lys Asp Thr Glu Leu Gly Ile Ala Asp
   625 630 635 640
   Ala Val Ile Ala Val Asp Gly Ile Asn His Asp Val Thr Thr Ala Trp
   645 650 655
   Gly Gly Asp Tyr Trp Arg Leu Leu Thr Pro Gly Asp Tyr Met Val Thr
   660 665 670
   Ala Ser Ala Glu Gly Tyr His Ser Val Thr Arg Asn Cys Arg Val Thr
   675 680 685
   Phe Glu Glu Gly Pro Phe Pro Cys Asn Phe Val Leu Thr Lys Thr Pro
   690 695 700
   Lys Gln Arg Leu Arg Glu Leu Leu Ala Ala Gly Ala Lys Val Pro Pro
   705 710 715 720
   Asp Leu Arg Arg Arg Leu Glu Arg Leu Arg Gly Gln Lys Asp
   725 730
<210> 42
   <211> 1815
   <212> DNA
   <213> Mus musculus
<220>
   <221> CDS
   <222> (98)..(535)
   <223> SF09, cDNA: NM_139295, Protein: NP_647456
<400> 42
   gtgcggagaa aagcgtccca gggacggcag ctggcaaggt tcacgttgga gtgcttcgcg 60
   actgcgtcgg ggattatcgg ggtacccacc cggaagc atg gca acc cta cag ctg 115
                                             Met Ala Thr Leu Gln Leu
                                             1               5
   ctc aga gct ccc ttg ctg tgt gtc ctg ctt tgg gtc ttt tgt gct cca       163
   Leu Arg Ala Pro Leu Leu Cys Val Leu Leu Trp Val Phe Cys Ala Pro
               10                  15                  20
   ggt gcc aga gcc cat gac cat ggg gct gat gtc cat cat ggc agc gtg       211
   Gly Ala Arg Ala His Asp His Gly Ala Asp Val His His Gly Ser Val
           25                  30                  35
   ggc ctg gat aag agc aca gtg cac gac caa gag cac atc atg gaa cat       259
   Gly Leu Asp Lys Ser Thr Val His Asp Gln Glu His Ile Met Glu His
       40                  45                  50
   ctg gaa ggt gtc atc gac cag cca gag gcg gag atg tcc cca cag gaa       307
   Leu Glu Gly Val Ile Asp Gln Pro Glu Ala Glu Met Ser Pro Gln Glu
   55                  60                  65                  70
   ctg cag ctc cat tac ttc aaa atg cat gat tac gac ggc aac agt ttg       355
   Leu Gln Leu His Tyr Phe Lys Met His Asp Tyr Asp Gly Asn Ser Leu
                   75                  80                  85
   ctt gac ggc cta gag ctc tcc ata gcc atc act cac gtg cac aag gag       403
   Leu Asp Gly Leu Glu Leu Ser Ile Ala Ile Thr His Val His Lys Glu
               90                  95                  100
   gag ggg agt gag cag gcg cca gtc atg agc gag gat gag ctc gtc agc       451
   Glu Gly Ser Glu Gln Ala Pro Val Met Ser Glu Asp Glu Leu Val Ser
           105                 110                 115
   atc ata gat ggt gtc ctg agg gac gat gac aag aac aat gac ggc tac       499
   Ile Ile Asp Gly Val Leu Arg Asp Asp Asp Lys Asn Asn Asp Gly Tyr
       120                 125                 130
   atc gac tac gct gag ttt gca aag tca ctg cag tag accgttggct            545
   Ile Asp Tyr Ala Glu Phe Ala Lys Ser Leu Gln
   135                 140                 145
   ctttcctttg tgcacatgtg acccttgcta atgtgatgga cgtgtctggt aatgcgaaac     605
   aacttatttc cgtctactgc tcagcacttt ggtaagagcc tgtggcagtc tgtaagagtg     665
   gggtgaggaa gaagccacat gactgtggag agaagtggga caggcctcag tccctagagg     725
   tgtgtttaag cttgttgggc aagagccgga tgcggatctt cggaagggcg gtgggtatcc     785
   cgagttctca ggaatccgac tgtagaatgc cactctgact tcttgatgtt aatccatgct     845
   acctaaagta aagacaggct gcttggccaa gtggacacac ttgagaaaca gtggagggag     905
   agtgtgaaag ccacacgctt gccctggttg gtcctgtctt taggcagatg tggtcagtat     965
   tctgttcccc aggcatacag catcatatat taaagccaca gcagaagagg aatgtcgccc    1025
   actgaggcca cccagatgca gagtctagga ttccttgccc actggccttt tggaaatgaa    1085
   gcaccactgg cctgaataat tagcattttc cagatcttca gtatcttcca caactactgc    1145
   cataccctgt gttgtatcat ttgaccagga gggaaacctt gaattggggt gtgttctcta    1205
   atcactttcc actgtctgag ctttcctgac ccctgtattg tatccttgct cccagggctc    1265
   ccttcatggc ttgtgaactg ttaacttggt atctcaggtt aaactgtcag ctggtctagc    1325
   ctgagcgagg cctgagacca tcagtcacta agagcagtgg ctaacctcat cgaagttgga    1385
   aggaatgttt ttaaaattac ctcttcgagc ctgaatacaa agaataaaag aataaaagaa    1445
   ttcttttaat ttcagggaag atcagaaaag aaagcctaaa gccctttagc gttgtgaacc   1505
   tcagtagtag ctgaaagaga agctgccaca ggttgtactt gctctgtgag atgttgtaga   1565
   cattccgtaa gagaatccag aatgatagca ggatcaggaa agaaatggag ccaaatctgc   1625
   tctaaggtga atagagactt atttttcttt attaaagtat tcttgtaaga cagttttctg   1685
   tgtcaagtat ttgtgaaatc agagctgaca tgtaagctat tcttgtaata tctcattatt   1745
   ttgaaagatt tatataatga actctggcta tctgacaata aaatggatga aaaagcaaaa   1805
   aaaaaaaaaa                                                          1815
<210> 43
   <211> 145
   <212> PRT
   <213> Mus musculus
<400> 43
   Met Ala Thr Leu Gln Leu Leu Arg Ala Pro Leu Leu Cys Val Leu Leu
   1               5                   10                  15
   Trp Val Phe Cys Ala Pro Gly Ala Arg Ala His Asp His Gly Ala Asp
               20                  25                  30
   Val His His Gly Ser Val Gly Leu Asp Lys Ser Thr Val His Asp Gln
           35                  40                  45
   Glu His Ile Met Glu His Leu Glu Gly Val Ile Asp Gln Pro Glu Ala
       50                  55                  60
   Glu Met Ser Pro Gln Glu Leu Gln Leu His Tyr Phe Lys Met His Asp
   65                  70                  75                  80
   Tyr Asp Gly Asn Ser Leu Leu Asp Gly Leu Glu Leu Ser Ile Ala Ile
                   85                  90                  95
   Thr His Val His Lys Glu Glu Gly Ser Glu Gln Ala Pro Val Met Ser
               100                 105                 110
   Glu Asp Glu Leu Val Ser Ile Ile Asp Gly Val Leu Arg Asp Asp Asp
           115                 120                 125
   Lys Asn Asn Asp Gly Tyr Ile Asp Tyr Ala Glu Phe Ala Lys Ser Leu
       130                 135                 140
   Gln
   145
<210> 44
   <211> 4144
   <212> DNA
   <213> Homo sapiens
<220>
   <221> CDS
   <222> (95)..(535)
   <223> SF09, cDNA: NM_139279, Protein: NP_644808
<400> 44
   ggggcgaagc cgaggaagag cgttttgggg acgggggctg gtgaggctca cgttggaggg     60
   cttcgcgtct gcttcggaga ccgtaaggat attg atg acc atg aga tcc ctg ctc    115
                                         Met Thr Met Arg Ser Leu Leu
                                         1               5
   aga acc ccc ttc ctg tgt ggc ctg ctc tgg gcc ttt tgt gcc cca ggc      163
   Arg Thr Pro Phe Leu Cys Gly Leu Leu Trp Ala Phe Cys Ala Pro Gly
           10                  15                  20
   gcc agg gct gag gag cct gca gcc agc ttc tcc caa ccc ggc agc atg      211
   Ala Arg Ala Glu Glu Pro Ala Ala Ser Phe Ser Gln Pro Gly Ser Met
       25                  30                  35
   ggc ctg gat aag aac aca gtg cac gac caa gag cat atc atg gag cat      259
   Gly Leu Asp Lys Asn Thr Val His Asp Gln Glu His Ile Met Glu His
   40                  45                  50                  55
   cta gaa ggt gtc atc aac aaa cca gag gcg gag atg tcg cca caa gaa      307
   Leu Glu Gly Val Ile Asn Lys Pro Glu Ala Glu Met Ser Pro Gln Glu
                   60                  65                  70
   ttg cag ctc cat tac ttc aaa atg cat gat tat gat ggc aat aat ttg      355
   Leu Gln Leu His Tyr Phe Lys Met His Asp Tyr Asp Gly Asn Asn Leu
               75                  80                  85
   ctt gat ggc tta gaa ctc tcc aca gcc atc act cat gtc cat aag gag      403
   Leu Asp Gly Leu Glu Leu Ser Thr Ala Ile Thr His Val His Lys Glu
           90                  95                  100
   gaa ggg agt gaa cag gca cca cta atg agt gaa gat gaa ctg att aac      451
   Glu Gly Ser Glu Gln Ala Pro Leu Met Ser Glu Asp Glu Leu Ile Asn
       105                 110                 115
   ata ata gat ggt gtt ttg aga gat gat gac aag aac aat gat gga tac      499
   Ile Ile Asp Gly Val Leu Arg Asp Asp Asp Lys Asn Asn Asp Gly Tyr
   120                 125                 130                 135
   att gac tat gct gaa ttt gca aaa tca ctg cag tag atgttatttg           545
   Ile Asp Tyr Ala Glu Phe Ala Lys Ser Leu Gln
                   140                 145
   gccatctcct ggttatatac aaatgtgacc cgtgataatg tgattgaaca ctttagtaat    605
   gcaaaataac tcatttccaa ctactgctgc agcattttgg taaaaacctg tagcgattcg    665
   ttacactggg gtgagaagag ataagagaaa tgaaagagaa gagaaatggg acatctaata    725
   gtccctaagt gctattaaat accttattgg acaagggctt gcttcaagca tctgtattag    785
   tctgtattaa tgctgctgat aaagacgtac ccgagactgg gaagaaaaag aggtttactt    845
   ggacttacag ttccacatgg ctggggaggc ctcagaatca tggcgggagg tgaaaggcac    905
   ttcttacatg gcagcaagag aaaatgagga agaagcaaaa gtggaaaccc ctgataagcc    965
   atcagatctt gtgaaactta ttcactatca caagaatagc atgggaaaga ctggccccca   1025
   tgattcaatt acctcccctt gggtctctcc cacaacacgt gggaattctg gtagatacaa   1085
   tttcaagttg agatttgggt ggggacatag ccaaaccata tcattctacc cctggcccct   1145
   ccaaatctca tgtcctcact attcaaaacc aatcatgcct tcctaacagt cccccaaagt 1205
   cttaactctt ttcagcatta acgcaaaaat ccacagtcca aagtctcatc tgagacaagg 1265
   caagtccctt ccacctatga gcctgtaaaa tcaaaagcaa gctagttact tcctagatac 1325
   caacaggggt acaggtattg attaaagacg gctgttccaa atgggagaaa ttggccaaaa 1385
   taaaggggtt acagggccca tgcaagtccg aaatccagca gggctgtcaa attttaaagt 1445
   tccagaataa tctcctttga ctccaggtct cacatccagg tcatactgat gcaagaagtg 1505
   ggttcccatg gtcttgggca gctctgcccc tgtggctttg tagggtacag cctccctcct 1565
   ggctgctttc acggctgttg ttcagtgcct gcggcttttc caggtgcacg gtgcaagctg 1625
   ttggtggatc taccattctg gggtctggag gacggtggcc ctcttctcac agctccacta 1685
   ggcagtgccc cagtagggac tctgtgtggg ggctcccaca ccacatttcc cttctgcact 1745
   gccctagcag aggttctctc ccctgccgct gagagggcct ctcccctgca gcaaacgttt 1805
   gcctgggcat tgaggcattt ccatacatct tctgaaaact aggcggaggt ttccaaatct 1865
   caattcttga cttctgtgca cctgcaggct taacagcaca tagaagctgc caaggcttgg 1925
   ggcttccact ctgaagccac agcccgagct gtatgttggc ccctttcagc catggctgga 1985
   gtggctggga cacaagacac caagtcccta ggctgcacac acatgtcagg ggctgccctg 2045
   acatggcctg gagacatttt ccccatggtg ttggggatta acattaggct ccttgctact 2105
   tatgcaaatt tctgcagctg gcttgaattt ctccccagaa aatgggtttt tcttttctat 2165
   tgcatagtca ggctgcaaat ttccaaactt ttatgctttg cttcccttat ttataaggga 2225
   atgcctttaa aagcacccaa gtcacctgtt gaacactttg ctgcttagaa atttcttccg 2285
   ctagttaacc taaatcatct ctctcaagtt caaagttcca caaatcccta tggaaggggc 2345
   aaaatgctgc cagtctcttt gctaaaacat aacaagagtc acctttactc cagttcccaa 2405
   caagttcctc atcttcatct gaggccacct cagcctggac tttgttgtcc atattgctat 2465
   cagcatttgg ggcaaagcca ttcaacaagt ctgtaggaag ttccaaactt tcccacattt 2525
   tcctgttttc ttctgagccc tccaaactgt tccagcctct gcctgttacc cagttccaaa 2585
   gtcacttcca cattttgggt atttcttcag caggtcccaa tctactggta ccaatttact   2645
   gtattagtcc gttttcacgc tgctgataaa gacatacccg agactgggaa gaaaaagtgg   2705
   tttaattgga cttaaagttc cacatggctg gggaggcctc agaatcatgg tgggaggcaa 2765
   aagacacttc ttacattgtg gcaagaaaaa atgaggaaga agcaaaagca gaaacccctg 2825
   ataaactgat cagatctcat gagacttatt cactgtcacg agaatagcac gggaaagact 2885
   ggcccccatg attcaattac ctccccctgg gtctgtccca caacacgtgg gaattctggg 2945
   agatacaatt caagttgaga tttgtggggg gacacaacca aaccatatca gcatcctttc 3005
   aagaatatta gataattgga gctgagtact caggaacttg actgtagtag aatactgcta 3065
   gtttcttaat tttaattcac atcacctgaa aagtaaaaca acaggctttg ccaagtggat 3125
   gcttttcagt aacagtgaag tggagtgaat accaaatgtt tgccctggtg gttcctatct 3185
   cttcaggcaa acatggtcag tattctgtaa agttcccctg gcctaaatga ttacttgctc   3245
   tgggcaagtg gatatttatt aggctatttc aaagccacag cataagaatg tcagcctagc   3305
   cacagagtct gagattctga gttcagccta gccacagagt ctaagattct gtatcctctg   3365
   acattttgga aatgatacac tactggctta agtgatgact ctttcagatt ttcagtattt   3425
   tatacaacta ctgccacatc cttatacttt attgcttttc tgtcttcttc aacctgggag   3485
   agaccctgaa tttgagtgtg ttctctaatc aatagtggtt tagctttctt ttctatttca   3545
   ctcgtttcta gggtttttta tttgcagttt aggaactatt aggaatgtca ggactttatc   3605
   agcaggggta aaactaccac ctggcctagc ctaagtagga agtgaaaaga taattcacca   3665
   aacaatgatt aatcagatag aagttctagt caagagggat attgttgaag ttacctcttt   3725
   tagcctagat acatggattc ttttcaaatc aggaaagatt agaaaaggaa cccaaaaaac   3785
   cctttaacag tgtgaatctt tatagtattt gaaaatgaga agaagcagca gattgtaatt   3845
   tggtttattg gatgtgatgg acgttctgta atagaaaacc tgaaacgatg attgaatggg   3905
   aaaaagagac tacaaaattt gtcgtaggat gtatacagac ttattttctt tattacagta   3965
   ttataagaaa acatatgtat ttgtaaaaat ggtttcctgt gtcaagtatt tgtgcagtca   4025
   gagctgactt gtaaactatt cttgtaatag ctcattattt tgaaagattt atatatgatg   4085
   aattctggat atatgaccaa taaaactgat gaagcaaaaa aaaaaaaaaa aaaaaaaaa    4144
<210> 45
   <211> 146
   <212> PRT
   <213> Homo sapiens
<400> 45
   Met Thr Met Arg Ser Leu Leu Arg Thr Pro Phe Leu Cys Gly Leu Leu
   1               5                   10                  15
   Trp Ala Phe Cys Ala Pro Gly Ala Arg Ala Glu Glu Pro Ala Ala Ser
               20                  25                  30
   Phe Ser Gln Pro Gly Ser Met Gly Leu Asp Lys Asn Thr Val His Asp
           35                  40                  45
   Gln Glu His Ile Met Glu His Leu Glu Gly Val Ile Asn Lys Pro Glu
       50                  55                  60
   Ala Glu Met Ser Pro Gln Glu Leu Gln Leu His Tyr Phe Lys Met His
   65                  70                  75                  80
   Asp Tyr Asp Gly Asn Asn Leu Leu Asp Gly Leu Glu Leu Ser Thr Ala
                   85                  90                  95
   Ile Thr His Val His Lys Glu Glu Gly Ser Glu Gln Ala Pro Leu Met
               100                 105                 110
   Ser Glu Asp Glu Leu Ile Asn Ile Ile Asp Gly Val Leu Arg Asp Asp
   115 120 125
   Asp Lys Asn Asn Asp Gly Tyr Ile Asp Tyr Ala Glu Phe Ala Lys Ser
   130 135 140
   Leu Gln
   145
<210> 46
   <211> 1513
   <212> DNA
   <213> Mus musculus
<220>
   <221> CDS
   <222> (37)..(810)
   <223> SF10, cDNA: NM_029568, Protein: NP_083844
<400> 46
   ccgagacagc cagcctctct caactgagct gacacc atg aag gcc ctc cca gcc     54
                                           Met Lys Ala Leu Pro Ala
                                           1               5
   ctg cca ctg atg ctg atg ctg ctc tcc atg cct ccc ccc tgc gcc ccg    102
   Leu Pro Leu Met Leu Met Leu Leu Ser Met Pro Pro Pro Cys Ala Pro
               10                  15                  20
   caa gcc tct ggg atc cgg gga gat gct ctg gag aag tcc tgt ctt cag    150
   Gln Ala Ser Gly Ile Arg Gly Asp Ala Leu Glu Lys Ser Cys Leu Gln
           25                  30                  35
   caa ccc ctg gac tgt gat gat atc tac gcc cag ggc tat cag gaa gac    198
   Gln Pro Leu Asp Cys Asp Asp Ile Tyr Ala Gln Gly Tyr Gln Glu Asp
       40                  45                  50
   ggc gtg tat ctc atc tac ccc tat ggc ccc agt gtg ccg gtg ccc gtc    246
   Gly Val Tyr Leu Ile Tyr Pro Tyr Gly Pro Ser Val Pro Val Pro Val
   55                  60                  65                  70
   ttc tgc gac atg aca act gag ggc ggc aag tgg acg gtt ttc cag aaa    294
   Phe Cys Asp Met Thr Thr Glu Gly Gly Lys Trp Thr Val Phe Gln Lys
                   75                  80                  85
   aga ttc aac ggc tca gtg agt ttc ttc cgg ggc tgg agc gac tac aag    342
   Arg Phe Asn Gly Ser Val Ser Phe Phe Arg Gly Trp Ser Asp Tyr Lys
               90                  95                  100
   ctg ggc ttt ggc cgt gct gac ggg gag tac tgg ctg ggg ctg cag aac    390
   Leu Gly Phe Gly Arg Ala Asp Gly Glu Tyr Trp Leu Gly Leu Gln Asn
           105                 110                 115
   ctg cac ctc ctg aca ctg aag cag aag tat gag ctg cgc gtg gac ttg    438
   Leu His Leu Leu Thr Leu Lys Gln Lys Tyr Glu Leu Arg Val Asp Leu
       120                 125                 130
   gaa gac ttt gag aac aac aca gcc tat gcc aag tac att gac ttc tcc    486
   Glu Asp Phe Glu Asn Asn Thr Ala Tyr Ala Lys Tyr Ile Asp Phe Ser
   135                 140                 145                 150
   atc tcc ccc aac gcc atc agt gct gag gag gat ggc tat acc ctc tac    534
   Ile Ser Pro Asn Ala Ile Ser Ala Glu Glu Asp Gly Tyr Thr Leu Tyr
                   155                 160                 165
   gtg gct ggc ttc gag gat ggc ggg gca ggt gac tca ctg tcc tac cac       582
   Val Ala Gly Phe Glu Asp Gly Gly Ala Gly Asp Ser Leu Ser Tyr His
               170                 175                 180
   agt ggc cag aag ttc tcc acc ttt gat cgg gac cag gac ctc ttc gtg       630
   Ser Gly Gln Lys Phe Ser Thr Phe Asp Arg Asp Gln Asp Leu Phe Val
           185                 190                 195
   cag aac tgt gca gcc ctc tcc tca gga gcc ttc tgg ttc cga agc tgc       678
   Gln Asn Cys Ala Ala Leu Ser Ser Gly Ala Phe Trp Phe Arg Ser Cys
       200                 205                 210
   cat ttc gcc aat ctc aac ggt ttc tac ctg ggt ggt tcc cat ctc tcc       726
   His Phe Ala Asn Leu Asn Gly Phe Tyr Leu Gly Gly Ser His Leu Ser
   215                 220                 225                 230
   tat gcc aat ggc atc aat tgg gcc caa tgg aaa ggc ttc tat tac tcc       774
   Tyr Ala Asn Gly Ile Asn Trp Ala Gln Trp Lys Gly Phe Tyr Tyr Ser
                   235                 240                 245
   ctc aag cgc acg gag atg aaa att cgt cgg gcc tga ggggctggcc            820
   Leu Lys Arg Thr Glu Met Lys Ile Arg Arg Ala
               250                 255
   caagcaggcc ccatctttcc cctgaagtcc caagggtcca tgttctccct ccacgcttta     880
   cccacaattc ctgagcacca gccatgccct ggcaaatccc tgtcccacat acagccacgc     940
   cctgatgcat tccacctgag gctaggctgt cagcagccct ccaggccttt ctgtggctga    1000
   gccatcctag cctggatctg gctgaaatcc attaaaaact ccaagttgct tctacccctt    1060
   cacgacagct gaaagccaga agctaccttc tagctgccag cttttgcacc ccacctcagc    1120
   agtttcctta ctgcagagcc ttctgtttgg ggctaccctc gacagagtca tgcagcacct    1180
   gtggcattgc caatcagctc ttgcacactg ccacacccca gcactcacat agctctcctc    1240
   agaatacttc ctaccttggc ctcatcactt tactccctac gtgagcatca tggagcccaa    1300
   tcccatctgc cttcactcat ccctcaaaat tcaccaccaa aacaatactc accacggcta    1360
   ctgctcaact ctgaagtcgt catggcaaag ataggcttgt tgacttggtc ccctacttgc    1420
   cctagcgatc gtcatgagag gcagcaggga tcaatatgtg gggctggaag tgggtgggta    1480
   gcagaggtct caataaactt caggatctga tgg                                 1513
<210> 47
   <211> 257
   <212> PRT
   <213> Mus musculus
<400> 47
   Met Lys Ala Leu Pro Ala Leu Pro Leu Met Leu Met Leu Leu Ser Met
   1               5                   10                  15
   Pro Pro Pro Cys Ala Pro Gln Ala Ser Gly Ile Arg Gly Asp Ala Leu
               20                  25                  30
   Glu Lys Ser Cys Leu Gln Gln Pro Leu Asp Cys Asp Asp Ile Tyr Ala
           35                  40                  45
   Gln Gly Tyr Gln Glu Asp Gly Val Tyr Leu Ile Tyr Pro Tyr Gly Pro
       50                  55                  60
   Ser Val Pro Val Pro Val Phe Cys Asp Met Thr Thr Glu Gly Gly Lys
   65                  70                  75                  80
   Trp Thr Val Phe Gln Lys Arg Phe Asn Gly Ser Val Ser Phe Phe Arg
                   85                  90                  95
   Gly Trp Ser Asp Tyr Lys Leu Gly Phe Gly Arg Ala Asp Gly Glu Tyr
               100                 105                 110
   Trp Leu Gly Leu Gln Asn Leu His Leu Leu Thr Leu Lys Gln Lys Tyr
           115                 120                 125
   Glu Leu Arg Val Asp Leu Glu Asp Phe Glu Asn Asn Thr Ala Tyr Ala
       130                 135                 140
   Lys Tyr Ile Asp Phe Ser Ile Ser Pro Asn Ala Ile Ser Ala Glu Glu
   145                 150                 155                 160
   Asp Gly Tyr Thr Leu Tyr Val Ala Gly Phe Glu Asp Gly Gly Ala Gly
                   165                 170                 175
   Asp Ser Leu Ser Tyr His Ser Gly Gln Lys Phe Ser Thr Phe Asp Arg
               180                 185                 190
   Asp Gln Asp Leu Phe Val Gln Asn Cys Ala Ala Leu Ser Ser Gly Ala
           195                 200                 205
   Phe Trp Phe Arg Ser Cys His Phe Ala Asn Leu Asn Gly Phe Tyr Leu
       210                 215                 220
   Gly Gly Ser His Leu Ser Tyr Ala Asn Gly Ile Asn Trp Ala Gln Trp
   225                 230                 235                 240
   Lys Gly Phe Tyr Tyr Ser Leu Lys Arg Thr Glu Met Lys Ile Arg Arg
                   245                 250                 255
   Ala
<210> 48
   <211> 1830
   <212> DNA
   <213> Homo sapiens
<220>
   <221> CDS
   <222> (26)..(793)
   <223> SF10, cDNA: NM_002404, Protein: NP_002395
<400> 48
   agccactctg agcagaactg acagc atg aag gca ctc ctg gcc ctg ccg ctg     52
                               Met Lys Ala Leu Leu Ala Leu Pro Leu
                               1               5
   ctg ctg ctt ctc tcc acg ccc ccg tgt gcc ccc cag gtc tcc ggg atc    100
   Leu Leu Leu Leu Ser Thr Pro Pro Cys Ala Pro Gln Val Ser Gly Ile
   10                  15                  20                  25
   cga gga gat gct ctg gag agg ttt tgc ctt cag caa ccc ctg gac tgt    148
   Arg Gly Asp Ala Leu Glu Arg Phe Cys Leu Gln Gln Pro Leu Asp Cys
                   30                  35                  40
   gac gac atc tat gcc cag ggc tac cag tca gac ggc gtg tac ctc atc    196
   Asp Asp Ile Tyr Ala Gln Gly Tyr Gln Ser Asp Gly Val Tyr Leu Ile
               45                  50                  55
   tac ccc tcg ggc ccc agt gtg cct gtg ccc gtc ttc tgt gac atg acc    244
   Tyr Pro Ser Gly Pro Ser Val Pro Val Pro Val Phe Cys Asp Met Thr
           60                  65                  70
   acc gag ggc ggg aag tgg acg gtt ttc cag aag aga ttc aat ggc tca    292
   Thr Glu Gly Gly Lys Trp Thr Val Phe Gln Lys Arg Phe Asn Gly Ser
       75                  80                  85
   gta agt ttc ttc cgc ggc tgg aat gac tac aag ctg ggc ttc ggc cgt    340
   Val Ser Phe Phe Arg Gly Trp Asn Asp Tyr Lys Leu Gly Phe Gly Arg
   90                  95                  100                 105
   gct gat gga gag tac tgg ctg ggg ctg cag aac atg cac ctc ctg aca    388
   Ala Asp Gly Glu Tyr Trp Leu Gly Leu Gln Asn Met His Leu Leu Thr
                   110                 115                 120
   ctg aag cag aag tat gag ctg cga gtg gac ttg gag gac ttt gag aac    436
   Leu Lys Gln Lys Tyr Glu Leu Arg Val Asp Leu Glu Asp Phe Glu Asn
               125                 130                 135
   aac acg gcc tat gcc aag tac gct gac ttc tcc atc tcc ccg aac gcg    484
   Asn Thr Ala Tyr Ala Lys Tyr Ala Asp Phe Ser Ile Ser Pro Asn Ala
           140                 145                 150
   gtc agc gca gag gag gat ggc tac acc ctc ttt gtg gca ggc ttt gag    532
   Val Ser Ala Glu Glu Asp Gly Tyr Thr Leu Phe Val Ala Gly Phe Glu
       155                 160                 165
   gat ggc ggg gca ggt gac tcc ctg tcc tac cac agt ggc cag aag ttc    580
   Asp Gly Gly Ala Gly Asp Ser Leu Ser Tyr His Ser Gly Gln Lys Phe
   170                 175                 180                 185
   tct acc ttc gac cgg gac cag gac ctc ttt gtg cag aac tgc gca gct    628
   Ser Thr Phe Asp Arg Asp Gln Asp Leu Phe Val Gln Asn Cys Ala Ala
                   190                 195                 200
   ctc tcc tca gga gcc ttc tgg ttc cgc agc tgc cac ttt gcc aac ctc    676
   Leu Ser Ser Gly Ala Phe Trp Phe Arg Ser Cys His Phe Ala Asn Leu
               205                 210                 215
   aat ggc ttc tac cta ggt ggc tcc cac ctc tct tat gcc aat ggc atc    724
   Asn Gly Phe Tyr Leu Gly Gly Ser His Leu Ser Tyr Ala Asn Gly Ile
           220                 225                 230
   aac tgg gcc cag tgg aag ggc ttc tac tac tcc ctc aaa cgc act gag    772
   Asn Trp Ala Gln Trp Lys Gly Phe Tyr Tyr Ser Leu Lys Arg Thr Glu
       235                 240                 245
   atg aaa atc cgc cgg gcc tga agggctggcc ccctcaggca cctttcctcc       823
   Met Lys Ile Arg Arg Ala
   250                 255
   cctggacacc catggtctcc atgagtgctc cctctgctgc ccctgatgca tgcttctgct 883
   gattcccgag caccaactcc ttacaagggg gccttgtggc tctcagccat gccacatccc 943
   tgtcacacac ccagggcatc cattcctaag ccagacccgg ctcccctaca cctgaagtta 1003
   cactgccagc agttccccag gcctcttccg agaggcacat ggttctagcc tggacctggc 1063
   tgggctccat gagaatgagt tgcctccacc ctgtcccaac agctgacagc caggagccac 1123
   tctcccagct gcaggccttt gtggtccatc ttgtcctgct tcctcactgt ggacccctgt 1183
   ctgggccacc ctagtgtgct aagctgagca gtgcagtgtg aacagggccc atggtgtatt 1243
   ctaggccaca gcccagcact cctctgggct gctctcaaac catgtcccat cttcagcatc 1303
   cctcccacca acttactccc ctgtggtgag taccgtggaa ccccagccca cctcactatc 1363
   atactcagct tcccctgatg gcccatccca gcccctgaag ctctatgcca agaacacagc 1423
   taccgcacac caccctgaaa cagccacagc caaggtaggc atgcatatga ggtcttcccc 1483
   ataccctctg ggtgttgaga ggtttagcca catgagggag cagaggacaa tctctgcagg 1543
   gctgggagtg ggtagggact gaaggtctca ataaaccttc agaacctgaa tgaactggct 1603
   tcatacacac aaacatattt gtttatcccc caaatgtagg cacctggctc ctccttgctc 1663
   ccctgctgat ggtgtcctac cccgaactcc aaaaattaca cctggagtca ggtgcagaag 1723
   ggaaccttgt atttcacagg cctcattttg atggcaaaaa gacagtgtaa taataacata 1783
   ataataataa aaatataata ctgaaaagga aaaaaaaaaa aaaaaaa 1830
<210> 49
   <211> 255
   <212> PRT
   <213> Homo sapiens
<400> 49
   Met Lys Ala Leu Leu Ala Leu Pro Leu Leu Leu Leu Leu Ser Thr Pro
   1 5 10 15
   Pro Cys Ala Pro Gln Val Ser Gly Ile Arg Gly Asp Ala Leu Glu Arg
   20 25 30
   Phe Cys Leu Gln Gln Pro Leu Asp Cys Asp Asp Ile Tyr Ala Gln Gly
   35 40 45
   Tyr Gln Ser Asp Gly Val Tyr Leu Ile Tyr Pro Ser Gly Pro Ser Val
   50 55 60
   Pro Val Pro Val Phe Cys Asp Met Thr Thr Glu Gly Gly Lys Trp Thr
   65 70 75 80
   Val Phe Gln Lys Arg Phe Asn Gly Ser Val Ser Phe Phe Arg Gly Trp
   85 90 95
   Asn Asp Tyr Lys Leu Gly Phe Gly Arg Ala Asp Gly Glu Tyr Trp Leu
   100 105 110
   Gly Leu Gln Asn Met His Leu Leu Thr Leu Lys Gln Lys Tyr Glu Leu
   115 120 125
   Arg Val Asp Leu Glu Asp Phe Glu Asn Asn Thr Ala Tyr Ala Lys Tyr
   130 135 140
   Ala Asp Phe Ser Ile Ser Pro Asn Ala Val Ser Ala Glu Glu Asp Gly
   145 150 155 160
   Tyr Thr Leu Phe Val Ala Gly Phe Glu Asp Gly Gly Ala Gly Asp Ser
   165 170 175
   Leu Ser Tyr His Ser Gly Gln Lys Phe Ser Thr Phe Asp Arg Asp Gln
   180 185 190
   Asp Leu Phe Val Gln Asn Cys Ala Ala Leu Ser Ser Gly Ala Phe Trp
   195 200 205
   Phe Arg Ser Cys His Phe Ala Asn Leu Asn Gly Phe Tyr Leu Gly Gly
   210 215 220
   Ser His Leu Ser Tyr Ala Asn Gly Ile Asn Trp Ala Gln Trp Lys Gly
   225 230 235 240
   Phe Tyr Tyr Ser Leu Lys Arg Thr Glu Met Lys Ile Arg Arg Ala
   245 250 255
<210> 50
   <211> 749
   <212> DNA
   <213> Mus musculus
<220>
   <221> CDS
   <222> (93) .. (515)
   <223> SF11, cDNA: NM_009976, Protein: NP_034106
<400> 50
   ggcatttggg taaaagtcgc acggagtagc agcgtctgtt ctgcaccaac tcagagtctt      60
   gttggagctt tatccctttg tcctagccaa cc atg gcc agc ccg ctg cgc tcc       113
                                       Met Ala Ser Pro Leu Arg Ser
                                       1               5
   ttg ctg ttc ctg ctg gcc gtc ctg gcc gtg gcc tgg gcg gcg acc cca       161
   Leu Leu Phe Leu Leu Ala Val Leu Ala Val Ala Trp Ala Ala Thr Pro
           10                  15                  20
   aaa caa ggc ccg cga atg ttg gga gcc ccg gag gag gca gat gcc aat       209
   Lys Gln Gly Pro Arg Met Leu Gly Ala Pro Glu Glu Ala Asp Ala Asn
       25                  30                  35
   gag gaa ggc gtg cgg cga gcg ttg gac ttc gct gtg agc gag tac aac       257
   Glu Glu Gly Val Arg Arg Ala Leu Asp Phe Ala Val Ser Glu Tyr Asn
   40                  45                  50                  55
   aag ggc agc aac gat gcg tac cac agc cgc gcc ata cag gtg gtg aga       305
   Lys Gly Ser Asn Asp Ala Tyr His Ser Arg Ala Ile Gln Val Val Arg
                   60                  65                  70
   gct cgt aag cag ctc gtg gct gga gtg aac tat ttt ttg gat gtg gag       353
   Ala Arg Lys Gln Leu Val Ala Gly Val Asn Tyr Phe Leu Asp Val Glu
               75                  80                  85
   atg ggc cga act aca tgt acc aag tcc cag aca aat ttg act gac tgt       401
   Met Gly Arg Thr Thr Cys Thr Lys Ser Gln Thr Asn Leu Thr Asp Cys
           90                  95                  100
   cct ttc cat gac cag ccc cat ctg atg agg aag gca ctc tgc tcc ttc       449
   Pro Phe His Asp Gln Pro His Leu Met Arg Lys Ala Leu Cys Ser Phe
       105                 110                 115
   cag atc tac agc gtg ccc tgg aaa ggc aca cac tcc ctg aca aaa ttc       497
   Gln Ile Tyr Ser Val Pro Trp Lys Gly Thr His Ser Leu Thr Lys Phe
   120                 125                 130                 135
   agc tgc aaa aat gcc taa gggctgagtc tagaaggatc atgcagactg              545
   Ser Cys Lys Asn Ala
                   140
   ttccttactt gtgctccttc cctatagtgt ttcatctcgc agaagggtgc tccggctctg     605
   gagggcaccg ccagtgtgtt tgcaccagga gacagtaaag gagctgctgc aggcaggttc     665
   tgcacatctg aacagctgtc ccctggctcc actcttcttg cagtacctgc catgccttgc     725
   tcaattaaaa aaaaaaaaaa ttcg                                              749
<210> 51
   <211> 140
   <212> PRT
   <213> Mus musculus
<400> 51
   Met Ala Ser Pro Leu Arg Ser Leu Leu Phe Leu Leu Ala Val Leu Ala
   1               5                   10                  15
   Val Ala Trp Ala Ala Thr Pro Lys Gln Gly Pro Arg Met Leu Gly Ala
               20                  25                  30
   Pro Glu Glu Ala Asp Ala Asn Glu Glu Gly Val Arg Arg Ala Leu Asp
           35                  40                  45
   Phe Ala Val Ser Glu Tyr Asn Lys Gly Ser Asn Asp Ala Tyr His Ser
       50                  55                  60
   Arg Ala Ile Gln Val Val Arg Ala Arg Lys Gln Leu Val Ala Gly Val
   65                  70                  75                  80
   Asn Tyr Phe Leu Asp Val Glu Met Gly Arg Thr Thr Cys Thr Lys Ser
                   85                  90                  95
   Gln Thr Asn Leu Thr Asp Cys Pro Phe His Asp Gln Pro His Leu Met
               100                 105                 110
   Arg Lys Ala Leu Cys Ser Phe Gln Ile Tyr Ser Val Pro Trp Lys Gly
           115                 120                 125
   Thr His Ser Leu Thr Lys Phe Ser Cys Lys Asn Ala
       130                 135                 140
<210> 52
   <211> 818
   <212> DNA
   <213> Homo sapiens
<220>
   <221> CDS
   <222> (76)..(516)
   <223> SF11, cDNA: NM_000099, Protein: NP_000090
<400> 52
   cgcagcgggt cctctctatc tagctccagc ctctcgcctg cgccccactc cccgcgtccc    60
   gcgtcctagc cgacc atg gcc ggg ccc ctg cgc gcc ccg ctg ctc ctg ctg    111
                    Met Ala Gly Pro Leu Arg Ala Pro Leu Leu Leu Leu
                    1               5                   10
   gcc atc ctg gcc gtg gcc ctg gcc gtg agc ccc gcg gcc ggc tcc agt     159
   Ala Ile Leu Ala Val Ala Leu Ala Val Ser Pro Ala Ala Gly Ser Ser
           15                  20                  25
   ccc ggc aag ccg ccg cgc ctg gtg gga ggc ccc atg gac gcc agc gtg     207
   Pro Gly Lys Pro Pro Arg Leu Val Gly Gly Pro Met Asp Ala Ser Val
       30                  35                  40
   gag gag gag ggt gtg cgg cgt gca ctg gac ttt gcc gtc ggc gag tac     255
   Glu Glu Glu Gly Val Arg Arg Ala Leu Asp Phe Ala Val Gly Glu Tyr
   45                  50                  55                  60
   aac aaa gcc agc aac gac atg tac cac agc cgc gcg ctg cag gtg gtg     303
   Asn Lys Ala Ser Asn Asp Met Tyr His Ser Arg Ala Leu Gln Val Val
                   65                  70                  75
   cgc gcc cgc aag cag atc gta gct ggg gtg aac tac ttc ttg gac gtg     351
   Arg Ala Arg Lys Gln Ile Val Ala Gly Val Asn Tyr Phe Leu Asp Val
               80                  85                  90
   gag ctg ggc cga acc acg tgt acc aag acc cag ccc aac ttg gac aac     399
   Glu Leu Gly Arg Thr Thr Cys Thr Lys Thr Gln Pro Asn Leu Asp Asn
           95                  100                 105
   tgc ccc ttc cat gac cag cca cat ctg aaa agg aaa gca ttc tgc tct     447
   Cys Pro Phe His Asp Gln Pro His Leu Lys Arg Lys Ala Phe Cys Ser
       110                 115                 120
   ttc cag atc tac gct gtg cct tgg cag ggc aca atg acc ttg tcg aaa     495
   Phe Gln Ile Tyr Ala Val Pro Trp Gln Gly Thr Met Thr Leu Ser Lys
   125                 130                 135                 140
   tcc acc tgt cag gac gcc tag gggtctgtac cgggctggcc tgtgcctatc       546
   Ser Thr Cys Gln Asp Ala
                   145
   acctcttatg cacacctccc accccctgta ttcccacccc tggactggtg gcccctgcct    606
   tggggaaggt ctccccatgt gcctgcacca ggagacagac agagaaggca gcaggcggcc    666
   tttgttgctc agcaaggggc tctgccctcc ctccttcctt cttgcttctc atagccccgg    726
   tgtgcggtgc atacaccccc acctcctgca ataaaatagt agcatcggca aaaaaaaaaa    786
   aaaaaaaaaa aaaaaaaaaa aaaaaaaaaa aa                                  818
<210> 53
   <211> 146
   <212> PRT
   <213> Homo sapiens
<400> 53
   Met Ala Gly Pro Leu Arg Ala Pro Leu Leu Leu Leu Ala Ile Leu Ala
   1 5 10 15
   Val Ala Leu Ala Val Ser Pro Ala Ala Gly Ser Ser Pro Gly Lys Pro
   20 25 30
   Pro Arg Leu Val Gly Gly Pro Met Asp Ala Ser Val Glu Glu Glu Gly
   35 40 45
   Val Arg Arg Ala Leu Asp Phe Ala Val Gly Glu Tyr Asn Lys Ala Ser
   50 55 60
   Asn Asp Met Tyr His Ser Arg Ala Leu Gln Val Val Arg Ala Arg Lys
   65 70 75 80
   Gln Ile Val Ala Gly Val Asn Tyr Phe Leu Asp Val Glu Leu Gly Arg
   85 90 95
   Thr Thr Cys Thr Lys Thr Gln Pro Asn Leu Asp Asn Cys Pro Phe His
   100 105 110
   Asp Gln Pro His Leu Lys Arg Lys Ala Phe Cys Ser Phe Gln Ile Tyr
   115 120 125
   Ala Val Pro Trp Gln Gly Thr Met Thr Leu Ser Lys Ser Thr Cys Gln
   130 135 140
   Asp Ala
   145
<210> 54
   <211> 2709
   <212> DNA
   <213> Mus musculus
<220>
   <221> CDS
   <222> (124)..(2241)
   <223> SF12, cDNA: NM_010180, Protein: NP_034310
<400> 54
   cctcctccgg gcgggataat tgaacgcggc gcgcaggagc ctcgcgttgg ctgcccaggc 60
   ctggctcgag cgtgtagccg ctgcccgccg tgcctttgtc cgccgccgga gcccgccagc 120
   ccc atg gag cgc ccc gtg ccg tcg cgc ctc gtc ccg ctg ccg ctg ctg      168
       Met Glu Arg Pro Val Pro Ser Arg Leu Val Pro Leu Pro Leu Leu
       1               5                   10                  15
   ctg ctc agc agc ctc tcg ctg ctg gca gcc cga gcg aat gca gac atc      216
   Leu Leu Ser Ser Leu Ser Leu Leu Ala Ala Arg Ala Asn Ala Asp Ile
                   20                  25                  30
   tcc atg gag gct tgc tgc acg gat cca aat cag atg gct aac cag cac      264
   Ser Met Glu Ala Cys Cys Thr Asp Pro Asn Gln Met Ala Asn Gln His
               35                  40                  45
   agg gac tgc tcg ctg ccg tac acc tca gaa tcc aag gag tgc agg atg      312
   Arg Asp Cys Ser Leu Pro Tyr Thr Ser Glu Ser Lys Glu Cys Arg Met
           50                  55                  60
   gtc cag gag caa tgt tgt cac aac caa ctg gaa gag ctg cac tgt gcc      360
   Val Gln Glu Gln Cys Cys His Asn Gln Leu Glu Glu Leu His Cys Ala
       65                  70                  75
   acg ggc atc aac ctg gcc agc gag cca gaa ggc tgc gcc tcg ctc cac      408
   Thr Gly Ile Asn Leu Ala Ser Glu Pro Glu Gly Cys Ala Ser Leu His
   80                  85                  90                  95
   agc tac aac agt agc ctt gag acc atc ttc ata aag agg tgc tgc cac      456
   Ser Tyr Asn Ser Ser Leu Glu Thr Ile Phe Ile Lys Arg Cys Cys His
                   100                 105                 110
   tgt tgc atg ctg gga aag gca tcc ctg gcc cga gac cag acc tgt gaa      504
   Cys Cys Met Leu Gly Lys Ala Ser Leu Ala Arg Asp Gln Thr Cys Glu
               115                 120                 125
   ccc att gtc atg ata agc tac cag tgt ggg ctg gtg ttc cgt gcc tgc      552
   Pro Ile Val Met Ile Ser Tyr Gln Cys Gly Leu Val Phe Arg Ala Cys
           130                 135                 140
   tgt gtg aag gcc cgg gag aat tca gac ttt gtc caa ggc aac ggt gca      600
   Cys Val Lys Ala Arg Glu Asn Ser Asp Phe Val Gln Gly Asn Gly Ala
       145                 150                 155
   gac ctt cag gac cca gct aag att cct gac gag gag gac caa gaa gac      648
   Asp Leu Gln Asp Pro Ala Lys Ile Pro Asp Glu Glu Asp Gln Glu Asp
   160                 165                 170                 175
   ccg tac ctg aat gac cgc tgt cga ggt ggc ggg ccc tgt aag cag cag      696
   Pro Tyr Leu Asn Asp Arg Cys Arg Gly Gly Gly Pro Cys Lys Gln Gln
                   180                 185                 190
   tgc cgt gac act ggg gac gag gtg atc tgc tct tgc ttt gtg ggc tac      744
   Cys Arg Asp Thr Gly Asp Glu Val Ile Cys Ser Cys Phe Val Gly Tyr
               195                 200                 205
   cag ctg cag tcg gat ggt gtc tcc tgc gaa gat atc aat gaa tgc atc      792
   Gln Leu Gln Ser Asp Gly Val Ser Cys Glu Asp Ile Asn Glu Cys Ile
           210                 215                 220
   aca ggc agc cat aac tgc cgg ctg gga gaa tcc tgc atc aat aca gtg      840
   Thr Gly Ser His Asn Cys Arg Leu Gly Glu Ser Cys Ile Asn Thr Val
       225                 230                 235
   ggc tct ttc cgc tgc cag cgg gac agc agc tgt ggg act ggc tat gag      888
   Gly Ser Phe Arg Cys Gln Arg Asp Ser Ser Cys Gly Thr Gly Tyr Glu
   240                 245                 250                 255
   ctc aca gag gat aat aac tgc aaa gat att gac gaa tgt gag act ggt      936
   Leu Thr Glu Asp Asn Asn Cys Lys Asp Ile Asp Glu Cys Glu Thr Gly
                   260                 265                 270
   att cat aac tgc ccc ccc gat ttt atc tgt cag aat act ctg gga tcc      984
   Ile His Asn Cys Pro Pro Asp Phe Ile Cys Gln Asn Thr Leu Gly Ser
                   275                 280                 285
   ttc cgt tgc aga ccg aag ctg cag tgc aag agc ggc ttc ata cag gat     1032
   Phe Arg Cys Arg Pro Lys Leu Gln Cys Lys Ser Gly Phe Ile Gln Asp
           290                 295                 300
   gct cta ggc aac tgc att gat atc aat gag tgt tta agt atc agt gct     1080
   Ala Leu Gly Asn Cys Ile Asp Ile Asn Glu Cys Leu Ser Ile Ser Ala
       305                 310                 315
   cca tgc cct gtg ggg cag aca tgc atc aat aca gag ggc tcc tac aca     1128
   Pro Cys Pro Val Gly Gln Thr Cys Ile Asn Thr Glu Gly Ser Tyr Thr
   320                 325                 330                 335
   tgc cag aag aat gtg ccc aac tgt ggc cgt ggt tat cat ctc aat gaa     1176
   Cys Gln Lys Asn Val Pro Asn Cys Gly Arg Gly Tyr His Leu Asn Glu
                   340                 345                 350
   gag ggg acc cgc tgt gtt gat gtg gat gag tgc gcc cca cca gcg gag     1224
   Glu Gly Thr Arg Cys Val Asp Val Asp Glu Cys Ala Pro Pro Ala Glu
               355                 360                 365
   ccc tgt ggg aag gga cac cac tgc ctg aac tcc ccc ggc agc ttc cgc     1272
   Pro Cys Gly Lys Gly His His Cys Leu Asn Ser Pro Gly Ser Phe Arg
           370                 375                 380
   tgc gag tgc aag gct ggc ttc tat ttt gat ggc atc agc agg acc tgc     1320
   Cys Glu Cys Lys Ala Gly Phe Tyr Phe Asp Gly Ile Ser Arg Thr Cys
       385                 390                 395
   gtg gat atc aac gag tgc cag cgc tat ccc ggg cgc ctg tgt ggc cac     1368
   Val Asp Ile Asn Glu Cys Gln Arg Tyr Pro Gly Arg Leu Cys Gly His
   400                 405                 410                 415
   aag tgt gag aac acg cca ggc tcc ttc cac tgc agc tgc tcc gcc ggc     1416
   Lys Cys Glu Asn Thr Pro Gly Ser Phe His Cys Ser Cys Ser Ala Gly
                   420                 425                 430
   ttc cgg ctg tct gtg gac ggc cgg tct tgt gaa gat gtg aac gag tgt     1464
   Phe Arg Leu Ser Val Asp Gly Arg Ser Cys Glu Asp Val Asn Glu Cys
               435                 440                 445
   ctc aac agc cct tgc agc cag gag tgt gct aat gtc tat ggc tcc tac     1512
   Leu Asn Ser Pro Cys Ser Gln Glu Cys Ala Asn Val Tyr Gly Ser Tyr
           450                 455                 460
   cag tgc tat tgc cga cga ggt tac cag ctc agt gac gtg gat ggg gtc     1560
   Gln Cys Tyr Cys Arg Arg Gly Tyr Gln Leu Ser Asp Val Asp Gly Val
       465                 470                 475
   acc tgc gaa gat att gat gag tgt gcc ctg ccc act gga ggt cac atc     1608
   Thr Cys Glu Asp Ile Asp Glu Cys Ala Leu Pro Thr Gly Gly His Ile
   480                 485                 490                 495
   tgc tcc tac cgc tgc atc aac atc ccc gga agc ttc cag tgc agc tgc     1656
   Cys Ser Tyr Arg Cys Ile Asn Ile Pro Gly Ser Phe Gln Cys Ser Cys
                   500                 505                 510
   ccc tca tct ggc tac agg cta gct ccc aat ggc cgc aac tgc caa gac     1704
   Pro Ser Ser Gly Tyr Arg Leu Ala Pro Asn Gly Arg Asn Cys Gln Asp
               515                 520                 525
   att gat gag tgt gtg acc ggc atc cat aac tgt tcc atc aat gag act     1752
   Ile Asp Glu Cys Val Thr Gly Ile His Asn Cys Ser Ile Asn Glu Thr
           530                 535                 540
   tgc ttc aac atc cag ggc agc ttc cgc tgt ctg tcc ttt gaa tgc ccc      1800
   Cys Phe Asn Ile Gln Gly Ser Phe Arg Cys Leu Ser Phe Glu Cys Pro
       545                 550                 555
   gag aac tat cgc cgc tcc gca gac acc ttc cgc caa gag aaa aca gac      1848
   Glu Asn Tyr Arg Arg Ser Ala Asp Thr Phe Arg Gln Glu Lys Thr Asp
   560                 565                 570                 575
   acc gtc cgc tgc atc aag tct tgc cgt ccc aac gat gag gcc tgc gtg      1896
   Thr Val Arg Cys Ile Lys Ser Cys Arg Pro Asn Asp Glu Ala Cys Val
                   580                 585                 590
   cgg gac cct gta cat acc gtc tcc cac acc gtc atc tcg ctg ccc acc      1944
   Arg Asp Pro Val His Thr Val Ser His Thr Val Ile Ser Leu Pro Thr
               595                 600                 605
   ttt cga gag ttc acc cgt cct gag gag atc atc ttt ctg agg gct gtc      1992
   Phe Arg Glu Phe Thr Arg Pro Glu Glu Ile Ile Phe Leu Arg Ala Val
           610                 615                 620
   aca ccg ctg tac ccc gcc aac cag gcc gac atc atc ttc gac atc aca      2040
   Thr Pro Leu Tyr Pro Ala Asn Gln Ala Asp Ile Ile Phe Asp Ile Thr
       625                 630                 635
   gaa ggg aac ctt cga gac tcc ttt gat atc atc aag cgc tat gag gac      2088
   Glu Gly Asn Leu Arg Asp Ser Phe Asp Ile Ile Lys Arg Tyr Glu Asp
   640                 645                 650                 655
   ggc atg act gtg ggt gtc gtg cgc caa gtg cgg ccc atc gtg ggc ccg      2136
   Gly Met Thr Val Gly Val Val Arg Gln Val Arg Pro Ile Val Gly Pro
                   660                 665                 670
   ttt tac gct gtc ctg aag ctg gag atg aac tac gtg ttg gga ggc gta      2184
   Phe Tyr Ala Val Leu Lys Leu Glu Met Asn Tyr Val Leu Gly Gly Val
               675                 680                 685
   gtt tcc cac cgg aac gtc gtc aat gta cac atc ttc gtc tcc gag tac      2232
   Val Ser His Arg Asn Val Val Asn Val His Ile Phe Val Ser Glu Tyr
           690                 695                 700
   tgg ttc tga gggccgggtt gcggctcagc caggagtgtg ccacgccctt              2281
   Trp Phe
       705
   gctgccagtg acagccaggt gcctgtctct acccctcggg cctcccttga tgtttcatat    2341
   tggtttgtat ggccacgtgc attaggctga gccgaatcac ttaagtccag ctggtgtact    2401
   gtggcgttta aacatggctc agccaggtgg ttgaatcatt gctttttttt ttttttaaat    2461
   gcaaaggcca aagcggaatt ttgttccctt ttctacctgt gagctaggca ttgctaagga    2521
   ccaattgaag gagttttcaa agcagactcc acccgaaagc cgaggcagcc agttctcact    2581
   gagtctgcat gaggattggc attttggtac tttttttttt ttaaccaact gggctctttg    2641
   tgtttcaagt tgatggctgc tgtagagtgg cgcataaata aatgtacaat aaattctccc    2701
   cagaaaaa                                                             2709
<210> 55
   <211> 705
   <212> PRT
   <213> Mus musculus
<400> 55
   Met Glu Arg Pro Val Pro Ser Arg Leu Val Pro Leu Pro Leu Leu Leu
   1               5                   10                  15
   Leu Ser Ser Leu Ser Leu Leu Ala Ala Arg Ala Asn Ala Asp Ile Ser
               20                  25                  30
   Met Glu Ala Cys Cys Thr Asp Pro Asn Gln Met Ala Asn Gln His Arg
           35                  40                  45
   Asp Cys Ser Leu Pro Tyr Thr Ser Glu Ser Lys Glu Cys Arg Met Val
       50                  55                  60
   Gln Glu Gln Cys Cys His Asn Gln Leu Glu Glu Leu His Cys Ala Thr
   65                  70                  75                  80
   Gly Ile Asn Leu Ala Ser Glu Pro Glu Gly Cys Ala Ser Leu His Ser
                   85                  90                  95
   Tyr Asn Ser Ser Leu Glu Thr Ile Phe Ile Lys Arg Cys Cys His Cys
               100                 105                 110
   Cys Met Leu Gly Lys Ala Ser Leu Ala Arg Asp Gln Thr Cys Glu Pro
           115                 120                 125
   Ile Val Met Ile Ser Tyr Gln Cys Gly Leu Val Phe Arg Ala Cys Cys
       130                 135                 140
   Val Lys Ala Arg Glu Asn Ser Asp Phe Val Gln Gly Asn Gly Ala Asp
   145                 150                 155                 160
   Leu Gln Asp Pro Ala Lys Ile Pro Asp Glu Glu Asp Gln Glu Asp Pro
                   165                 170                 175
   Tyr Leu Asn Asp Arg Cys Arg Gly Gly Gly Pro Cys Lys Gln Gln Cys
               180                 185                 190
   Arg Asp Thr Gly Asp Glu Val Ile Cys Ser Cys Phe Val Gly Tyr Gln
           195                 200                 205
   Leu Gln Ser Asp Gly Val Ser Cys Glu Asp Ile Asn Glu Cys Ile Thr
       210                 215                 220
   Gly Ser His Asn Cys Arg Leu Gly Glu Ser Cys Ile Asn Thr Val Gly
   225                 230                 235                 240
   Ser Phe Arg Cys Gln Arg Asp Ser Ser Cys Gly Thr Gly Tyr Glu Leu
                   245                 250                 255
   Thr Glu Asp Asn Asn Cys Lys Asp Ile Asp Glu Cys Glu Thr Gly Ile
               260                 265                 270
   His Asn Cys Pro Pro Asp Phe Ile Cys Gln Asn Thr Leu Gly Ser Phe
   275 280 285
   Arg Cys Arg Pro Lys Leu Gln Cys Lys Ser Gly Phe Ile Gln Asp Ala
   290 295 300
   Leu Gly Asn Cys Ile Asp Ile Asn Glu Cys Leu Ser Ile Ser Ala Pro
   305 310 315 320
   Cys Pro Val Gly Gln Thr Cys Ile Asn Thr Glu Gly Ser Tyr Thr Cys
   325 330 335
   Gln Lys Asn Val Pro Asn Cys Gly Arg Gly Tyr His Leu Asn Glu Glu
   340 345 350
   Gly Thr Arg Cys Val Asp Val Asp Glu Cys Ala Pro Pro Ala Glu Pro
   355 360 365
   Cys Gly Lys Gly His His Cys Leu Asn Ser Pro Gly Ser Phe Arg Cys
   370 375 380
   Glu Cys Lys Ala Gly Phe Tyr Phe Asp Gly Ile Ser Arg Thr Cys Val
   385 390 395 400
   Asp Ile Asn Glu Cys Gln Arg Tyr Pro Gly Arg Leu Cys Gly His Lys
   405 410 415
   Cys Glu Asn Thr Pro Gly Ser Phe His Cys Ser Cys Ser Ala Gly Phe
   420 425 430
   Arg Leu Ser Val Asp Gly Arg Ser Cys Glu Asp Val Asn Glu Cys Leu
   435 440 445
   Asn Ser Pro Cys Ser Gln Glu Cys Ala Asn Val Tyr Gly Ser Tyr Gln
   450 455 460
   Cys Tyr Cys Arg Arg Gly Tyr Gln Leu Ser Asp Val Asp Gly Val Thr
   465 470 475 480
   Cys Glu Asp Ile Asp Glu Cys Ala Leu Pro Thr Gly Gly His Ile Cys
   485 490 495
   Ser Tyr Arg Cys Ile Asn Ile Pro Gly Ser Phe Gln Cys Ser Cys Pro
   500 505 510
   Ser Ser Gly Tyr Arg Leu Ala Pro Asn Gly Arg Asn Cys Gln Asp Ile
   515 520 525
   Asp Glu Cys Val Thr Gly Ile His Asn Cys Ser Ile Asn Glu Thr Cys
   530 535 540
   Phe Asn Ile Gln Gly Ser Phe Arg Cys Leu Ser Phe Glu Cys Pro Glu
   545 550 555 560
   Asn Tyr Arg Arg Ser Ala Asp Thr Phe Arg Gln Glu Lys Thr Asp Thr
   565 570 575
   Val Arg Cys Ile Lys Ser Cys Arg Pro Asn Asp Glu Ala Cys Val Arg
   580 585 590
   Asp Pro Val His Thr Val Ser His Thr Val Ile Ser Leu Pro Thr Phe
   595 600 605
   Arg Glu Phe Thr Arg Pro Glu Glu Ile Ile Phe Leu Arg Ala Val Thr
   610 615 620
   Pro Leu Tyr Pro Ala Asn Gln Ala Asp Ile Ile Phe Asp Ile Thr Glu
   625 630 635 640
   Gly Asn Leu Arg Asp Ser Phe Asp Ile Ile Lys Arg Tyr Glu Asp Gly
   645 650 655
   Met Thr Val Gly Val Val Arg Gln Val Arg Pro Ile Val Gly Pro Phe
   660 665 670
   Tyr Ala Val Leu Lys Leu Glu Met Asn Tyr Val Leu Gly Gly Val Val
   675 680 685
   Ser His Arg Asn Val Val Asn Val His Ile Phe Val Ser Glu Tyr Trp
   690 695 700
   Phe
   705
<210> 56
   <211> 2947
   <212> DNA
   <213> Homo sapiens
<220>
   <221> CDS
   <222> (148)..(2259)
   <223> SF12, cDNA: NM_006486, Protein: NP_006477
<400> 56
   ctcctcccgg gcgggataat tgaacggcgc ggccctggcc cagcgttggc tgccgaggct      60
   cggccggagc gtggagcccg cgccgctgcc ccaggaccgc gcccgcgcct ttgtccgccg     120
   ccgcccaccg cccgtcgccc gccgccc atg gag cgc gcc gcg ccg tcg cgc cgg     174
                                 Met Glu Arg Ala Ala Pro Ser Arg Arg
                                 1               5
   gtc ccg ctt ccg ctg ctg ctg ctc ggc ggc ctt gcg ctg ctg gcg gcc       222
   Val Pro Leu Pro Leu Leu Leu Leu Gly Gly Leu Ala Leu Leu Ala Ala
   10                  15                  20                  25
   gga gtg gac gcg gat gtc ctc ctg gag gcc tgc tgt gcg gac gga cac      270
   Gly Val Asp Ala Asp Val Leu Leu Glu Ala Cys Cys Ala Asp Gly His
                   30                  35                  40
   cgg atg gcc act cat cag aag gac tgc tcg ctg cca tat gct acg gaa      318
   Arg Met Ala Thr His Gln Lys Asp Cys Ser Leu Pro Tyr Ala Thr Glu
               45                  50                  55
   tcc aaa gaa tgc agg atg gtg cag gag cag tgc tgc cac agc cag ctg      366
   Ser Lys Glu Cys Arg Met Val Gln Glu Gln Cys Cys His Ser Gln Leu
           60                  65                  70
   gag gag ctg cac tgt gcc acg ggc atc agc ctg gcc aac gag cag gac      414
   Glu Glu Leu His Cys Ala Thr Gly Ile Ser Leu Ala Asn Glu Gln Asp
       75                  80                  85
   cgc tgt gcc acg ccc cac ggt gac aac gcc agc ctg gag gcc aca ttt      462
   Arg Cys Ala Thr Pro His Gly Asp Asn Ala Ser Leu Glu Ala Thr Phe
   90                  95                  100                 105
   gtg aag agg tgc tgc cat tgc tgt ctg ctg ggg agg gcg gcc cag gcc      510
   Val Lys Arg Cys Cys His Cys Cys Leu Leu Gly Arg Ala Ala Gln Ala
                   110                 115                 120
   cag ggc cag agc tgc gag tac agc ctc atg gtt ggc tac cag tgt gga      558
   Gln Gly Gln Ser Cys Glu Tyr Ser Leu Met Val Gly Tyr Gln Cys Gly
               125                 130                 135
   cag gtc ttc cgg gca tgc tgt gtc aag agc cag gag acc gga gat ttg      606
   Gln Val Phe Arg Ala Cys Cys Val Lys Ser Gln Glu Thr Gly Asp Leu
           140                 145                 150
   gat gtc ggg ggc ctc caa gaa acg gat aag atc att gag gtt gag gag      654
   Asp Val Gly Gly Leu Gln Glu Thr Asp Lys Ile Ile Glu Val Glu Glu
       155                 160                 165
   gaa caa gag gac cca tat ctg aat gac cgc tgc cga gga ggc ggg ccc      702
   Glu Gln Glu Asp Pro Tyr Leu Asn Asp Arg Cys Arg Gly Gly Gly Pro
   170                 175                 180                 185
   tgc aag cag cag tgc cga gac acg ggt gac gag gtg gtc tgc tcc tgc      750
   Cys Lys Gln Gln Cys Arg Asp Thr Gly Asp Glu Val Val Cys Ser Cys
                   190                 195                 200
   ttc gtg ggc tac cag ctg ctg tct gat ggt gtc tcc tgt gaa gat gtc      798
   Phe Val Gly Tyr Gln Leu Leu Ser Asp Gly Val Ser Cys Glu Asp Val
               205                 210                 215
   aat gaa tgc atc acg ggc agc cac agc tgc cgg ctt gga gaa tcc tgc      846
   Asn Glu Cys Ile Thr Gly Ser His Ser Cys Arg Leu Gly Glu Ser Cys
           220                 225                 230
   atc aac aca gtg ggc tct ttc cgc tgc cag cgg gac agc agc tgc ggg      894
   Ile Asn Thr Val Gly Ser Phe Arg Cys Gln Arg Asp Ser Ser Cys Gly
       235                 240                 245
   act ggc tat gag ctc aca gag gac aat agc tgc aaa gat att gac gag      942
   Thr Gly Tyr Glu Leu Thr Glu Asp Asn Ser Cys Lys Asp Ile Asp Glu
   250                 255                 260                 265
   tgt gag agt ggt att cat aac tgc ctc ccc gat ttt atc tgt cag aat      990
   Cys Glu Ser Gly Ile His Asn Cys Leu Pro Asp Phe Ile Cys Gln Asn
                   270                 275                 280
   act ctg gga tcc ttc cgc tgc cga ccc aag cta cag tgc aag agt ggc     1038
   Thr Leu Gly Ser Phe Arg Cys Arg Pro Lys Leu Gln Cys Lys Ser Gly
               285                 290                 295
   ttt ata caa gat gct cta ggc aac tgt att gat atc aat gag tgt ttg      1086
   Phe Ile Gln Asp Ala Leu Gly Asn Cys Ile Asp Ile Asn Glu Cys Leu
           300                 305                 310
   agt atc agt gcc ccg tgc cct atc ggg cat aca tgc atc aac aca gag      1134
   Ser Ile Ser Ala Pro Cys Pro Ile Gly His Thr Cys Ile Asn Thr Glu
       315                 320                 325
   ggc tcc tac acg tgc cag aag aac gtg ccc aac tgt ggc cgt ggc tac      1182
   Gly Ser Tyr Thr Cys Gln Lys Asn Val Pro Asn Cys Gly Arg Gly Tyr
   330                 335                 340                 345
   cat ctc aac gag gag gga acg cgc tgt gtt gat gtg gac gag tgc gcg      1230
   His Leu Asn Glu Glu Gly Thr Arg Cys Val Asp Val Asp Glu Cys Ala
                   350                 355                 360
   cca cct gct gag ccc tgt ggg aag gga cat cgc tgc gtg aac tct ccc      1278
   Pro Pro Ala Glu Pro Cys Gly Lys Gly His Arg Cys Val Asn Ser Pro
               365                 370                 375
   ggc agt ttc cgc tgc gaa tgc aag acg ggt tac tat ttt gac ggc atc      1326
   Gly Ser Phe Arg Cys Glu Cys Lys Thr Gly Tyr Tyr Phe Asp Gly Ile
           380                 385                 390
   agc agg atg tgt gtc gat gtc aac gag tgc cag cgc tac ccc ggg cgc      1374
   Ser Arg Met Cys Val Asp Val Asn Glu Cys Gln Arg Tyr Pro Gly Arg
       395                 400                 405
   ctg tgt ggc cac aag tgc gag aac acg ctg ggc tcc tac ctc tgc agc      1422
   Leu Cys Gly His Lys Cys Glu Asn Thr Leu Gly Ser Tyr Leu Cys Ser
   410                 415                 420                 425
   tgt tcc gtg ggc ttc cgg ctc tct gtg gat ggc agg tca tgt gaa gac      1470
   Cys Ser Val Gly Phe Arg Leu Ser Val Asp Gly Arg Ser Cys Glu Asp
                   430                 435                 440
   atc aat gag tgc agc agc agc ccc tgt agc cag gag tgt gcc aac gtc      1518
   Ile Asn Glu Cys Ser Ser Ser Pro Cys Ser Gln Glu Cys Ala Asn Val
               445                 450                 455
   tac ggc tcc tac cag tgt tac tgc cgg cga ggc tac cag ctc agc gat      1566
   Tyr Gly Ser Tyr Gln Cys Tyr Cys Arg Arg Gly Tyr Gln Leu Ser Asp
           460                 465                 470
   gtg gat gga gtc acc tgt gaa gac atc gac gag tgc gcc ctg ccc acc      1614
   Val Asp Gly Val Thr Cys Glu Asp Ile Asp Glu Cys Ala Leu Pro Thr
       475                 480                 485
   ggg ggc cac atc tgc tcc tac cgc tgc atc aac atc cct gga agc ttc      1662
   Gly Gly His Ile Cys Ser Tyr Arg Cys Ile Asn Ile Pro Gly Ser Phe
   490                 495                 500                 505
   cag tgc agc tgc ccc tcg tct ggc tac agg ctg gcc ccc aat ggc cgc      1710
   Gln Cys Ser Cys Pro Ser Ser Gly Tyr Arg Leu Ala Pro Asn Gly Arg
                   510                 515                 520
   aac tgc caa gac att gat gag tgt gtg act ggc atc cac aac tgc tcc      1758
   Asn Cys Gln Asp Ile Asp Glu Cys Val Thr Gly Ile His Asn Cys Ser
               525                 530                 535
   atc aac gag acc tgc ttc aac atc cag ggc ggc ttc cgc tgc ctg gcc      1806
   Ile Asn Glu Thr Cys Phe Asn Ile Gln Gly Gly Phe Arg Cys Leu Ala
           540                 545                 550
   ttc gag tgc cct gag aac tac cgc cgc tcc gca gcc acg ctc cag cag      1854
   Phe Glu Cys Pro Glu Asn Tyr Arg Arg Ser Ala Ala Thr Leu Gln Gln
       555                 560                 565
   gag aag aca gac acg gtc cgc tgc atc aag tcc tgc cgc ccc aac gat      1902
   Glu Lys Thr Asp Thr Val Arg Cys Ile Lys Ser Cys Arg Pro Asn Asp
   570                 575                 580                 585
   gtc aca tgc gtg ttc gac ccc gtg cac acc atc tcc cac acc gtc atc      1950
   Val Thr Cys Val Phe Asp Pro Val His Thr Ile Ser His Thr Val Ile
                   590                 595                 600
   tcg ctg cct acc ttc cgc gag ttc acc cgc cct gaa gag atc atc ttc      1998
   Ser Leu Pro Thr Phe Arg Glu Phe Thr Arg Pro Glu Glu Ile Ile Phe
               605                 610                 615
   ctc cgg gcc atc acg cca ccg cat cct gcc agc cag gct aac atc atc      2046
   Leu Arg Ala Ile Thr Pro Pro His Pro Ala Ser Gln Ala Asn Ile Ile
           620                 625                 630
   ttc gac atc acg gaa ggg aac ctg cgg gac tct ttt gac atc atc aag      2094
   Phe Asp Ile Thr Glu Gly Asn Leu Arg Asp Ser Phe Asp Ile Ile Lys
       635                 640                 645
   cgt tac atg gac ggc atg acc gtg ggt gtc gtg cgc cag gtg cgg ccc      2142
   Arg Tyr Met Asp Gly Met Thr Val Gly Val Val Arg Gln Val Arg Pro
   650                 655                 660                 665
   atc gtg ggc cca ttt cat gcc gtc ctg aag ctg gag atg aac tat gtg      2190
   Ile Val Gly Pro Phe His Ala Val Leu Lys Leu Glu Met Asn Tyr Val
                   670                 675                 680
   gtc ggg ggc gtg gtc tcc cac cga aat gtt gtc aac gtc cac atc ttc      2238
   Val Gly Gly Val Val Ser His Arg Asn Val Val Asn Val His Ile Phe
               685                 690                 695
   gtc tct gag tac tgg ttc tga gggctggtct gccgcacagc cgcaggtgca         2289
   Val Ser Glu Tyr Trp Phe
           700
   cctccaggcc aaatcattgc tgccagtgac tgtggtctgt acttgtttat accctcagac    2349
   ttttttaatg ttaggtattt gtagcattag gccaacatgt attaagctga gccagatgaa     2409
   taagtccatc tgatgtattt tcggtgttta aaaaatgagc ccagttgctc aactgtttgg    2469
   ttgaaaacct tgctcatttt ttaatgcgaa ggctaagtgt cacccccttt ctctgcctct     2529
   ggctgggcct tgctaagggc caaggaaaga aagacatttt ttagggggca gccagtccaa    2589
   atgccaaaag aagaccagtt cttgccctga ttgtatgaaa tttgacattt tggcactttt    2649
   tttttttttt tggccaatca gattttctat gttctaagga catggctgct gtagaatagc     2709
   acagacgtgg atgataaatt atccccagaa gcagcatgac agaatgcctc ggggagcact    2769
   tggaagggaa attgcagttc tgttgaaata gaggaaaatc ccttggtaaa gacacagcct    2829
   gttaggctcg tgtgggcctc cagtatgttc accaggggaa tggctgggat ttctcggcac    2889
   tctgcatcat ccatcttttc ttataggtgg gaaaataaac aactttgtga tcctcctg      2947
<210> 57
   <211> 703
   <212> PRT
   <213> Homo sapiens
<400> 57
   Met Glu Arg Ala Ala Pro Ser Arg Arg Val Pro Leu Pro Leu Leu Leu
   1               5                   10                  15
   Leu Gly Gly Leu Ala Leu Leu Ala Ala Gly Val Asp Ala Asp Val Leu
               20                  25                  30
   Leu Glu Ala Cys Cys Ala Asp Gly His Arg Met Ala Thr His Gln Lys
           35                  40                  45
   Asp Cys Ser Leu Pro Tyr Ala Thr Glu Ser Lys Glu Cys Arg Met Val
       50                  55                  60
   Gln Glu Gln Cys Cys His Ser Gln Leu Glu Glu Leu His Cys Ala Thr
   65                  70                  75                  80
   Gly Ile Ser Leu Ala Asn Glu Gln Asp Arg Cys Ala Thr Pro His Gly
                   85                  90                  95
   Asp Asn Ala Ser Leu Glu Ala Thr Phe Val Lys Arg Cys Cys His Cys
               100                 105                 110
   Cys Leu Leu Gly Arg Ala Ala Gln Ala Gln Gly Gln Ser Cys Glu Tyr
           115                 120                 125
   Ser Leu Met Val Gly Tyr Gln Cys Gly Gln Val Phe Arg Ala Cys Cys
       130                 135                 140
   Val Lys Ser Gln Glu Thr Gly Asp Leu Asp Val Gly Gly Leu Gln Glu
   145                 150                 155                 160
   Thr Asp Lys Ile Ile Glu Val Glu Glu Glu Gln Glu Asp Pro Tyr Leu
                   165                 170                 175
   Asn Asp Arg Cys Arg Gly Gly Gly Pro Cys Lys Gln Gln Cys Arg Asp
               180                 185                 190
   Thr Gly Asp Glu Val Val Cys Ser Cys Phe Val Gly Tyr Gln Leu Leu
           195                 200                 205
   Ser Asp Gly Val Ser Cys Glu Asp Val Asn Glu Cys Ile Thr Gly Ser
       210                 215                 220
   His Ser Cys Arg Leu Gly Glu Ser Cys Ile Asn Thr Val Gly Ser Phe
   225                 230                 235                 240
   Arg Cys Gln Arg Asp Ser Ser Cys Gly Thr Gly Tyr Glu Leu Thr Glu
                   245                 250                 255
   Asp Asn Ser Cys Lys Asp Ile Asp Glu Cys Glu Ser Gly Ile His Asn
               260                 265                 270
   Cys Leu Pro Asp Phe Ile Cys Gln Asn Thr Leu Gly Ser Phe Arg Cys
               275                 280                 285
   Arg Pro Lys Leu Gln Cys Lys Ser Gly Phe Ile Gln Asp Ala Leu Gly
       290                 295                 300
   Asn Cys Ile Asp Ile Asn Glu Cys Leu Ser Ile Ser Ala Pro Cys Pro
   305                 310                 315                 320
   Ile Gly His Thr Cys Ile Asn Thr Glu Gly Ser Tyr Thr Cys Gln Lys
                   325                 330                 335
   Asn Val Pro Asn Cys Gly Arg Gly Tyr His Leu Asn Glu Glu Gly Thr
               340                 345                 350
   Arg Cys Val Asp Val Asp Glu Cys Ala Pro Pro Ala Glu Pro Cys Gly
           355                 360                 365
   Lys Gly His Arg Cys Val Asn Ser Pro Gly Ser Phe Arg Cys Glu Cys
       370                 375                 380
   Lys Thr Gly Tyr Tyr Phe Asp Gly Ile Ser Arg Met Cys Val Asp Val
   385                 390                 395                 400
   Asn Glu Cys Gln Arg Tyr Pro Gly Arg Leu Cys Gly His Lys Cys Glu
                   405                 410                 415
   Asn Thr Leu Gly Ser Tyr Leu Cys Ser Cys Ser Val Gly Phe Arg Leu
               420                 425                 430
   Ser Val Asp Gly Arg Ser Cys Glu Asp Ile Asn Glu Cys Ser Ser Ser
           435                 440                 445
   Pro Cys Ser Gln Glu Cys Ala Asn Val Tyr Gly Ser Tyr Gln Cys Tyr
       450                 455                 460
   Cys Arg Arg Gly Tyr Gln Leu Ser Asp Val Asp Gly Val Thr Cys Glu
   465                 470                 475                 480
   Asp Ile Asp Glu Cys Ala Leu Pro Thr Gly Gly His Ile Cys Ser Tyr
                   485                 490                 495
   Arg Cys Ile Asn Ile Pro Gly Ser Phe Gln Cys Ser Cys Pro Ser Ser
               500                 505                 510
   Gly Tyr Arg Leu Ala Pro Asn Gly Arg Asn Cys Gln Asp Ile Asp Glu
           515                 520                 525
   Cys Val Thr Gly Ile His Asn Cys Ser Ile Asn Glu Thr Cys Phe Asn
       530                 535                 540
   Ile Gln Gly Gly Phe Arg Cys Leu Ala Phe Glu Cys Pro Glu Asn Tyr
   545                 550                 555                 560
   Arg Arg Ser Ala Ala Thr Leu Gln Gln Glu Lys Thr Asp Thr Val Arg
                   565                 570                 575
   Cys Ile Lys Ser Cys Arg Pro Asn Asp Val Thr Cys Val Phe Asp Pro
               580                 585                 590
   Val His Thr Ile Ser His Thr Val Ile Ser Leu Pro Thr Phe Arg Glu
           595                 600                 605
   Phe Thr Arg Pro Glu Glu Ile Ile Phe Leu Arg Ala Ile Thr Pro Pro
       610                 615                 620
   His Pro Ala Ser Gln Ala Asn Ile Ile Phe Asp Ile Thr Glu Gly Asn
   625                 630                 635                 640
   Leu Arg Asp Ser Phe Asp Ile Ile Lys Arg Tyr Met Asp Gly Met Thr
                   645                 650                 655
   Val Gly Val Val Arg Gln Val Arg Pro Ile Val Gly Pro Phe His Ala
               660                 665                 670
   Val Leu Lys Leu Glu Met Asn Tyr Val Val Gly Gly Val Val Ser His
           675                 680                 685
   Arg Asn Val Val Asn Val His Ile Phe Val Ser Glu Tyr Trp Phe
       690                 695                 700
<210> 58
   <211> 979
   <212> DNA
   <213> Mus musculus
<220>
   <221> CDS
   <222> (98) .. (748)
   <223> SF13, cDNA: NM_011149, Protein: NP_035279
<400> 58
   cccgggtcca ccccacgcct gggcggccac gcgcacgctg cgcgtccaca cccttttccg     60
   gtccgggctg cccgaccgct cttgctgctg ccggtgg atg ctg cgc ctc tcg gag     115
                                            Met Leu Arg Leu Ser Glu
                                            1               5
   cgc aat atg aag gtg ctc ttc gcc gcc gcc ctc atc gtg ggc tcc gtc     163
   Arg Asn Met Lys Val Leu Phe Ala Ala Ala Leu Ile Val Gly Ser Val
               10                  15                  20
   gtc ttc ctt ttg ctg ccc gga ccc tcc gtg gcc aac gat aag aag aag     211
   Val Phe Leu Leu Leu Pro Gly Pro Ser Val Ala Asn Asp Lys Lys Lys
           25                  30                  35
   gga cct aaa gtc aca gtc aag gta tac ttt gat tta caa att gga gat     259
   Gly Pro Lys Val Thr Val Lys Val Tyr Phe Asp Leu Gln Ile Gly Asp
       40                  45                  50
   gaa tct gta gga cga gtc gtc ttt gga ctc ttt gga aag act gtt cca     307
   Glu Ser Val Gly Arg Val Val Phe Gly Leu Phe Gly Lys Thr Val Pro
   55                  60                  65                  70
   aaa aca gtg gat aat ttt gta gcc tta gct aca gga gag aaa gga ttt     355
   Lys Thr Val Asp Asn Phe Val Ala Leu Ala Thr Gly Glu Lys Gly Phe
                   75                  80                  85
   ggc tac aaa aac agc aag ttc cat cgt gtc atc aag gac ttc atg atc     403
   Gly Tyr Lys Asn Ser Lys Phe His Arg Val Ile Lys Asp Phe Met Ile
               90                  95                  100
   cag ggt gga gac ttc acc agg gga gat ggc aca gga gga aag agc atc     451
   Gln Gly Gly Asp Phe Thr Arg Gly Asp Gly Thr Gly Gly Lys Ser Ile
           105                 110                 115
   tat ggt gag cgc ttc cca gat gag aac ttc aag ctg aag cac tac ggg     499
   Tyr Gly Glu Arg Phe Pro Asp Glu Asn Phe Lys Leu Lys His Tyr Gly
       120                 125                 130
   cct ggc tgg gtg agc atg gcc aat gca ggc aaa gac acc aat ggc tca     547
   Pro Gly Trp Val Ser Met Ala Asn Ala Gly Lys Asp Thr Asn Gly Ser
   135                 140                 145                 150
   cag ttc ttc ata acc aca gtc aag acc tcc tgg ctg gat ggc aag cat     595
   Gln Phe Phe Ile Thr Thr Val Lys Thr Ser Trp Leu Asp Gly Lys His
                   155                 160                 165
   gtg gtt ttc ggc aaa gtt cta gag ggc atg gat gtg gta cgg aag gtg     643
   Val Val Phe Gly Lys Val Leu Glu Gly Met Asp Val Val Arg Lys Val
               170                 175                 180
   gag agc acc aag aca gac agc cgg gac aag cca ctg aag gat gtc atc     691
   Glu Ser Thr Lys Thr Asp Ser Arg Asp Lys Pro Leu Lys Asp Val Ile
           185                 190                 195
   att gtc gac tcc ggc aag atc gaa gtg gag aaa ccc ttc gcc att gcc     739
   Ile Val Asp Ser Gly Lys Ile Glu Val Glu Lys Pro Phe Ala Ile Ala
       200                 205                 210
   aag gag tag agagcctggg ggacctcatc cctctaagca gctgtctgtg            788
   Lys Glu
   215
   tgggtcctgt caatccccac acagacgaag gtagccagtc acaaggttct gtgccaccct  848
   ggccctagtg cttccatctg atggggtgac cacacccctc acattccaca ggcctgattt  908
   ttataaaaaa ctaccaatgc tgatcaataa agtgggtttt ttttatagct tgaaaaaaaa  968
   aaaaaaaaaa a                                                        979
<210> 59
   <211> 216
   <212> PRT
   <213> Mus musculus
<400> 59
   Met Leu Arg Leu Ser Glu Arg Asn Met Lys Val Leu Phe Ala Ala Ala
   1               5                   10                  15
   Leu Ile Val Gly Ser Val Val Phe Leu Leu Leu Pro Gly Pro Ser Val
               20                  25                  30
   Ala Asn Asp Lys Lys Lys Gly Pro Lys Val Thr Val Lys Val Tyr Phe
           35                  40                  45
   Asp Leu Gln Ile Gly Asp Glu Ser Val Gly Arg Val Val Phe Gly Leu
       50                  55                  60
   Phe Gly Lys Thr Val Pro Lys Thr Val Asp Asn Phe Val Ala Leu Ala
   65                  70                  75                  80
   Thr Gly Glu Lys Gly Phe Gly Tyr Lys Asn Ser Lys Phe His Arg Val
                   85                  90                  95
   Ile Lys Asp Phe Met Ile Gln Gly Gly Asp Phe Thr Arg Gly Asp Gly
               100                 105                 110
   Thr Gly Gly Lys Ser Ile Tyr Gly Glu Arg Phe Pro Asp Glu Asn Phe
           115                 120                 125
   Lys Leu Lys His Tyr Gly Pro Gly Trp Val Ser Met Ala Asn Ala Gly
       130                 135                 140
   Lys Asp Thr Asn Gly Ser Gln Phe Phe Ile Thr Thr Val Lys Thr Ser
   145                 150                 155                 160
   Trp Leu Asp Gly Lys His Val Val Phe Gly Lys Val Leu Glu Gly Met
                   165                 170                 175
   Asp Val Val Arg Lys Val Glu Ser Thr Lys Thr Asp Ser Arg Asp Lys
               180                 185                 190
   Pro Leu Lys Asp Val Ile Ile Val Asp Ser Gly Lys Ile Glu Val Glu
           195                 200                 205
   Lys Pro Phe Ala Ile Ala Lys Glu
       210                 215
<210> 60
   <211> 1045
   <212> DNA
   <213> Homo sapiens
<220>
   <221> CDS
   <222> (170)..(820)
   <223> SF13, cDNA: NM_000942, Protein: NP_000933
<400> 60
actatccggc gccgagccgg aggggggaaa cggcgcccgc cgcccgcccg gagcccgcga      60
   gcaaccccag tcccccccac ccgcgcgtgg cggcgccggc tccctagcca ccgcggcccc     120
   accctcttcc ggcctcagct gtccgggctg ctttcgcctc cgcctgtgg atg ctg cgc     178
   ctc tcc gaa cgc aac atg aag gtg ctc ctt gcc gcc gcc ctc atc gcg      226
   ggg tcc gtc ttc ttc ctg ctg ctg ccg gga cct tct gcg gcc gat gag      274
   aag aag aag ggg ccc aaa gtc acc gtc aag gtg tat ttt gac cta cga      322
   att gga gat gaa gat gta ggc cgg gtg atc ttt ggt ctc ttc gga aag      370
   act gtt cca aaa aca gtg gat aat ttt gtg gcc tta gct aca gga gag      418
   aaa gga ttt ggc tac aaa aac agc aaa ttc cat cgt gta atc aag gac      466
   ttc atg atc cag ggc gga gac ttc acc agg gga gat ggc aca gga gga      514
   aag agc atc tac ggt gag cgc ttc ccc gat gag aac ttc aaa ctg aag      562
   cac tac ggg cct ggc tgg gtg agc atg gcc aac gca ggc aaa gac acc      610
   aac ggc tcc cag ttc ttc atc acg aca gtc aag aca gcc tgg cta gat      658
   ggc aag cat gtg gtg ttt ggc aaa gtt cta gag ggc atg gag gtg gtg      706
   cgg aag gtg gag agc acc aag aca gac agc cgg gat aaa ccc ctg aag      754
   gat gtg atc atc gca gac tgc ggc aag atc gag gtg gag aag ccc ttt      802
   gcc atc gcc aag gag tag ggcacaggga catctttctt tgagtgaccg            850
   tctgtgcagg ccctgtagtc cgccacaggg ctctgagctg cactggcccc ggtgctggca   910
   tctggtggag cggacccact cccctcacat tccacaggcc catggactca cttttgtaac   970
   aaactcctac caacactgac caataaaaaa aaatgtgggt tttttttttt ttaatataaa   1030
   aaaaaaaaaa aaaaa                                                    1045
<210> 61
   <211> 216
   <212> PRT
   <213> Homo sapiens
<400> 61

## Description

This invention rotates to the use of secreted SF06 proteins, and to the use of polynucleotides encoding these, in the diagnosis, and study of diabetes, obesity and/or metabolic syndrome.

Many human proteins serve as pharmaceutical active compounds. Several classes of human proteins that serve as such active compounds include hormones, cytokines, can growth factors, and cell differentiation factors. Most proteins that can be used as a pharmaceutical active compound fall within the family of secreted proteins. Secreted proteins are generally produced within cells at rough endoplasmic reticulum, are then exported to the golgl complex and then move to secretory vesicles or granules, where they are secreted to the exterior of the cell via oxocytosis. Examples for commercially used secreted proteins are human insulin, thrombolytic agents, interferons, interleukins, colony stimulating factors, human growth hormone, transforming growth factor beta, tissue plasminogen activator, erythropoletin, and various other proteins. Receptors of secreted proteins, which are membrane-bound proteins, also have potential as therapeutic or diagnostic agents. It is, therefore, important for developing new pharmaceutical compounds to identify secreted proteins that can be tested for activity in a variety of animal models. Thus, in light of the pervasive role of secreted proteins in human physiology, a need exists for identifying and characterizing novel functions for human secreted proteins and the genes that encode them. This knowledge will allow one to detect, to treat, and to prevent medical diseases, disorders, and/or conditions by using secreted proteins or the gene that encode them.

The pancreas is an essential organ possessing both an exocrine function involved In the delivery of enzymes into the digestive tract and an endocrine function by which various hormones are secreted into the blood stream. The exocrine function is assured by acinar and centroacinar cells that produce various digestive enzymes and intercalated ducts that transport these enzyme in alkaline solution to the duodenum. The functional unit of the endocrine pancreas is the islet of Langerhans. Islets are scattered throughout the exocrine portion of the pancreas and are composed of four cell types: alpha-, beta-, delta- and PP-cells, reviewed for example in Kim S.K. and Hebrok M., (2001) Genes Dev. 15: 111-127. Beta-cells produce insulin, represent the majority of the endocrine cells and form the core of the islets, while alpha-cells secrete glucagon and are located in the periphery. Delta-cells and PP-cells are less numerous and secrete somatostatin and pancreatic polypeptide, respectively.

Early pancreatic development has been well studied in different species, including chicken, zebrafish, and mice (for a detailed review, see Kim & Hebrok, 2001, supra). The pancreas develops from distinct dorsal and ventral anlagen. Pancreas development requires specification of the pancreas anlage along both anterior-posterior and dorsal-ventral axes. A number of factors, which are critical for proper pancreatic development have been identified (see Kim & Hebrok, 2001, supra; Wilson M.E. et al., (2003) Mech Dev. 120: 65-80).

In postnatal/adult humans, the acinar and ductal cells retain a significant proliferative capacity that can ensure cell renewal and growth, whereas the islet cells become mostly mitotically inactive. This is in contrast to rodents where beta-cell replication is an important mechanism in the generation of new beta cells. It has been suggested, that during embryonic development, pancreatic islets of Langerhans originate from differentiating duct cells or other cells with epithelial morphology (Bonner-Weir S. and Sharma A., (2002) J Pathol. 197: 519-526; Gu G. et al., (2003) Mech Dev. 120: 35-43). In adult humans, new beta cells arise in the vicinity of ducts (Butler A.E. et al., (2003) Diabetes 52: 102-110; Bouwens L. and Pipeleers D.G., (1998) Diabetologia 41: 629-633). However, also an intra-islet location or an origin in the bone marrow has been suggested for precursor cells of adult beta cells (Zulewski H. et al., (2001) Diabetes 50: 521-533; lanus A. et al., (2003) J Clin Invest. 111: 843-850). Pancreatic islet growth is dynamic and responds to changes in insulin demand, such as during pregnancy or during the increase in body mass occuring during childhood. In adults, there is a good correlation between body mass and islet mass (Yoon K.H. et al., (2003) J Clin Endocrinol Metab. 88: 2300-2308).

Pancreatic beta-cells secrete insulin, which is stimulated by high blood glucose levels. Insulin amongst other hormones plays a key role in the regulation of the fuel metabolism. Insulin leads to the storage of glycogen and triglycerides and to the synthesis of proteins. The entry of glucose into muscles and adipose cells is stimulated by insulin. In patients who suffer from diabetes mellitus the amount of insulin produced by the pancreatic islet cells is too low, resulting in elevated blood glucose levels (hyperglycemia). In diabetes type I beta cells are lost due to autoimmune destruction. In type 2 diabetic patients, liver and muscle cells loose their ability to respond to normal blood insulin levels (insulin resistance). High blood glucose levels (and also high blood lipid levels) lead to an impairment of beta-cell function and to an increase in beta-cell apoptosis. It is interesting to note that the rate of beta-cell neogenesis does not appear to change in type 11 diabetics (Butler et al., 2003 supra), thus causing a reduction in total beta-cell mass over time. Eventually the application of exogenous insulin becomes necessary in type 2 diabetics.

Improving metabolic parameters such as blood sugar and blood lipid levels (e.g. through dietary changes, exercise, medication or combinations thereof) before beta cell mass has fallen below a critical threshold leads to a relatively rapid restoration of beta cell function. However, after such a treatment the pancreatic endocrine function would remain impaired due to the only slightly increased regeneration rate.

In type I diabetics, the lifespan of pancreatic islets is dramatically shortened due to autoimmune destruction. Treatments have been devised which modulate the immune system and may be able to stop or strongly reduce islet destruction (Raz I. et al., (2001) Lancet 358: 1749-1753; Chatenoud L. et al., (2003) Nat Rev Immunol. 3: 123-132). However, due to the relatively slow regeneration of human beta cells such treatments could only be fully successful at improving the diabetic condition if they are combined with an agent which can stimulate beta cell regeneration.

Thus, both for type I and type II diabetes (early and late stages) there is a need to find novel agents which stimulate beta cell regeneration.

Diabetes is a very disabling disease, because medications do not control blood sugar levels well enough to prevent swinging between high and low blood sugar levels. Patients with diabetes are at risk for major complications, including diabetic ketoacidosis, end-stage renal disease, diabetic retinopathy and amputation. There are also a host of related conditions, such as metabolic syndrome, obesity, hypertension, heart disease, peripheral vascular disease, and infections, for which persons with diabetes are at substantially increased risk. The treatment of these complications contributes to a considerable degree to the enormous cost which is imposed by diabetes on health care systems world wide.

Obesity is one of the most prevalent metabolic disorders in the world. It is still a poorly understood human disease that becomes as a major health problem more and more relevant for western society. Obesity is defined as a body weight more than 20% in excess of the ideal body weight, frequently resulting in a significant impairment of health. Obesity may be measured by body mass index, an indicator of adiposity or fatness. Further parameters for defining obesity are waist circumferences, skinfold thickness and bioimpedance. It is associated with an increased risk for cardiovascular disease, hypertension, diabetes mellitus type II, hyperlipidaemia and an increased mortality rate. Obesity is influenced by genetic, metabolic, biochemical, psychological, and behavioral factors and can be caused by different reasons such as non-insulin dependent diabetes, increase in triglycerides, increase in carbohydrate bound energy and low energy expenditure (Kopelman P.G., (2000) Nature 404: 635-643).

The concept of 'metabolic syndrome' (syndrome x, insulin-resistance syndrome, deadly quartet) was first described 1966 by Camus and reintroduced 1988 by Reaven (Camus J.P., (1966) Rev Rhum Mal Osteoartic 33: 10-14; Reaven G.M., (1988), Diabetes 37: 1595-1607). Today, metabolic syndrome is commonly defined as clustering of cardiovascular risk factors like hypertension, abdominal obesity, high blood levels of triglycerides and fasting glucose as well as low blood levels of HDL cholesterol. Insulin resistance greatly increases the risk of developing the metabolic syndrome (Reaven G., (2002) Circulation 106: 286-288). The metabolic syndrome often precedes the development of type II diabetes and cardiovascular disease (Lakka H.M. et al., (2002) JAMA 288: 2709-2716). The control of blood lipid levels and blood glucose levels is essential for the treatment of the metabolic syndrome (see, for example, Santomauro A.T. et al., (1999) Diabetes, 48: 1836-1841).

The molecular factors regulating food intake and body weight balance are Incompletely understood. Even if several candidate genes have been described which are supposed to influence the homeostatic system(s) that regulate body mass/weight, like leptin or the peroxisome proliferator-activated receptor-gamma co-activator, the distinct molecular mechanisms and/or molecules influencing obesity or body weight/body mass regulations are not known.

There is a need in the prior art for the identification of candidate genes that are specifically expressed in early development in certain pancreatic tissues. These genes and the thereby encoded proteins can provide tools to the diagnosis and treatment of severe pancreatic disorders and related diseases. Therefore, this specification describes secreted proteins that are specifically expressed in pancreatic tissues early in the development. Further described is the use of these genes and proteins in the diagnosis, prevention and/or treatment of pancreatic dysfunctions, such as diabetes, and other related diseases such as obesity and/or metabolic syndrome. These proteins and genes are especially useful in regeneration processes, such as regeneration of the pancreas cells.

In this specification, we describe secreted factory referred to as SF01-SF13, which are involved in pancreas development regeneration, and in the regulation of energy homeostasis. SF01-SF13 corresponds to mammalian proteins as described in Table 1.

The invention relates to the use of a composition comprising a SF06 protein as **characterized by** SEQ ID Nos:30 or 32 and/or a nucleic acid molecule encoding a SF06 protein as **characterized by** SEQ ID Nos:30 or 32, the nucleic acid molecule being preferably a DNA molecule, more preferably a cDNA or a genomic DNA, and optionally pharmaceutically acceptable carriers, diluents, and/or additives for the preparation of an agent for detecting diseases or dysfunctions selected from the group consisting of diabetes, obesity, and/or metabolic syndrome. Further, the invention relates to the identification of a (poly)peptide involved in the regulation of energy homeostasis and/or metabolism of a mammal and to the screening of an agent, which modulates/effects the activity of a SF06 polypeptide. Any information about other SF01-05 and SF07-13 proteins is provided for the purpose of comparison only.

The function of SF01, a TNF-related molecule, was previously unknown. Homologues of SF01 exist In human, mouse and Danio rerio. Human SF01 is expressed in brain, hippocampus, and islets of Langerhans. In fish, SF01 is expressed in the brain.

SF02 is conserved from Drosophila to human. SF02 is a developmentally regulated vital protein. Drosophila SF02 mutants are dying from neural system defects. In mouse, SF02 seems to be enriched in endoderm and embryo, and to be present in pancreas. In humans, SF02 Is present in pancreas, liver, thymus, and spleen.

Glypicans are a family of glycosylphosphatidylinositol (GPI)-anchored cell surface heparan sulfate proteoglycans (HSPGs) SF03 is a member of the glypican family of heparan sulfate proteoglycans, which attaches to the cell membrane via a GPI anchor. SF03 is mutated in the Simpson-Golabi-Behmel syndrome (SGBS). SGBS is characterized by pre- and post-natal overgrowth and is a recessive X-linked condition. SF03 is expressed in embryonic mesodermal lung, liver and kidney tissues and is thought to interact with various growth factors to regulate tissue and organ growth.

The SF04 gene is a shared precursor for alpha-microglobulin and bikunin. Alpha microglobulin is a lipocalin with immunosuppressive properties, bikunin is a plasma proteinase inhibitor. The SF04 mRNA is strongly transcribed in liver parenchyma, pancreas, and intestine epithelium. The both encoded proteins are accordingly present in developing hepatocytes, pancreas, kidney, and gut. Bikunin functions as tumor suppressor.

SF05 is a neural-specific serine protease inhibitor, which is expressed in the whole developing CNS in mouse. SF05 inhibits the extracellular protease tissue-type plasminogen activator and plasmin, but not thrombin. SF05 deficient mice are were viable and healthy, except behavioral defects. Mutant SF05 protein aggregates and causes familial dementia in humans.

SF06 is secreted by brain, adrenal cortex and adrenocortical tumors. SF06 is involved in the regulation of steroid hormone secretion and the proliferation of adrenocortical cells as autocrine and/or paracrine factor.

SF07 is a putative tumor suppressor gene, which is inactivated in hepatocarcinomas, colorectal cancer and non-small cell lung cancers.

SF08 is a carboxypeptidase which has no known enzymatic activity. SF08 is expressed in developing bones and cartilage.

The SF09 protein is 145 aa long and contains calcium ion binding EF-hand motifs. According to expressed sequence tag (EST) assembly, SF09 is expressed in many tissues including the pancreas.

The mouse homologue of human SF10 is an extracellular integrin-binding matrix protein. Mutations of SF10 are frequent in patients with Smith-Magenis syndrome (SMS), a clinically recognizable multiple congenital anomaly/mental retardation syndrome.

SF11 is a cysteine-proteinase inhibitor for cathepsins B and L, which is well characterised. The expression of SF11 is controlled by TGF-beta and EGF in decidual cultures and by TGF-beta in astrocyte precursors. A glycosylated form of SF11 is required for a FGF-2-responsive neural stem cell proliferation. Combined delivery of FGF-2 and SF11 to the adult dentate gyrus stimulated neurogenesis. SF11 deficient mice showed reduced tumor growth.

SF12 Is an extracellular matrix and plasma glycoprotein. Expression of SF12 in the adult pancreatic islet is mostly confined to the blood vessels.

SF13 is a ubiquitously expressed cytosolic peptidyl-prolyl cis-trans Isomerase that is inhibited by the immunosuppressive drug cyclosporin. SF13 is a proinflammatory factor for T-lymphocytes. SF13 signals through CD147 (basigin) preceptor, and is expressed in acinar but not in islet membranes or MIN-6 cells.

Accordingly, the present specification describes secreted proteins with functions in the human metabolism, regeneration, and pancreatic developmental processes. Described are specific genes and proteins encoded thereby and offectors/modulators thereof involved in the regulation of pancreatic function and metabolism, especially in pancreas diseases such as diabetes mellitus, e.g, insulin dependent diabetes mellitus and/or non insulin dependent diabetes mellitus, and/or metabolic syndrome, obesity, and/or related disorders such as coronary heart disease, eating disorder, cachexia, hypertension, hypercholesterolemia (dyslipidemia), liver fibrosis, and/or galistones. Further, described are specific genes and proteins encoded thereby and effectors/modulators thereof involved in the regeneration of pancreatic cells or tissues, e.g. cells having exocrinous functions such as acinar cells, centroacinar cells and/or ductal cells and/or cells having endocrinous functions, particularly cells in Langerhans islets such as alpha-, beta-, delta- and/or PP-cells, more particularly beta-cells.

In this specification, we used a screen for secreted factors expressed in developing mammalian (mouse) pancreas, as described in more detail in the Examples section (see Example 1). This screen identified SF01-SF13 as secreted factors expressed in developing mouse pancreas. Mammalian SF01-SF13 proteins and the polynucleotides encoding these, in particular human SF01-SF13, are involved in the conditions and processes mentioned above.

SF01-SP13 homologous proteins and nucleic acid molecules coding therefore are obtainable from vertebrate species. For use in the present invention, particularly preferred are nucleic acids encoding the human SF06 protein as characterized by SEQ ID NOs: 30 or 32 is as defined in claim 4.

The function of the mammalian SF01-SF13 in mammalian metabolism was validated by analysing the expression of the transcripts in different tissues and by analizing the role in adipocyte differentiation (see Examples 3 and 4 for more detail).

Expression profiling studies (see Examples for more detail) confirm the particular relevance of SF02-SF04 as regulators of energy metabolism in mammals.

Microarrays are analytical tools routinely used in bioanalysis. A microarray has molecules distributed over, and stably associated with, the surface of a Solid support. The term "microarray" refers to an arrangement of a plurality of polynucleotides, polypeptides, antibodies, or other chemical compounds on a substrate. Microarrays of polypeptides, polynucleotides, and/or antibodies have been developed and find use in a variety of applications, such as monitoring gene expression, drug discovery, gene sequencing, gene mapping, bacterial identification, and combinatorial chemistry. One area in particular in which microarrays find use is in gene expression analysis (see Example 4). Array technology can be used to explore the expression of a single polymorphic gene or the expression profile of a large number of related or unrelated genes. When the expression of a single gene is examined, arrays are employed to detect the expression of a specific gene or its variants. When an expression profile is examined, arrays provide a platform for identifying genes that are tissue specific, are affected by a substance being tested in a toxicology assay, are part of a signaling cascade, carry out housekeeping functions, or are specifically related to a particular genetic predisposition, condition, disease, or disorder.

Microarrays may be prepared, used, and analyzed using methods known in the art (see for example, Brennan T.M., (1995) U.S. Patent No. 5,474,796; Schena M. et al., (1996) Proc. Natl. Acad. Sci. USA 93:10614-10619; Baldeschwieler J.D. et al., (1995) PCT application WO 95/251116; Shalon T.D. and Brown P.O., (1995) PCT application WO 95/35505; Heller R.A. et al., (1997) Proc. Natl. Acad. Sci. USA 94: 2150-2155; Heller, M.J. and Tu E., (1997) U.S. Patent No. 5,605,662). Various types of microarrays are well known and thoroughly described in Schena M., ed. (1999; DNA Microarrays: A Practical Approach, Oxford University Press, London).

Oligonucleotides or longer fragments derived from any of the polynucleotides described herein may be used as elements on a microarray. The microarray can be used in transcript imaging techniques, which monitor the relative expression levels of large numbers of genes simultaneously as described below. The microarray may also be used to identify genetic variants, mutations, and polymorphisms. This information may be used to determine gene function, to understand the genetic basis of a disorder, to diagnose a disorder, to monitor progression/regression of disease as a function of gene expression, and to develop and monitor the activities of therapeutic agents in the treatment of disease. In particular, this information may be used to develop a pharmacogenomic profile of a patient in order to select the most appropriate and effective treatment regimen for that patient. For example, therapeutic agents, which are highly effective and display the fewest side effects may be selected for a patient based on his/her pharmacogenomic profile.

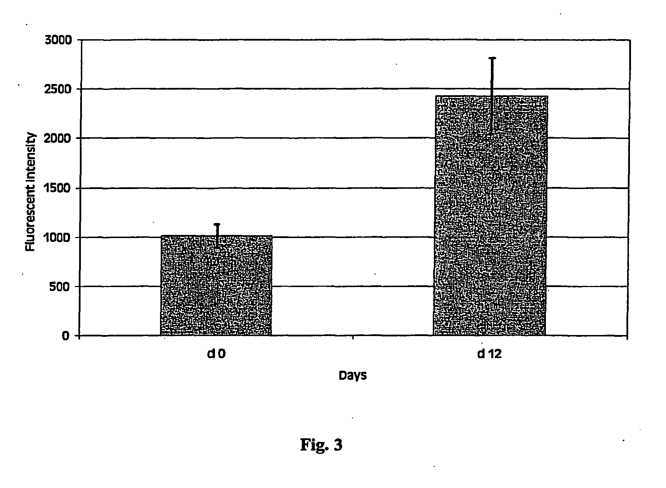

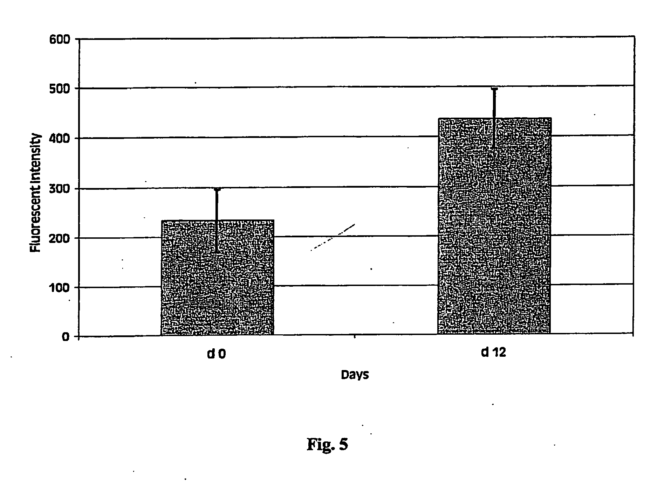

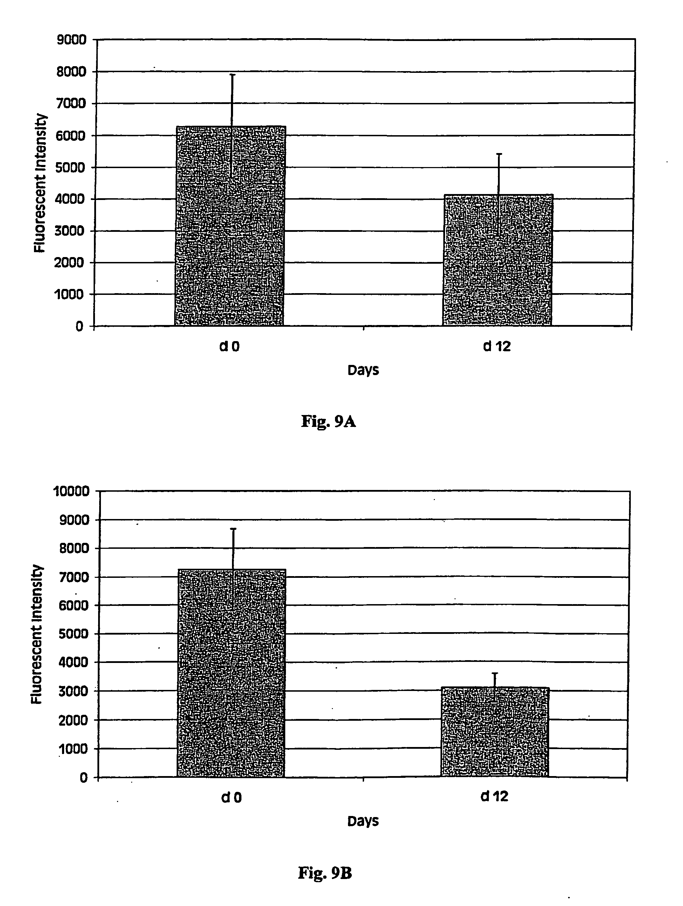

As determined by microarray analysis, SF02, SF03, and SF13 show differential expression in human primary adipocytes. A strong up-regulation is observed concerning the expression of SF02 and SF03 during the human adipocyte differentiation (see Fig. 3 and 5) and a strong down-regulation is observed concerning the expression of SF13 during the human adipocyte differentiation (see Fig. 9). The SF02 and SF03 proteins in preadipocyctes have the potential to enhance adipocyte differentiation, and the SF13 protein in preadipocyctes has the potential to enhance adipocyte differentiation at a very early stage. Therefore, the SF02, SF03, and SF13 proteins might play an essential role in adipogenesis. The results are suggesting a role of SF02, SF03, and SF13 in the regulation in human metabolism, for example, as effectors/modulators (for example, enhancers or inhibitors) of adipogenesis. Thus, SF02, SF03, and SF13 are strong candidates for the manufacture of pharmaceutical compositions and medicaments for the treatment of conditions related to human metabolism, such as diabetes, obesity, and/or metabolic syndrome.

Further, we show whole mount and sectional in situ hybridisations (see Examples and Fig. 1, 7, and 8). The nucleic acid sequence encoding the mouse SF01 protein Is expressed in the region of the ventral pancreas. The nucleic acid sequence encoding the mouse SF05 and SF06 proteins are expressed in the pancreas tissue (see Fig. 7 and 8).

Hybridization conditions are based on the melting temperature (Tm) of the nucleic acid binding complex or probe, as described in Wahl G.M. et al., (1987; Methods Enzymol. 152: 399-407) and Kimmel A.R. (1987; Methods Enzymol. 162: 607-611), and may be used at a defined stringency. Preferably, hybridization under stringent conditions means that after washing for 1 h with 1 x SSC and 0.1 % SDS at 50°C, preferably at 55°C, more preferably at 62°C and most preferably at 65°C, particularly for 1 h in 0.2 x SSC and 0.1% SDS at 50°C, preferably at 55°C, more preferably at 62°C and most preferably at 65°C, a positive hybridization signal is observed.

In order to express a biologically active protein, the nucleotide sequences encoding the proteins or functional equivalents, may be inserted into appropriate Expression vectors, i.e., a vector which contains the necessary elements for the transcription and translation of the inserted coding sequences. Methods, which are well known to those skilled in the art, may be used to construct expression vectors containing sequences encoding the proteins and the appropriate transcriptional and translational control elements. Regulatory elements include for Example a promoter, an initiation colon, a stop codon, a mRNA stability regulatory element, and a polyadenylation signal. Expression of a polynucleotide can be assured by (i) constitutive promoters such as the Cytomegalovirus (CMV) promoter/enhancer region, (ii) tissue specific promoters such as the insulin promoter (see, Soria B. et al., (2000), Diabetes 49: 157-162), SOX2 gene promoter (see U M. et al., (1998) Curr. Biol. 8: 971-974). Msi-1 promoter (see Sakakibara S. and Okano H., (1997) J. Neuroscience 17: 8300-8312), alpha-cardia myosin heavy chain promoter or human atrial natriuretic factor promoter (Klug M.G. et al., (1998) J. Clin. Invest 98: 216-224: Wu J. et al., (1989) J. Biol. Chem. 264: 8472-8479) or (III) inducible promoters such as the tetracycline inducible system. Expression vectors can also contain a selection agent or marker gene that confers antibiotic resistance such as the neomycin, hygromycin or puromycin resistance genes. These methods include in vitro recombinant DNA techniques, synthetic techniques, and in vivo genetic recombination. Such techniques are described in Sambrook J. et al., (1989) Molecular Cloning, A Laboratory Manual, Cold Spring Harbor Press, Plainview, N.Y. and Ausubel F.M. et al., (1989) Current Protocols in Molecular Biology, John Wiley 8 Sons, New York, N.Y.

In a further embodiment of the invention, natural, iodized or recombinant nucleic acid sequences encoding the SF06 proteins may be ligated to a heterologous sequence to encode a fusion protein.

A variety of expression vector/host systems, as known in the art, may be utilized to contain and express sequences encoding the proteins or fusion proteins. These include, but are not limited to, micro-organisms such as bacteria transformed with recombinant bacteriophage, plasmid or cosmic DNA expression vectors; yeast transformed with yeast expression vectors; insect cell systems infected with virus expression vectors (e.g., baculovirus, adenovirus, adeno-associated virus, lentiverus, retrovirus); plant cell systems transformed with virus expression vectors (e.g., cauliflower mosaic virus, CaMV; tobacco mosaic virus. TMV) or with bacterial expression vectors (e.g., Ti or PBR322 plasmids); or animal cell systems.

The presence of polynucleotide sequences in a sample can be detected by DNA-DNA or DNA-RNA hybridization and/or amplification using probes or portions or fragments of said polynucleotides. Nucleic add amplification based assays involve the use of oligonucleotides or oligomers based on the sequences specific for the gene to detect transformants containing DNA or DNA encoding the corresponding protein. As used herein 'oligonucleotides' or 'oligomers' refer to a nucleic acid sequence of at least about 10 nucleotides and as many as about 60 nucleotides, preferably about 15 to 30 nucleotides, and more preferably about 20-25 nucleotides, which can be used as a probe or amplimer.

A wide variety of labels and conjugation techniques are known by those skilled in the art and may be used in various nucleic add and amino acid assays. Means for producing labeled hybridization or PCR probes for detecting polynucleotide sequences include oligo-labeling, nick translation, end-labeling of RNA probes, PCR amplification using a labeled nucleotide, or enzymatic synthesis. These procedures may be conducted using a variety of commercially available kits (Pharmacia & Upjohn, (Kalamazoo, Mich.); Promega (Madison Wis.); and U.S. Biochemical Corp., (Cleveland, Ohio).

The presence of SF01-SF13 in a sample can be determined by immunological methods or activity measurement. A variety of protocols for detecting and measuring the expression of proteins, using either polyclonal or monoclonal antibodies specific for the protein or reagents for determining protein activity are known in the art. Examples include enzyme-linked immunosorbent assay (ELISA), radioimmunoassay (RIA), and fluoresoence activated cell sorting (FACS). A two-site, monoclonal-based immunoassay utilizing monoclonal antibodies reactive to two non-interfering epitopes on the protein is preferred, but a competitive binding assay may be employed. These and other assays are described, among other places, In Hampton R. at al. (1990; Serological Methods, a Laboratory Manual, APS Press, St Paul, Minn.) and Maddox D.E. et al. (1983; J. Exp. Med. 158: 1211-1226).

Suitable reporter molecules or labels, which may be used, include radionuclides, enzymes, fluorescent, chemiluminescent or chromogenic agents as well as substrates, co-factors, inhibitors, magnetic particles, and the like. The nucleic acids encoding the SF06 proteins can be used to generate non-human transgenic animal or site specific gene modifications in cell lines non-human Transgenic animals may be made through homologous combination, where the normal locus of the genes encoding the proteins of the invention is altered. Alternatively, a nucleic acid construct is randomly integrated into the genome. Vectors for stable integration include plasmids, retroviruses and other animal viruses, YACs, and the like. The modified cells or animal are useful in the study of the function and regulation of the SF06 proteins. For example, a series of small deletions and/or substitutions may be made in the genes that encode the proteins to determine the role of particular domains of the protein, functions in pancreatic differentiation, etc.

Specific constructs of interest include anti-sense molecules, which will block the expression of the SF06 proteins, or expression of dominant negative mutations. A detectable marker, such as for example lac-Z, may be introduced in the locus of the genes, where up-regulation of expression of the genes will result In an easily detected change In phenotype.

One may also provide for expression of the genes in cells or tissues where it is not normally expressed or at abnormal times of development. In addition, by providing expression of the SF06 proteins in cells in which they are not normally produced, one can induce changes in cell behavior.

DNA constructs for homologous recombination will comprise at least portions of the genes with the desired genetic modification, and will include regions of homology to the target locus. DNA constructs for random integration need not include regions of homology to mediate recombination. Conveniently, markers for positive and/or negative selection are includes. Methods for generating cells having targeted gene modifications through homologous recombination are known in the art. For non-human embryonic stem (ES) cells, an ES cell line may be employed, or embryonic cells may be obtained freshly from a host, e.g. mouse, rat, guinea pig etc. Such cells are grown on an appropriate fibroblast-feeder layer or grown in presence of leukemia inhibiting factor (LIF).

The data described in this specification show that the SF06 nucleic acids and proteins are useful in diagnostic applications concerning diabetes, (such as insulin dependent diabetes mellitus and/or non insulin dependent diabetes mellitus), obesity, and/or metabolic syndrome. Hence, diagnostic uses for the nucleic acids and proteins are, for example but not limited to, the following: (i) antibody target (diagnostic), (ii) diagnostic and/or prognostic marker, and / or (iii) research tools.

The SF06 nucleic acids and proteins are useful in diagnostic applications implicated in various embodiments as described below.

The nucleic acids may further be useful in diagnostic applications, wherein the presence or amount of the nucleic acids or the proteins are to be assessed, selected diabetes, obesity and metabolic syndrome.

A variety of protocols including ELISA, RIA, and FACS for measuring proteins are known in the art and provide a basis for diagnosing altered or abnormal levels of gene expression. Normal or standard values for gene expression are established by combining body fluids or cell extracts taken from normal mammalian subjects, preferably human, with antibodies to the protein under conditions suitable for complex formation. The amount of standard complex formation may be quantified by various methods, but preferably by photometric means. Quantities of protein expressed in control and diseases, samples e.g.. from biopsied tissues are compared with the standard values. Deviation between standard and subject values establishes the parameters for diagnosing disease.

In another embodiment of the invention, the polynucleotides specific for the SF06 proteins may be used for diagnostic purposes, The polynucleotides, which may be used, include oligonucleotide sequences, antisense . RNA and DNA molecules, and PNAs. The polynucleotides may be used to detect and quantitate gene expression in biopsied tissues in which gene expression may be correlated with disease. The diagnostic assay may be used to distinguish between absence, presence, and excess gene expression, and to monitor regulation of protein levels during therapeutic intervention.

In one aspect, hybridization with probes which are capable of detecting polynucleotide sequences, including genomic sequences, encoding the SF06 proteins may be used to identify nucleic acid sequences which encode the respective protein. The hybridization probes may be DNA or RNA and are preferably derived from the nucleotide sequence of the polynucleotide encoding the SF06 proteins or from a genomic sequence including promoter, enhancer elements, and introns of the naturally occurring gene. Hybridization probes may be labeled by a variety of reporter groups, for example, radionuclides such as ³²P or ³⁵S or enzymatic labels, such as alkaline phosphatase coupled to the probe via avidin/biotin coupling systems, and the like.

Polynucleotide sequences specific for SF06 proteins may be used for the diagnosis of conditions or diseases, which are associated with the expression of the proteins, selected from diabetes, obesity and metabolic syndrome. Polynucleotide sequences specific for the SF06 proteins may also be used to monitor the progress of patients receiving treatment for diabetes, obesity, and/or metabotic syndrome. The polynucleotide sequences may be used qualitative or quantitative assays, e.g. in Southern or Northern analysis, dot blot or other membrane-based technologies: in PCR technologies; or in dip stick, pin, ELISA or chip assays utilizing fluids or tissues from patient biopsies to detect altered gene expression.

In a particular aspect, the SF06 nucleotide sequences may be useful in assays that detect activation or induction of the above metabolic diseases or dysfunctions. The nucleotide sequences may be labeled by standard methods, and added to a fluid or tissue sample from a patient under conditions suitable for the formation of hybridization complexes. After a suitable incubation period, the sample is washed and the signal is quantitated and compared with a standard value. The presence of altered levels of nucleotide sequences encoding the SF06 proteins in the sample indicates the presence of the associated disease. Such assays may also be used to evaluate the efficacy of a particular therapeutic treatment regimen in animal studies, in clinical trials or in monitoring the treatment of an individual patient.

In order to provide a basis for the diagnosis of a disease associated with expression of the SF06 proteins, a normal or standard profile for expression is established. This may be accomplished by combining body fluids or cell extracts taken from normal subjects, either animal or human, with a sequence or a fragment thereof, which is specific for the nucleic acids encoding the SF06 proteins, under conditions suitable for hybridization or amplification. Standard hybridization may be quantified by comparing the values obtained from normal subjects with those from an experiments where a known amount of a substantially purified polynucleotide is used. Standard values obtained from normal samples may be compared with values obtained from samples from patients who are symptomatic for disease. Deviation between standard and subject values is used to establish the presence of disease. Once disease is established and a treatment protocol is initiate, hybridization assays may be repeated on a regular basis to evaluate whether the level of expression in the patient begins to approximate that, which is observed in the normal patient. The results obtained from successive assays may be used to show the efficacy of treatment over a period ranging from several days to months.

With respect to diabetes, obesity, and/or metabolic syndrome, the presence of an unusual amount of transcript in biopsled tissue from an individual may indicate a predisposition for the development of the disease or may provide a means for detecting the disease prior to the appearance of actual clinical symptoms. A more definitive diagnosis of this type may allow health professionals to employ preventative measures or aggressive treatment earlier thereby preventing the development or further progression of the metabolic diseases and disorders.

Additional diagnostic uses for oligonucleotides designed from the sequences encoding the SF06 proteins may involve the use of PCR. Such oligomers may be chemically synthesized, generated enzymatically or produced from a recombinant source. Oligomers will preferably consist of two nucleotide sequences, one with sense orientation (5prime.fwdarw.3prime) and another with antisense (3prime.rarw.5prime), employed under optimized conditions for identification of a specific gene or condition. The same two oligomers, nested sets of oligomers or even a degenerate pool of oligomers may be employed under less stringent conditions for detection and/or quantification of closely related DNA or RNA sequences.

The nucleic acid sequences may also be used to generate hybridization probes, which are useful for mapping the naturally occurring genomic sequence. The sequences may be mapped to a particular chromosome or to a specific region of the chromosome using well known techniques. Such techniques include FISH, FACS or artificial chromosome constructions, such as yeast artificial chromosomes, bacterial artificial chromosomes, bacterial P1 constructions or single chromosome cDNA libraries as reviewed in Price C.M., (1993) Blood Rev..7: 127-134, and Trask B.J., (1991) Trends Genet. 7: 149-154. FISH (as described in Verma RS. and Babu A., (1989) Human Chromosomes: A Manual of Basic Techniques, Pergamon Press, New York, N.Y.). The results may be correlated with other physical chromosome mapping techniques and genetic map data. Examples of genetic map data can be found in the 1994 Genome Issue of Science (265: 1981f). Correlation between the location of the gene encoding the SF06 proteins on a physical chromosomal map and a specific disease or predisposition to a specific disease, may help to delimit the region of DNA associated with that genetic disease.

The nucleotide sequences may be used to detect differences in gene sequences between normal, carrier or affected individuals. An analysis of polymorphisms, e.g. single nucleotide polymorphism may be carried out Further, in situ hybridization of chromosomal preparations and physical mapping techniques such as linkage analysis using established chromosomal markers may be used for extending genetic maps. Often the placement of a gene on the chromosome of another mammalian species, such as mouse, may reveal associated markers even if the number or arm of a particular human chromosome is not known. New sequences can be assigned to chromosomal arms or parts thereof, by physical mapping. This provides valuable information to investigators searching for disease genes using positional cloning or other gene discovery techniques. Once the disease or syndrome has been crudely localized by genetic linkage to a particular genomic region, for example, AT to 11q22-23 (Gatti R.A. et al., (1988) Nature 336: 577-580), any sequences mapping to that area may represent associated or regulatory genes for further investigation. The nucleotide sequences may also be used to detect differences in the chromosomal location due to translocation, inversion, etc. among normal, carrier or affected individuals.

In another embodiment of the invention, the SF06 proteins can be used for screening libraries of compounds in any of a variety of drug screening techniques. One can identify effectors, e.g. receptors, enzymes, proteins, ligands, or substrates that bind to modulate or mimic the action of one or more of the SF06 proteins. The protein or fragment thereof employed In such screening may be free in solution, affixed to a solid support, borne on a cell surface, or located intracellulary. The formation of binding complexes, between the SF06 proteins and the agent tested, may be measured. Agents could also, either directly or indirectly, influence the activity of the SF06 proteins.

In addition activity of the SF06 proteins against their physiological substrate(s) or derivatives thereof could be measured in cell-based or cell-free assays. Agents may also interfere with posttranslational modifications of the protein, such as phosphorylation and dephosphorylation, farnesylation, palmitoylation, acetylation, alkylation, ubiquitination, proteolytic processing, subcellular localization and degradation. Moreover, agents could influence the dimerization or oligomerization of the SF06 proteins or, in a heterologous manner, of the SF06 proteins with other proteins, for example, but not exclusively, docking proteins, enzymes, receptors, or translation factors. Agents could also act on the physical interaction of the SF06 proteins with other proteins, which are required for protein function, for example, but not exclusively, their downstream signaling.

Methods for determining protein-protein interaction are well known In the art. For example binding of a fluorescently labeled peptide derived from the interacting proteins to the SF06 protein, or vice versa, could be detected by a change in polarisation. In case that both binding partners, which can be either the full length proteins as well as one binding partner as the full length protein and the other just represented as a peptide are fluorescently labeled, binding could be detected by fluorescence energy transfer (FRET) from one fluorophore to the other. In addition, a variety of commercially available assay principles suitable for detection of protein-protein Interaction are well known in the art, for example but not exclusively AlphaScreen (PeridnElmer) or Scintillation Proximity Assays (SPA) by Amersham. Alternatively, the interaction of the SF06 proteins with cellular proteins could be the basis for a cell-based screening assay, in which both proteins are fluorescently labeled and interaction of both proteins is detected by analysing cotranslocation of both proteins with a cellular imaging reader, as has been developed for example, but not exclusively, by Cellomics or EvotecOAI. In all cases the two or more binding partners can be different proteins with one being the SF06 protein, or in case of dimerization and/or oligomerization the SF06 protein itself.

Of particular interest are screening assays for agents that have a low toxicity for mammalian cells. The term "agent" as used herein describes any molecule, e.g. protein or pharmaceutical, with the capability of altering or mimicking the physiological function of one or more of the SF06 proteins. Candidate agents encompass numerous chemical classes, though typically they are organic molecules, preferably small organic compounds having a molecular weight of more than 50 and less than about 2,500 Daltons. Candidate agents comprise functional groups necessary for structural Interaction with proteins, particularly hydrogen bonding, and typically include at least an amine, carbonyl, hydroxyl or carboxyl group, preferably at least two of the functional chemical groups. The candidate agents often comprise carbocyclic or heterocyclic structures and/or aromatic or polyaromatic structures substituted with one or more of the above functional groups.

Candidate agents are also found among biomolecules including peptides, saccharides, fatty acids, steroids, purines, pyrimidines, nucleic acids and derivatives, structural analogs or combinations thereof. Candidate agents are obtained from a wide variety of sources including libraries of synthetic or natural compounds. For example, numerous means are available for random and directed synthesis of a wide variety of organic compounds and biomolecules, including expression of randomized oligonucleotides and oligopeptides. Alternatively, libraries of natural compounds in the form of bacterial, fungal, plant and animal extracts are available or readily produced. Additionally, natural or synthetically produced libraries and compounds are readily modified through conventional chemical, physical and biochemical means, and may be used to produce combinatorial libraries. Known pharmacological agents may be subjected to directed or random chemical modifications, such as acylation, alkylation, esterification, amidification, etc. to produce structural analogs. Where the screening assay is a binding assay, one or more of the molecules may be joined to a label, where the label can directly or indirectly provide a detectable signal.

Another technique for drug screening, which may be used, provides for high throughput screening of compounds having suitable binding affinity to the protein of interest as described in published PCT application WO84/03564. In this method, as applied to the SF06 proteins large numbers of different small test compounds, e.g. aptamers, peptides, low-molecular weight compounds etc., are provided or synthesized on a solid substrate, such as plastic pins or some other surface. The test compounds are reacted with the proteins or fragments thereof, and washed. Bound proteins are then detected by methods well known in the art. Purified proteins can also be coated directly onto plates for use in the aforementioned drug screening techniques. Alternatively, non-neutralizing antibodies can be used to capture the peptide and immobilize it on a solid support. One may use competitive drug screening assays in which neutralizing antibodies capable of binding the protein specifically compete with a test compound for binding the protein. In this manner, the antibodies can be used to detect the presence of any peptide, which shares one or more antigenic determinants with the protein.

Compounds that bind SF06 proteins, e.g. antibodies, are useful for the identification or enrichment of cells, which are positive for the expression of the SF06 proteins, from complex cell mixtures. Such cell populations are useful in transplantation, for experimental evaluation, and as source of lineage and cell specific products, including mRNA species useful in identifying genes specifically expressed in these cells, and as target for the identification of factors of molecules that can affect them. Cells expressing the SF06 protein or which have been treated with the SF06 protein are useful in transplantation to provide a recipient with pancreatic islet cells, including insulin producing beta cells; for drug screening; experimental models of islet differentiation and interaction with other cell types; in vitro screening assays to define growth and differentiation factors, and to additionally characterize genes involved In islet development and regulation; and the like. The native cells may be used for these purposes, or they may be genetically modified to provide altered capabilities. Cells from a regenerating pancreas, from embryonic foregut, stomach and duodenum, or other sources of pancreatic progenitor cells may be used as a starting population. The progenitor cells may be obtained from any mammalian species, e.g. equine, bovine, porcine, canine, feline, rodent, e.g. mice, rats, hamster, primate, etc. particularly human.

In another embodiment, in a high-throughput screening method, the cells are transfected with a DNA construct, e.g. a viral or non-viral vector containing a reporter gene. e.g. the lacZ gene or the GFP gene, under regulatory control of a promoter of a gene involved in for example beta-cell differentiation, e.g. a promoter of a gene stimulation beta-cell differentiation, preferably a Pax4 promoter. The transfected cells are divided into aliquots and each aliquot is contacted with a test substance, e.g., candidate 1, candidate 2 and candidate 3. The activity of the reporter gene corresponds to the capability of the test compound to induce beta-cell differentiation.

In a further embodiment, which may be combined with the high-throughput screening as described above, a medium throughput validation is carried out. Therein, the test compound is added to stem cells being cultivated and the Insulin production is determined. Following an initial high throughput assay, such as the cell based assay outlined above where for example a Pax4 promoter is used as marker for beta-cell regeneration, the activity of candidate molecules to induce beta-cell differentiation is tested in a validation assay comprising adding said compounds to the culture media of the embryoid bodies. Differentiation into insulin-producing cells is then evaluated, e.g. by comparison to wild type and/or Pax4 expressing ES cells to assess the effectiveness of a compound.

The nucleic acids encoding the SF06 proteins can be used to generate transgenic cell lines and non-human animals. These transgenic non-human animals are useful in the study of the function and regulation of the SF06 proteins in vivo. Non-human transgenic animals, particularly mammalian transgenic animals, can serve as a model system for the investigation of many developmental and cellular processes common to humans. A variety of non-human models of metabolic disorders can be used to test modulators of the SF06. Misexpression (for example, over-expression or lack of expression) of the SF06 protein, particular feeding conditions, and/or administration of biologically active compounds can create models of metabolic disorders.

In one embodiment of the invention, such assays use mouse models of insulin resistance and/or diabetes, such as mice carrying gene knockouts in the leptin pathway (for example, ob (leptin) or db (leptin receptor) mice), as described above. In addition to testing the expression of the SF06 proteins in such mouse strains (see Examples), these mice could be used to test whether administration of a candidate modulator alters for example lipid accumulation In the liver, in plasma, or adipose tissues using standard assays well known in the art, such as FPLC, colorimetric assays, blood glucose level tests, Insulin tolerance tests and others.

Non-human transgenic animals may be made through homologous recombination In embryonic stem cells, where the normal locus of the gene encoding the SF06 protein is mutated. Alternatively, a nucleic add construct encoding the protein is injected into cocytes and is randomly Integrated into the genome. One may also express the genes in tissues where they are not normally expressed or at abnormal times of development. Furthermore, variants of the genes like specific constructs expressing anti-sense molecules or expression of dominant negative mutations, which will block or alter the expression of the SF06, proteins may be randomly integrated into the genome. A detectable marker, such as lac Z or luciferase may be introduced into the locus of the genes, where up-regulation of expression of the genes will result in an easily detectable change in phenotype. Vectors for stable integration include plasmids, retroviruses and other animal viruses, yeast artificial chromosomes (YACs), and the like. DNA constructs for homologous recombination will contain at least portions of the genes with the desired genetic modifications, and will include regions of homology to the target locus. Conveniently, markers for positive and negative selection are included. DNA constructs for random integration do not need to contain regions of homology to mediate recombination. DNA constructs for random integration will consist of the nucleic acids encoding the proteins, a regulatory element (promoter), an intron and a poly-adenylation signal. Methods for generating cells having targeted gene modifications through homologous recombination are known in the field. For embryonic stem (ES) cells, an ES cell line may be employed, or embryonic cells may be obtained freshly from a host, e.g. mouse, rat, guinea pig, etc. Such cells are grown on an appropriate fibroblast-feeder layer and are grown in the presence of leukemia inhibiting factor (LIF). ES or embryonic cells may be transfected and can then be used to produce transgenic animals. After transfection, the ES cells are plated onto a feeder layer in an appropriate medium. Cells containing the construct may be selected by employing a selection medium. After sufficient time for colonies to grow, they are picked and analyzed for the occurrence of homologous recombination. Colonies that are positive may then be used for embryo manipulation and morula aggregation. Briefly, morulae are obtained from 4 to 6 week old superovulated females, the Zona Pellucida is removed and the morulae are put into small depressions of a tissue culture dish. The ES cells are trypsinized, and the modified cells are placed into the depression closely to the morulae. On the following day the aggregates are transfered into the uterine horns of pseudopregnant females. Females are then allowed to go to term. Chimeric offsprings can be readily detected by a change in coat color and are subsequently screened for the transmission of the mutation Into the next generation (F1-generation). Offspring of the F1-generation are screened for the presence of the modified gene and males and females having the modification are mated to produce homozygous progeny. If the gene alterations cause lethality at some point in development, tissues or organs can be maintained as allogenic or congenic grafts or transplants, or In vitro culture. The transgenic animals may be any non-human mammal, such as laboratory animal, domestic animals, etc., for example, mouse, rat, guinea pig, sheep, cow, pig, and others. The transgenic animals may be used in functional studies, drug screening, and other applications and are useful in the study of the function and regulation of the SF06 proteins in vivo.

The Figures show:

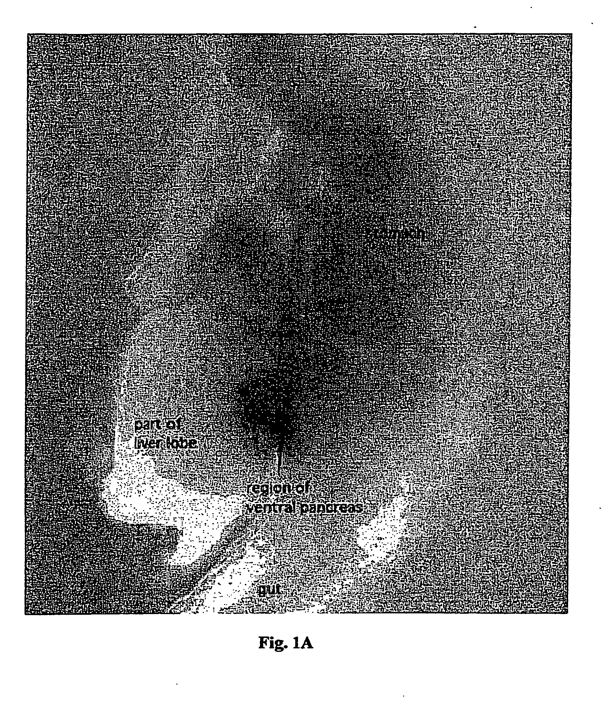

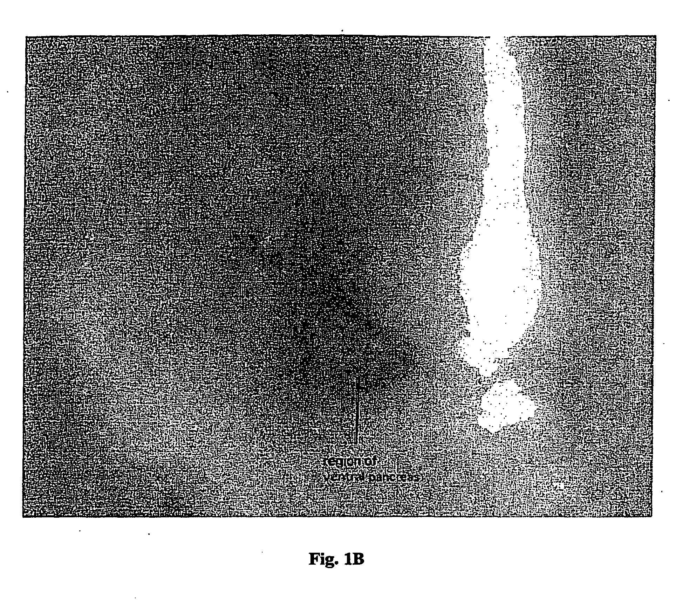

**Fig. 1** shows the in situ hybridization results for the SF01 protein.
   **Fig. 1A** shows a whole mount in situ hybridisation of mouse embryonic pancreas at day E11.5 (lateral view).
   **Fig. 1B** shows a whole mount in situ hybridisation of mouse embryonic pancreas at day E11.6 (ventral view; liver removed; higher magnification of the stained region).

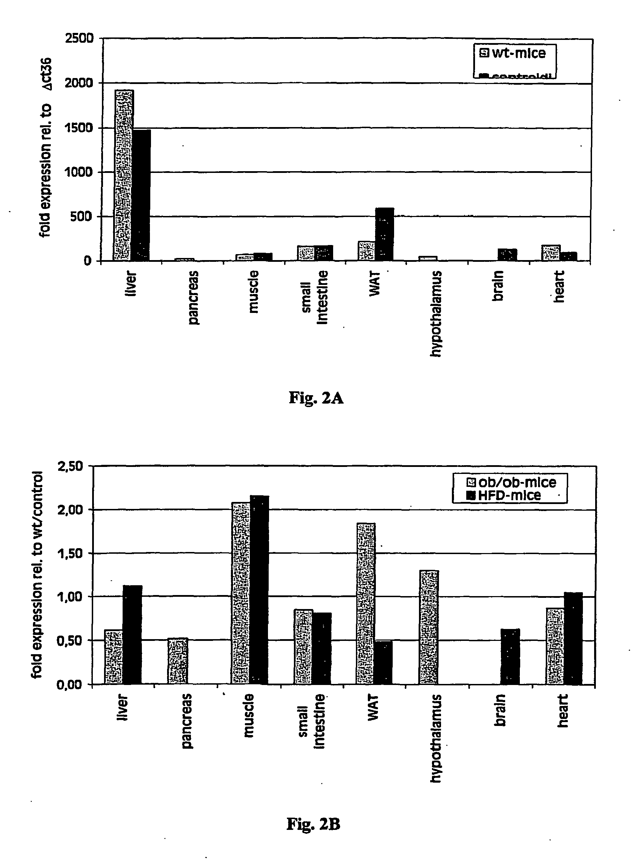

**Fig. 2** shows the expression of SF02 In mammalian (mouse) tissues.
   **Fig. 2A** shows the real-time PCR analysis of SF02 expression in mouse wild type (referred to as wt-mice) and control diet (referred to as controldiet) tissues.
   **Fig. 2B** shows the real-time PCR analysis of SF02 expression in genetically obese mice (referred to as ob/ob-mice) compared to wild type mice and in mice fed with a high fat diet (referred to as HFD-mice) compared to mice fed with a control diet.
**Fig. 3** shows the microarray analysis of SF02 expression in human abdominal adipocyte cells, during the differentiation from preadipocytes to mature adipocytes.

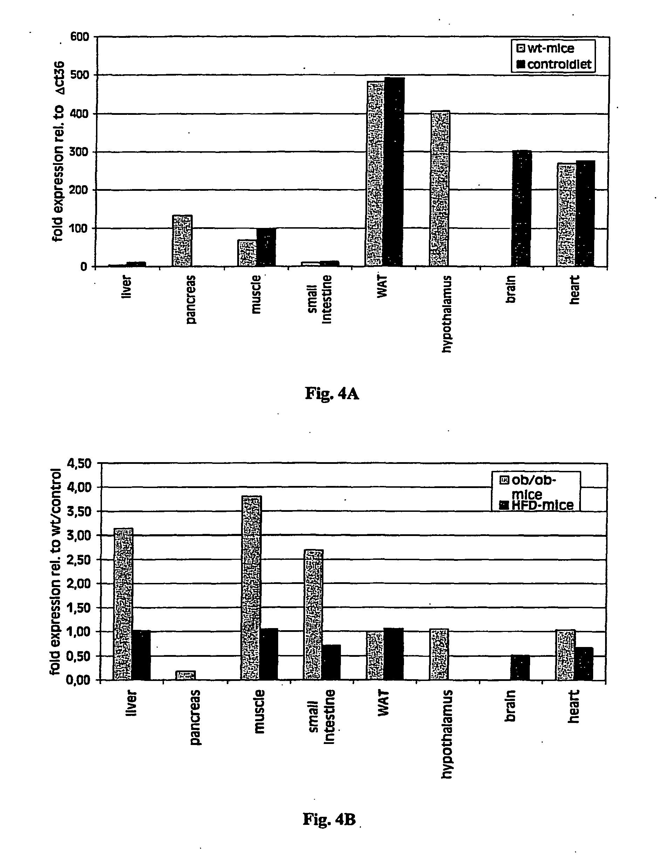

**Fig. 4** shows the expression of SF03 in mammalian (mouse) tissues.
   **Fig. 4A** shows the real-time PCR analysis of SF03 expression in mouse wild type (referred to as wt-mice) and control diet (referred to as controldiet) tissues.
   **Fig. 4B** shows the real-time PCR analysis of SF03 expression in genetically obese mice (referred to as ob/ob-mice) compared to wild type mice and in mice fed with a high fat diet (referred to as HFD-mice) compared to mice fed with a control diet.
**Fig. 5** shows the microarray analysis of SF03 expression in human abdominal adipocyte cells during the differentiation from preadipocytes to mature adipocytes.

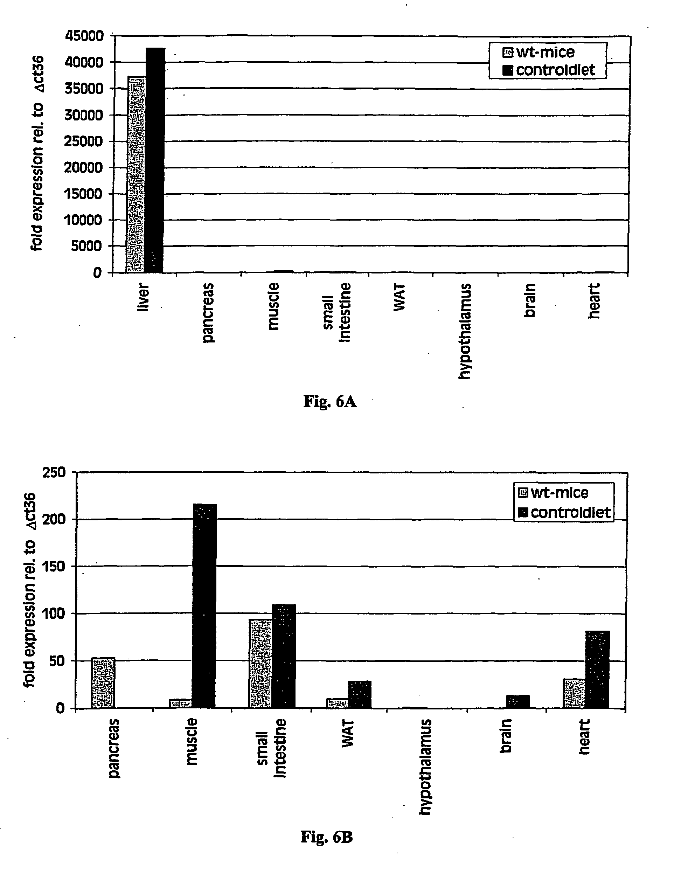

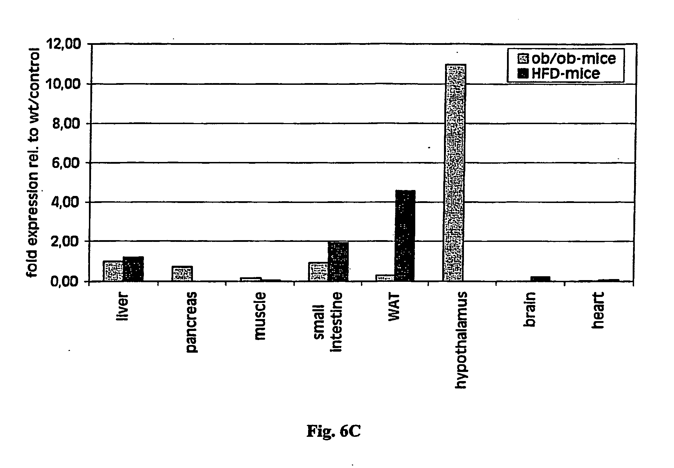

**Fig. 6** shows the expression of SF04 in mammalian (mouse) tissues.
   **Fig. 6A** shows the real-time PCR analysis of SF04 expression in mouse wild type (referred to as wt-mice) and control diet (referred to as controldiet) tissues (including liver).
   **Fig. 6B** shows the real-time PCR analysis of SF04 expression in mouse wild type and control diet tissues (without liver).
   **Fig. 6C** shows the real-time PCR analysis of SF04 expression in genetically obese mice (referred to as ob/ob-mice) compared to wild type mice and in mice fed with a high fat diet (referred to as HFD-mice) compared to mice fed with a control diet.

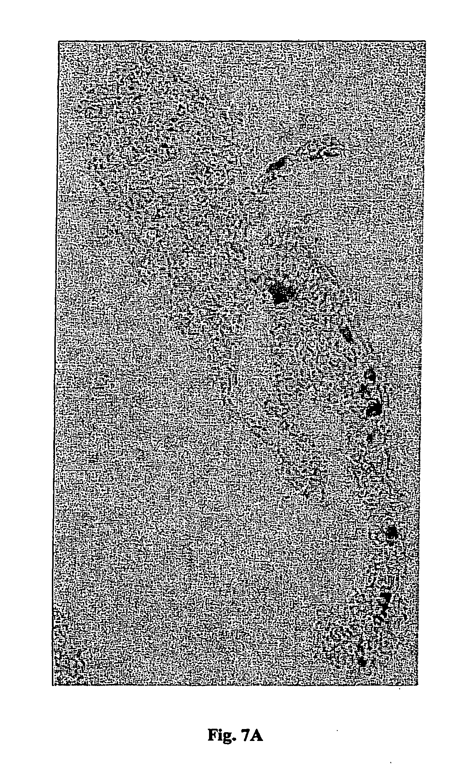

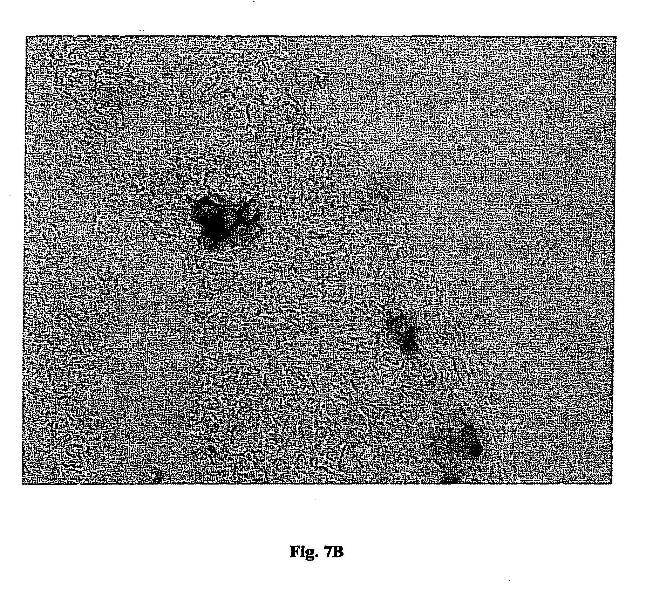

**Fig. 7** shows in situ hybridization results for the SF05 protein.
   **Fig. 7A** shows a cryosection of mouse embryonic pancreas at day E17.5.
   **Fig. 7B** shows the cryosection of mouse embryonic pancreas at day E17.5 in a larger magnification.

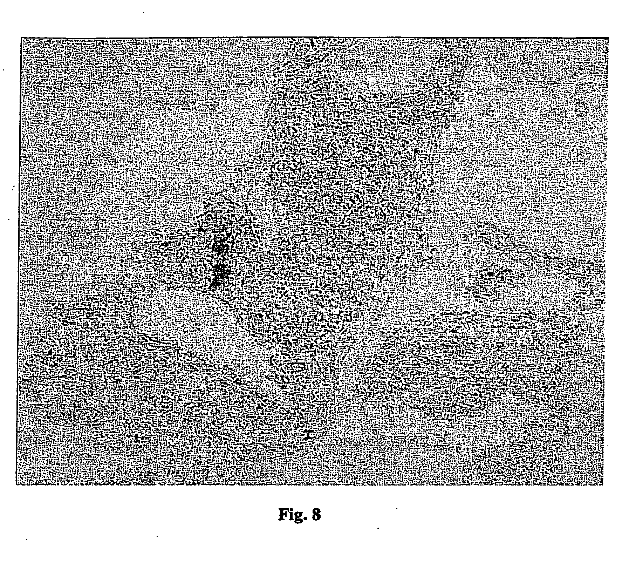

**Fig. 8** shows in situ hybridization results for the SF06 protein. Shown is the cryosection of mouse embryonic pancreas at day E17.5.
**Fig. 9** shows the microarray analysis of SF13 expression in human adipocyte cells during the differentiation from preadipocytes to mature adipocytes.
   **Fig. 9A** shows the microarray analysis of SF13 expression in human primary abdominal adipocyte cells during the differentiation from preadipocytes to mature adipocytes.
   **Fig. 9B** shows the microarray analysis of SF13 expression in human SGBS cells during the differentiation from preadipocytes to mature adipocytes.

### Examples

### Example 1: Identification of secreted factors expressed in pancreas

A screen for secreted factors expressed in developing mouse pancreas was carried out according to methods known by those skilled in the art (see, for example Pera E.M. and De Robertis E.M., (2000) Mech Dev 96(2): 183-195) with several modifications.

### Expression cDNA library:

During organogenesis, the pancreatic bud is surrounded and influenced by the associated mesenchyme. (see for example, Madsen O.D. et al., (1996) Eur. J. Biochem. 242: 435-445 and Slack, J.M., (1995) Development 121: 1569-1580). Recently, it was suggested, that white adipocytes origin directly from mesenchymal cells (Atanossova P.K., (2003) Folia Med. 45: 41-45). During embryogenesis, the innervation and vascularization of the pancreas can be observed. Therefore, the tissue used in the screen might have contained besides pancreatic cells some adipocyte precursors, blood vessels, as well as neuronal cells.

A mouse embryonic stage 9.5-15 pancreatic bud library was prepared in pCMVSPORT-6 vector using SUPERSCRIPT Plasmid System from Invitrogen according to the manufacturer's instructions. The non-amplified library was electroporated into MaxEff DH10B cells (Invitrogen).

### Secretion cloning

Bacterial clones were picked with sterile toothpicks from agar plates and cultured in 96-deep-well microtiter plates in LB-ampicillin (see Sambrook et al., supra). Aliquots of 8 cultures were pooled, and plasmid DNA was isolated using the BioRobot_9600 apparatus according to the manufacturer's instructions (Qiagen; QIAprep(r) Turbo BioRobot Kit. Human 293 cell culture cells were cultured in 75 ml tissue culture flasks in DMEM and 10% fetal calf serum. At 90-99% confluence, the cells were splitted at 1:3 ratio and plated onto poly-D-lysine (Sigma) coated 96-well plates. Cells were transfected with 100-500 ng plasmid using lipofectamine 2000 (Invitrogen). After 6 hours, the medium was exchanged for fresh complete growth medium. 24 hours after transfection, the cells were washed twice with DMEM without cysteine and methionine (Invitrogen), supplemented with 1% dialysed Bovine serum (Sigma) with 50 microgram per ml Heparin (Sigma) and glutamine. The cells were labeled radioactively ('S35 Met-label', from Hartmann Analytic GmbH). After 12 hours, aliquots of the supernatants were harvested in 96-well PCR plates and subjected to SDS gel electrophoresis in precast 4□20% gradient polyacrylamide Criterion gels (Biorad) under reducing conditions, using Criterion Dodeca Cell gel running chamber (Biorad). The gels were fixed in 10% acetic acid, 25% isopropanol for 30 min, soaked 15-30 min in AMPLIFY reagent (Amersham), dried and exposed to X-OMAT (AR) film (Kodak). Positive clones were identified and regrown in 96-well-plates. DNA of individual clones was prepared and used for transfection as described above. If one of the clones yielded proteins of the same size as that of the original pool, a positive clone was identified. Positive clones were partially sequenced from the 5' end (SEQLAB, Goettingen).

### Example 2: Identification of the human homologous nucleic acid and protein sequences

The term "polynucleotide comprising the nucleotide sequence as shown in GenBank Accession number" relates to the expressible gene of the nucleotide sequences deposited under the corresponding GenBank Accession number. The term "GenBank Accession number" relates to NCBI GenBank database entries (Ref.: Benson D.A. et al., (2000) Nucleic Acids Res. 28: 15-18).

Sequences homologous to the mouse sequences were identified using the publicly available program BLASTP 2.2.3 of the non-redundant protein data base of the National Center for Biotechnology information (NCBI) (see, Altschul S.F. et al., (1997) Nucleic Acids Res. 25: 3389-3402).

SF01-SF13 proteins and nucleic acid molecules coding therefore are obtainable from insect or vertebrate species, e.g. mammals or fish. Particularly preferred are nucleic add molecules and proteins encoded thereby comprising human SF01-SF13 and mouse SF01-SF13 sequences identified in the "secreted factor screen", as described in Table 2.

**Table 2: Mammalian genes and proteins of the invention (SF01-SF13)**

| **Name** | **Genbank Accession Numbers** | | | |
|---|---|---|---|---|
| | **Mus musculus genes and proteins** | | **Homo sapiens genes and proteins** | |
| | **cDNA** | **Protein** | **cDNA** | **Protein** |
| SF01 | NM_026161 | NP_080437 | NM_031909 | NP_114115 |
| SF02 | NM_178644 | NP_848759 | NM_178507 | NP_848602 |
| SF03 | NM_016697 | NP_057906 | NM_004484 | NP_004475 |
| SF04 | NM_007443 | NP_031469 | NM_001633 | NP_001624 |
| SF05 | NM_009250 | NP_033276 | NM_005025 | NP_005016 |
| SF06 | NM_172633 | NP_766221 | NM_182511 | NP_872317 |
| SF07 | NM_026840 | NP_081116 | NM_006207 | NP_006198 |
| SF08 | NM_019696 | NP_062670 | NM_019609 | NP_062555 |
| SF09 | NM_139295 | NP_647456 | NM_139279 | NP_644808 |
| SF10 | NM_029568 | NP_083844 | NM_002404 | NP_002395 |
| SF11 | NM_009976 | NP_034106 | NM_000099 | NP_600090 |
| SF12 | NM_010180 | NP_034310 | NM_006486 | NP_006477 |
| SF13 | NM_011149 | NP_035279 | NM_000942 | NP_000933 |

### Example 3: Analysis of the expression of the described nucleic acids in mammalian (mouse) tissues

To analyse the expression of the mRNAs described in this specification in mammalian tissues, several mouse strains (preferably mice strains C57BI/6J, C57BI/6 ob/ob, C57BI/KS db/db, and Non-Obese-Diabetic (NOD) mice, which are standard model systems In obesity and diabetes research) were purchased from Harlan Winkelmann (33178 Borchen, Germany) and Taconic M & B (Germantown, NY 12526, U.S.A), respectively, and maintained under constant temperature (preferably 22°C), 40% humidity and a light / dark cycle of preferably 14 / 10 hours. The mice were fed a standard chow (for example, from ssniff Spezialitäten GmbH, order number ssniff M-Z V1126-000). In a further experiment wild-type (wt) mice were fed a control diet (preferably Altromin C1057 mod control, 4.5% crude fat) or high fat diet (preferably Altromin C1057mod. high fat, 23.5% crude fat). Animals were sacrificed at an age of 6 to 8 weeks. The animal tissues were isolated according to standard procedures known to those skilled in the art, snap frozen in liquid nitrogen and stored at -80°C until needed.

For analyzing the role of the described proteins in the *in vitro* differentiation of mammalian cell culture cells for the conversion of preadipocytes to adipocytes, mammalian fibroblast (3T3-L1) cells (e.g., Green H. and Kehinde O., (1974) Cell 1: 113-116) were obtained from the American Tissue Culture Collection (ATCC, Hanassas, VA, USA; ATCC- CL 173). 3T3-L1 cells were maintained as fibroblasts and differentiated into adipocytes as described in the prior art (e.g., Qiu Z. et al., (2001) J. Biol. Chem. 276: 11988-11995; Slieker L.J. et al., (1998) BBRC 251: 225-229). In brief, cells were plated In DMEM/10% FCS (Invitrogen, Karisruhe, Germany) at 50,000 cells/well in duplicates in 6-well plastic dishes and cultured in a humidified atmosphere of 5% CO₂ at 37°C. At confluence (defined as day 0: d0) cells were transferred to serum-free (SF) medium, containing DMEM/HarnF12 (3:1; invitrogen), fetuin (300 µg/ml: Sigma, Munich, Germany), transferrin (2 µg/ml; Sigma), pantothenate (17 µM; Sigma), biotin (1 µM; Sigma), and EGF (0.8 nM; Hoffmann-La Roche, Basel, Switzerland). Differentiation was induced by adding dexamethasone (DEX: 1 µM; Sigma), 3-methyl-isobutyl-1-methylxanthine (MIX; 0.5 mM; Sigma), and bovine insulin (5 µg/ml; Invitrogen). Four days after confluence (d4), cells were kept in SF medium, containing bovine insulin (5 µg/ml) until differentiation was completed. At various time points of the differentiation procedure, beginning with day 0 (day of confluence) and day 2 (hormone addition; for example, dexamethasone and 3-Isobutyl-1-methylxanthine), up to 10 days of differentiation, suitable aliquots of cells were taken every two days,

RNA was isolated from mouse tissues or cell culture cells using Trizol Reagent (for example, from Invitrogen, Karlsruhe, Germany) and further purified with the RNeasy Kit (for example, from Qiagen, Germany) in combination with an DNase-treatment according to the instructions of the manufacturers and as known to those skilled in the art. Total RNA was reverse transcribed (preferably using Superscript II RNaseH⁻ Reverse Transcriptase, from Invitrogen, Karlsruhe, Germany) and subjected to Taqman analysis preferably using the Taqman 2xPCR Master Mix (from Applied Biosystems, Weiterstadt, Germany; the Mix contains according to the Manufacturer for example AmpliTaq Gold DNA Polymerase, AmpErase UNG, dNTPs with dUTP, passive reference Rox and optimized buffer components) on a GeneAmp 5700 Sequence Detection System (from Applied Biosystems, Weiterstadt, Germany).

The following prime/probe pairs were used for the TaqMan analysis (GenBank Accession Number NM_178644 (mouse) for the mouse SF02 sequence):
Mouse SF02 forward primer (Seq ID NO:1): 5'- CGG ACA GCA TCA GCC TTG A -3'; mouse SF02 reverse primer (Seq ID NO:2): 5'- CCG CGA TGA AGG AGA TGA GA -3'; mouse SF02 Taqman probe (Seq ID NO:3): (5/6-FAM)- CTG CGC AAA CCC GAC GGC A -(5/6-TAMRA).

The following prime/probe pairs were used for the TaqMan analysis (GenBank Accession Number NM_016697 (mouse) for the mouse SF03 sequence):
Mouse SF03 forward primer (Seq ID NO:4): 5'- GTT GTT CGC CAT GCC AAG A -3'; mouse SF03 reverse primer (Seq ID NO:5): 5'- CAA AAG CTT GTG GAG TCA GGC T -3'; mouse SF03 Taqman probe (Seq ID NO:6): (5/6-FAM)- ACA CCA ACG CCA TGT TCA AGA ATA ACT ACC C -(5/6-TAMRA).

The following prime/probe pairs were used for the TaqMan analysis (GenBank Accession Number NM_007443 (mouse) for the mouse SF04 sequence):
Mouse SF04 forward primer (Seq ID NO:7): 5'- GGT ACA ACC TGG CGG TGG -3'; mouse SF04 reverse primer (Seq ID NO:8): 5'- GCT CAC GCT CAT CTT GTC CTT AA -3'; mouse SF04 Taqman probe (Seq ID NO:9): (5/6-FAM)- TGC CCG TGG CTG AGC CGC -(5/6-TAMRA).

The function of the mammalian SF02, SF03, and SF04 in metabolism was further validated by analyzing the expression of the transcripts in different tissues.

In Fig. 2, 4, and 6, the relative RNA-expression is shown on the Y-axis. In Fig. 2, 4, and 6, the tissues tested are given on the X-axis, "WAT" refers to white adipose tissue. In Fig. 2, 4, and 6, the panel of the wild type mice tissues comprises liver, pancreas, muscle, small intestine, WAT, hypothalamus, and heart, and the panel of the control diet-mice tissues comprises liver, muscle, small intestine, WAT, brain, and heart.

The function of the SF02, SF03, and SF04 proteins in metabolism was further validated by analyzing the expression of the transcripts in different tissues. Mouse models of insulin resistance and/or diabetes were used, such as mice carrying gene knockouts In the leptin pathway (for example, ob/ob (leptin) or db/db (leptin receptor/ligand) mice) to study the expression of the proteins. Such mice develop typical symptoms of diabetes, show hepatic lipid accumulation and frequently have increased plasma lipid levels (see Bruning J.C. et al, (1998) Mol. Cell. 2: 559-569).

Expression of the mRNAs encoding the described proteins was also examined in susceptible wild type mice (for example, C57BI/6) that show symptoms of diabetes, lipid accumulation, and high plasma lipid levels, if fed a high fat diet. Expression profiling studies confirm the particular relevance of the proteins as regulators of energy metabolism in mammals.

Expression, profiling studies confirm the particular relevance of SF02, SF03, and SF04 as regulators of energy metabolism in mammals.

Taqman analysis revealed that SF02 is expressed in several mammalian tissues, showing highest level of expression in liver, and higher levels In further tissues, e.g. WAT, small intestine, heart, brain, muscle. Furthermore SF02 is expressed on lower but still robust levels in the hypothalamus and pancreas as depicted in Fig. 2A. We found, for example, that the expression of SF02 is up-regulated in the muscle of ob/ob mice compared to wild-type mice (see Fig. 2B). In wild type mice fed a high fat diet, the expression of SF02 is up-regulated in muscle and down-regulated in WAT compared to mice fed a control diet. The high expression of SF02 in metabolic active tissues (e.g. liver and WAT) and the regulation of gene expression in different mouse models used to study metabolic disorders as described above suggests that it plays a role in the regulation of energy homeostasis.

Taqman analysis revealed that SF03 is expressed in several mammalian tissues, showing highest level of expression in WAT and hypothalamus, and higher levels in further tissues, e.g. brain and heart. Furthermore SF03 is expressed on lower but still robust levels in the pancreas, muscle, small intestine, and liver as depicted in Fig. 4A. We found, for example, that the expression of SF03 is up-regulated in muscle, liver, and small intestine and down-regulated in the pancreas of ob/ob mice compared to wild-type mice (see Fig. 4B). In wild type mice fed a high fat diet, the expression of SF03 is not regulated. The high expression of SF03 in WAT and in the hypothalamus, which is known to be involved in appetite control, as well as he regulation of gene expression in the mouse model for the metabolic syndrome as described above, suggests that it plays a role in the regulation of energy homeostasis.

Taqman analysis revealed that SF04 is expressed in several mammalian tissues, showing highest level of expression in liver (Fig. 6A), and lower but still robust levels in further tissues, e.g., small intestine, heart, muscle, pancreas, WAT, brain, but not in the hypothalamus, as shown in Fig. 6B. We found, for example, that the expression of SF04 is strongly up-regulated in the hypothalamus and down-regulated in the heart, muscle, and WAT of ob/ob mice compared to wild-type mice (see Fig. 6C). In wild type mice fed a high fat diet, the expression of SF04 is up-regulated in the WAT and down-regulated in muscle, heart, and brain when compared to control diet mice. The high expression levels of SF04 in liver suggest that it plays an essential role in metabolism. The regulation of gene expression in different mouse models used to study metabolic disorders as described above suggests that it also plays a role in the regulation of energy homeostasis.

### Example 4: Analysis of the differential expression of transcripts of the described proteins in human tissues

RNA preparation from human primary adipose tissues was done as described In Example 3. The target preparation, hybridization, and scanning was performed as described in the manufactures manual (see Affymetrix Technical Manual, 2002, obtained from Affmetrix, Santa Clara, USA).

In Fig. 3, 5, and 9, the Y-axis represents fluorescence intensity and the X-axis represents the time axis. "d0" refers to day 0 (start of the experiment), "d12" refers to day 12 of adipocyte differentiation.

The expression analysis (using Affymetrix GeneChips) of the genes using primary human abdominal adipocycte differentiation clearly shows differential expression of the human SF02. SF03, and SF13 genes in adipocytes. Several independent experiments where done. The experiments show that the SF02 and SF03 transcripts are most abundant at day 12 compared to day 0 during differentiation (see Fig. 3 and 5) and that the SF13 transcript is most abundant at day 0 compared to day 12 during differentiation (see Fig. 9). Thus, the SF02 and SF03 proteins have to be increased, and the SF13 proteins have to be decreased in order for the preadipocyctes to differentiate into mature adipocyctes. The SF02 and SF03 proteins in preadipocyctes have the potential to enhance adipose differentiation, and the SF13 protein in preadipocyctes has the potential to inhibit adipose differentiation. Therefore, the SF02, SF03, and SF13 proteins play an essential role in the regulation of human metabolism, in particular in the regulation of adipogenesis and thus it might be an essential role in pancreatic diseases (e.g. diabetes), obesity, and/or metabolic syndrome.

### Example 5: In situ hybridisations

Whole-mount and sectional in situ hybridizations were performed according to standard protocols as known to those skilled in the art and as described previously (for example, Pelton, R.W. et al., (1990) Development 110,609-620; Belo, J. A. et al., (1997) Mech. Dev. 68. 45-57).

The nucleic add sequence encoding the mouse SF01 protein is expressed in the ventral panceas (see Fig. 1). The nucleic acid sequences encoding the mouse SF05 (see Fig. 7) and mouse SF06 (see Fig. 8) proteins are expressed in the pancreas.

Although the invention has been described in connection with specific preferred embodiments, it should be understood that the invention as claimed should not be unduly limited to such specific embodiments. Indeed, various modifications of the described modes for carrying out the invention which are obvious to those skilled In molecular biology or related fields are intended to be within the scope of the following claims.

## Claims

1. Use of a composition comprising a SF06 protein as **characterized by** SEQ ID Nos:30 or 32 and/or a nucleic acid molecule encoding a SF06 protein as **characterized by** SEQ ID Nos:30 or 32, the nucleic acid molecule being preferably a DNA molecule, more preferably a cDNA or a genomic DNA, and optionally pharmaceutically acceptable carriers, diluents, and/or additives for the preparation of an agent for detecting diseases or dysfunctions selected from the group consisting of diabetes, obesity, and/or metabolic syndrome.

2. Use of a composition comprising an SF06 protein as **characterized by** SEQ ID Nos:30 or 32 and/or a nucleic acid molecule encoding an SF06 protein as **characterized by** SEQ ID Nos:30 or 32, that nucleic acid molecule being preferably a DNA molecule, more preferably a cDNA or genomic DNA, and optionally pharmaceutically acceptable carriers, diluents, and/or additives for the preparation of a medicament for the treatment, alleviation and/or prevention of diseases or dysfunctions selected from the group consisting of diabetes, obesity and/or metabolic syndrome.

3. Use according to claim 1 or 2, wherein the nucleic acid molecule is a mammalian SF06 nucleic acid as **characterized by** SEQ ID Nos:30 or 32, particularly encoding the human SF06 polypeptide as **characterized by** SEQ ID No:32 and/or a nucleic acid molecule, which is complementary thereto.

4. Use according to any one of claims 1-3, wherein said nucleic acid molecule is selected from the group consisting of
(a) a nucleic acid molecule encoding a polypeptide, the polypeptide having a sequence as shown in SEQ ID NO: 30 or SEQ ID NO: 32, or an isoform of the polypeptide according to SEQ ID NO:30 or SEQ ID NO: 32;
(b) a nucleic acid molecule which comprises or is the nucleic acid molecule according to SEQ ID NO: 30 or SEQ ID NO: 32;
(c) a nucleic acid molecule being degenerate with as a result of the genetic code to the nucleic acid sequences as defined in (a) or (b),
(d) a nucleic acid molecule that hybridizes at 50°C in a solution containing 1 x SSC and 0.1% SDS to a nucleic acid molecule as defined in claim 3 or as defined in (a) to (c) and/or a nucleic acid molecule which is complementary thereto;
(e) a nucleic acid molecule that encodes a polypeptide which is at least 85%, preferably at least 90%, more preferably at least 95%, more preferably at least 98% and up to 99,6% identical to the human SF06 as defined in claim 3 or to a polypeptide as defined in (a).

5. Use according to any one of claims 1-4, wherein said nucleic acid molecule is a recombinant nucleic acid molecule and/or a vector, particularly an expression vector and/or a hybridization probe, a primer or an anti-sense oligonucleotide.

6. Use according to any one of claims 1-5, wherein the polypeptide is a recombinant polypeptide and/or a fusion polypeptide.

7. Use according to any one of claims 1-6 for monotherapy or combination therapy together with at least one further pharmaceutical agent, the at least one further pharmaceutical agent being selected from the group consisting of 2-amino-1,3-propanediol derivatives, 2-amino-2[2-(4-octylphenyl)ethyl] propane-1,3-diol hydrochloride, 40-O(2-hydroxyethyl)-rapamycin, SDZ-RAD, Everolimus, 6-(3-dimethyl-aminopropionyl) forskolin, 6-mercaptopurine (6-MP), A-420983, ABX-CBL (CBL-1), Alefacept, Antisense ICAM-1 inhibitor (ISIS 2302), Enlimomab, BIRR1, Alicaforsen, Antithymocyte immunoglobulin (ATGAM), Azathioprine, Baohuoside-1, basiliximab, BMS-279700, BTI-322, Cladnbine, CP-690550, Cyclophosphamide (CTX), Cyclosporine (cyclosporin A, cyclosporin), Daclizumab, HAT (Humanized-Anti-Tac), SMART anti-Tac, anti-CD25, humanized anti-IL2-receptor, Dexamethasone (Decadron, Dexone, Dexasone), DIAPEP-277, Dipeptide Boronic Acid (DPBA), Docosahexaenoic acid (DHA), efalizumab, Efomycine M, FTY720 (oral myriocin derivative), Glatiramer acetate (co-polymer-1), Glial Fibrillary acidic protein (GFAP), Gusperimus (15-deoxyspergualin), HLA-B2702 peptide, hu1124 (anti-CD11a), hOKT31y (Ala-Ala), Infliximab, Interferon, ISAtx247, isotretinoin, L-683,742, Leflunomide (ARAVA), Medi-500 (T10B9), Medi-507, Methotrexate, Mitoxantrone, mycophenolate mofetil, OKT4A, Muromonab-CD3, Prednisolone, Psora-4, Rifampicin, Rituximab, S100β, Sirolimus, Rapamycin, Tacrolimus (Prograf; FK-506), or Triptofide.

8. Method of identifying a (poly)peptide involved in the regulation of energy homeostasis and/or metabolism in a mammal comprising the steps of
(a) contacting a collection of (poly)peptides with a SF06 polypeptide as **characterized by** SEQ ID Nos:30 or 32 or a SF06 protein encoded by a nucleic acid selected from the group consisting of
(i) a nucleic acid molecule encoding a polypeptide, the polypeptide having a sequence as shown in SEQ ID NO: 30 or SEQ ID NO: 32, or an isoform of the polypeptide according to SEQ ID NO:30 or SEQ ID NO: 32;
(ii) a nucleic acid molecule which comprises or is the nucleic acid molecule according to SEQ ID NO: 30 or SEQ ID NO: 32;
(iii) a nucleic acid molecule being degenerate with as a result of the genetic code to the nucleic acid sequences as defined in (i) or (ii),
(iv) a nucleic add molecule that hybridizes at 50°C in a solution containing 1 x SSC and 0.1% SDS to a mammalian SF06 nucleic acid as **characterized by** SEQ ID Nos 30 or 32 or as defined in (i) to (iii) and/or a nucleic add molecule which is complementary thereto;
(v) a nucleic add molecule that encodes a polypeptide which is at least 85%, preferably at least 90%, more preferably at least 95%, more preferably at least 98% and up to 99,6% identical to the human SF06 as **characterized by** SEQ ID No. 32 or to a polypeptide as defined in (i) under conditions that allow binding of said (poly)peptides;
(b) removing (poly)peptides which do not bind and
(c) identifying (poly)peptides that bind to said SF06 polypeptide as **characterized by** SEQ ID Nos:30 or 32 or to said SF06 protein encoded by a nucleic acid selected from the group consisting of
(i) a nucleic acid molecule encoding a polypeptide, the polypeptide having a sequence as shown in SEQ ID NO: 30 or SEQ ID NO: 32, or an isoform of the polypeptide according to SEQ ID NO:30 or SEQ ID NO: 32;
(ii) a nucleic acid molecule which comprises or is the nucleic acid molecule according to SEQ ID NO: 30 or SEQ ID NO: 32;
(iii) a nucleic acid molecule being degenerate with as a result of the genetic code to the nucleic acid sequences as defined in (i) or (ii),
(iv) a nucleic acid molecule that hybridizes at 50°C in a solution containing 1 x SSC and 0.1% SDS to a mammalian SF06 nucleic add as **characterized by** SEQ ID Nos 30 or 32 or as defined in (i) to (iii) and/or a nucleic acid molecule which is complementary thereto;
(v) a nucleic acid molecule that encodes a polypeptide which is at least 85%, preferably at least 90%, more preferably at least 95%, more preferably at least 98% and up to 99,6% identical to the human SF06 as **characterized by** SEQ ID No. 32 or to a polypeptide as defined in (i) under conditions that allow binding of said (poly)peptides;

9. Method for screening for an agent, which effects/modulates the activity of a SF06 polypeptide as **characterized by** SEQ ID Nos:30 or 32, comprising the steps of
(a) incubating a mixture comprising
(aa) a SF06 polypeptide as **characterized by** SEQ ID Nos:30 or 32; and
(ab) a candidate agent
under conditions whereby said SF06 polypeptide or fragment thereof exhibits a reference activity,
(b) detecting the activity of said SF06 polypeptide to determine an activity for the agent; and
(c) determining a difference between activity for the agent and reference activity.

## Patentansprüche

1. Verwendung einer Zusammensetzung, umfassend ein SF06 Protein wie durch SEQ ID Nr. 30 oder 32 **gekennzeichnet** und/oder ein Nukleinsäuremolekül, das ein SF06 Protein kodiert wie durch SEQ ID Nr. 30 oder 32 **gekennzeichnet**, wobei das Nukleinsäuremolekül vorzugsweise ein DNS-Molekül ist, weiter bevorzugt eine cDNS oder eine genomische DNS, und wahlweise pharmazeutisch annehmbare Träger, Verdünner und/oder Additive, zur Herstellung eines Mittels zum Nachweis von Erkrankungen oder Dysfunktionen, die ausgewählt sind aus der Gruppe bestehend aus Diabetes, Obesitas und/oder metabolischem Syndrom.

2. Verwendung einer Zusammensetzung, umfassend ein SF06 Protein wie durch SEQ ID Nr. 30 oder 32 **gekennzeichnet** und/oder ein Nukleinsäuremolekül, das ein SF06 Protein kodiert wie durch SEQ ID Nr. 30 oder 32 **gekennzeichnet**, wobei das Nukleinsäuremolekül vorzugsweise ein DNS-Molekül ist, weiter bevorzugt eine cDNS oder eine genomische DNS, und wahlweise pharmazeutisch annehmbare Träger, Verdünner und/oder Additive, zur Herstellung eines Mittels für die Behandlung, Linderung und/oder Vorbeugung von Erkrankungen oder Dysfunktionen, die ausgewählt sind aus der Gruppe bestehend aus Diabetes, Obesitas und/oder metabolischem Syndrom.

3. Verwendung nach Anspruch 1 oder 2, wobei das Nukleinsäuremolekül eine Säuger-SF06-Nukleinsäure ist, wie durch SEQ ID Nr. 30 oder 32 **gekennzeichnet**, welche insbesondere das humane SF06 Polypeptid kodiert wie durch SEQ ID Nr. 32 **gekennzeichnet** und/oder ein Nukleinsäuremolekül, welches dazu komplementär ist.

4. Verwendung nach einem der Ansprüche 1 bis 3, wobei das Nukleinsäuremolekül ausgewählt ist aus der Gruppe bestehend aus:
(a) einem Nukleinsäuremolekül welches ein Polypeptid kodiert, wobei das Polypeptid die in SEQ ID Nr. 30 oder 32 gezeigte Sequenz aufweist, oder eine Isoform des Polypeptids gemäß SEQ ID Nr. 30 oder 32;
(b) einem Nukleinsäuremolekül welches das Nukleinsäuremolekül gemäß SEQ ID Nr. 30 oder 32 ist oder dieses umfasst;
(c) einem Nukleinsäuremolekül das aufgrund des genetischen Codes degeneriert ist zu den in (a) oder (b) definierten Nukleinsäuresequenzen,
(d) einem Nukleinsäuremolekül das bei 50°C in einer Lösung die 1 x SSC und 0.1 % SDS enthält an ein Nukleinsäuremolekül wie in Anspruch 3 oder in (a) bis (c) definiert hybridisiert und/oder ein Nukleinsäuremolekül welches dazu komplementär ist;
(e) einem Nukleinsäuremolekül welches ein Polypeptid kodiert, das zu wenigstens 85%, vorzugsweise wenigstens 90%, weiter bevorzugt wenigstens 95%, weiter bevorzugt wenigstens 98% und bis zu 99,6% identisch ist zu dem humanen SF06 wie in Anspruch 3 definiert oder zu einem Polypeptid wie in (a) definiert.

5. Verwendung nach einem der Ansprüche 1-4, wobei das Nukleinsäuremolekül ein rekombinantes Nukleinsäuremolekül und/oder ein Vektor ist, insbesondere ein Expressionsvektor und/oder eine Hybridisierungssonde, ein Primer oder ein antisense-Oligonukleotid.

6. Verwendung nach einem der Ansprüche 1-5, wobei das Polypeptid ein rekombinantes Polypeptid und/oder ein Fusionspolypeptid ist.

7. Verwendung nach einem der Ansprüche 1-6 für die Monotherapie oder Kombinationstherapie zusammen mit wenigstens einem weiteren pharmazeutischen Wirkstoff, wobei der wenigstens eine pharmazeutische Wirkstoff ausgewählt ist aus der Gruppe bestehend aus 2-Amino-1,3-propandiol-Derivaten, 2-Amino-2[2-(4-octylphenyl)ethyl]propan-1,3-diol-Hydrochlorid, 40-O(2-Hydroxyethyl)rapamycin, SDZ-RAD, Everolimus, 6-(3-Dimethyl-aminopropionyl)forskolin, 6-Mercaptopurin (6-MP), A-420983, ABX-CBL (CBL-1), Alefacept, Antisense ICAM-1 Inhibitor (ISIS 2302), Enlimomab, BIRR1, Alicaforsen, Antithymocyt-Immunglobulin (ATGAM), Azathioprin, Baohuosid-1, Basiliximab, BMS-279700, BTI-322, Cladribin, CP-690550, Cyklophosphamid (CTX), Cyklosporin, Daclizumab, HAT (Humanisierter Anti-Tac), SMART anti-Tac, anti-CD25, humanisierter anti-IL2-Rezeptor, Dexamethasone (Decadron, Dexon, Dexason), DIAPEP-277, Dipeptid-Borsäure (DPBA), Docosahexaensäure (DHA), Efalizumab, Efomycin M, FTY720 (orales Myriocin-Derivat), Glatirameracetat (Copolymer-1), saures Gliafaserprotein (GFAP), Gusperimus (15-Desoxyspergualin), HLA-B2702 Peptid, hu1124 (anti-CD11a), hOKT31γ (Ala-Ala), Infliximab, Interferon, ISAtx247, Isotretinoin, L-683,742, Leflunomid (ARAVA), Medi-500 (T10B9), Medi-507, Methotrexat, Mitoxantron, Mykophenolat-Mofetil, OKT4A , Muromonab-CD3, Prednisolon, Psora-4, Rifampicin, Rituximab, S100ß, Sirolimus, Rapamycin, Tacrolimus (Prograf; FK-506) oderTriptolid.

8. Verfahren zur Identifizierung eines (Poly)peptids das an der Regulierung der Energiehomöostase und/oder dem Metabolismus in einem Säuger beteiligt ist, umfassend die Schritte:
(a) Inkontaktbringen einer Sammlung von (Poly)peptiden mit einem SF06-Polypeptid wie durch SEQ ID Nr. 30 oder 32 **gekennzeichnet** oder einem SF06 Protein welches von einer Nukleinsäure kodiert wird, die ausgewählt ist aus der Gruppe bestehend aus
(i) einem Nukleinsäuremolekül welches ein Polypeptid kodiert, wobei das Polypeptid eine in SEQ ID Nr. 30 oder SEQ ID Nr. 32 gezeigte Sequenz aufweist, oder eine Isoform des Polypeptids gemäß SEQ ID Nr. 30 oder 32;
(ii) einem Nukleinsäuremolekül welches das Nukleinsäuremolekül gemäß SEQ ID Nr. 30 oder SEQ ID Nr. 32 ist oder dieses umfasst;
(iii) einem Nukleinsäuremolekül das aufgrund des genetischen Codes degeneriert ist zu den in (i) oder (ii) definierten Nukleinsäuresequenzen,
(iv) einem Nukleinsäuremolekül das bei 50°C in einer Lösung die 1 x SSC und 0.1% SDS enthält an eine Säuger-SF06-Nukleinsäure wie durch SEQ ID Nr. 30 oder 32 **gekennzeichnet** oder wie in (i) bis (iii) definiert hybridisiert und/oder einem Nukleinsäuremolekül welches dazu komplementär ist;
(v) einem Nukleinsäuremolekül das ein Polypeptid kodiert, welches zu wenigstens 85%, vorzugsweise wenigstens 90%, weiter bevorzugt wenigstens 95%, weiter bevorzugt wenigstens 98% und bis zu 99,6% identisch ist zu dem humanen SF06 wie durch SEQ ID Nr. 32 **gekennzeichnet** oder zu einem in (i) definierten Polypeptid,
unter Bedingungen welche die Bindung der (Poly)peptide erlauben;
(b) Entfernen von (Poly)peptiden, welche nicht binden und
(c) Identifizieren von (Poly)peptiden, welche an das durch SEQ ID Nr. 30 oder 32 **gekennzeichnet**e SF06 Polypeptid oder an das SF06 Protein binden, welches von einer Nukleinsäure kodiert wird, die ausgewählt ist aus der Gruppe bestehend aus
(i) einem Nukleinsäuremolekül welches ein Polypeptid kodiert, wobei das Polypeptid eine in SEQ ID Nr. 30 oder SEQ ID Nr. 32 gezeigte Sequenz aufweist, oder eine Isoform des Polypeptids gemäß SEQ ID Nr. 30 oder SEQ ID Nr. 32;
(ii) einem Nukleinsäuremolekül welches das Nukleinsäuremolekül gemäß SEQ ID Nr. 30 oder SEQ ID Nr. 32 ist oder dieses umfasst;
(iii) einem Nukleinsäuremolekül das aufgrund des genetischen Codes degeneriert ist zu den in (i) oder (ii) definierten Nukleinsäuresequenzen,
(iv) einem Nukleinsäuremolekül das bei 50°C in einer Lösung die 1 x SSC und 0.1 % SDS enthält an eine Säuger-SF06-Nukleinsäure wie durch SEQ ID Nr. 30 oder 32 **gekennzeichnet** oder wie in (i) bis (iii) definiert hybridisiert und/oder einem Nukleinsäuremolekül welches dazu komplementär ist;
(v) einem Nukleinsäuremolekül das ein Polypeptid kodiert, welches zu wenigstens 85%, vorzugsweise wenigstens 90%, weiter bevorzugt wenigstens 95%, weiter bevorzugt wenigstens 98% und bis zu 99,6% identisch ist zu dem humanen SF06 wie durch SEQ ID Nr. 32 **gekennzeichnet** oder zu einem in (i) definierten Polypeptid,
unter Bedingungen die eine Bindung der (Poly)peptide erlauben.

9. Verfahren zum Screening nach einem Wirkstoff, welcher die Aktivität eines SF06-Polypeptids wie durch SEQ ID Nr. 30 oder 32 **gekennzeichnet** hervorruft/moduliert, umfassend die Schritte:
(a) Inkubieren eines Gemischs umfassend
(aa) ein SF06-Polypeptid wie durch SEQ ID Nr. 30 oder 32 **gekennzeichnet**; und
(ab) einen Wirkstoffkandidaten
unter Bedingungen, bei denen das SF06-Polypeptid oder Fragment davon eine Referenzaktivität aufweist,
(b) Detektieren der Aktivität des SF06-Polypeptids um eine Aktivität des Wirkstoffs zu bestimmen und
(c) Bestimmen eines Unterschiedes zwischen der Aktivität für den Wirkstoff und der Referenzaktivität.

## Revendications

1. Utilisation d'une composition comprenant une protéine SF06 comme **caractérisée par** SEQ ID No : 30 ou 32 et/ou une molécule d'acide nucléique codant une protéine SF06 comme **caractérisée par** SEQ ID No : 30 ou 32, la molécule d'acide nucléique étant de préférence une molécule d'ADN, de préférence encore un ADNc ou un ADN génomique, et éventuellement des vecteurs, diluants et/ou additifs pharmaceutiquement acceptables pour la préparation d'un agent pour détecter des maladies ou des dysfonctionnements choisis dans le groupe consistant en le diabète, l'obésité et/ou le syndrome métabolique.

2. Utilisation d'une composition comprenant une protéine SF06 comme **caractérisée par** SEQ ID No : 30 ou 32 et/ou une molécule d'acide nucléique codant une protéine SF06 comme **caractérisée par** SEQ ID No : 30 ou 32, cette molécule d'acide nucléique étant de préférence une molécule d'ADN, de préférence encore un ADNc ou ADN génomique, et éventuellement des vecteurs, diluants et/ou additifs pharmaceutiquement acceptables pour la préparation d'un médicament pour le traitement, l'atténuation et/ou la prévention de maladies ou de dysfonctionnements choisis dans le groupe consistant en le diabète, l'obésité et/ou le syndrome métabolique.

3. Utilisation selon la revendication 1 ou 2, où la molécule d'acide nucléique est un acide nucléique SF06 de mammifère comme **caractérisé par** SEQ ID No : 30 ou 32, codant en particulier le polypeptide SF06 humain comme **caractérisé par** SEQ ID No : 32 et/ou une molécule nucléique, qui est complémentaire de celui-ci.

4. Utilisation selon l'une quelconque des revendications 1-3, où ladite molécule d'acide nucléique est choisie dans le groupe consistant en
(a) une molécule d'acide nucléique codant un polypeptide, le polypeptide ayant une séquence comme illustré dans SEQ ID NO : 30 ou SEQ ID NO : 32, ou une isoforme du polypeptide selon SEQ ID NO : 30 ou SEQ ID NO : 32 ;
(b) une molécule d'acide nucléique qui comprend ou est la molécule d'acide nucléique selon SEQ ID NO : 30 ou SEQ ID NO : 32 ;
(c) une molécule d'acide nucléique qui est dégénérée par suite du code génétique par rapport aux séquences d'acide nucléique telles que définies en (a) ou (b),
(d) une molécule d'acide nucléique qui s'hybride à 50°C dans une solution contenant 1 x SSC x et 0,1 % de SDS à une molécule d'acide nucléique, telle que définie dans la revendication 3 ou telle que définie en (a) à (c) et/ou une molécule d'acide nucléique qui est complémentaire de celle-ci ;
(e) une molécule d'acide nucléique qui code un polypeptide qui est identique à au moins 85 %, préférence au moins 90 %, de préférence encore au moins 95 %, de préférence encore au moins 98 % et jusqu'à 99,6 % au SF06 humain tel que défini dans la revendication 3 ou à un polypeptide tel que défini en (a).

5. Utilisation selon l'une quelconque des revendications 1-4, où ladite molécule d'acide nucléique est une molécule d'acide nucléique recombinant et/ou un vecteur, particulièrement un vecteur d'expression et/ou une sonde d'hybridation, une amorce ou un oligonucléotide anti-sens.

6. Utilisation selon l'une quelconque des revendications 1-5, où le polypeptide est un polypeptide recombinant et/ou un polypeptide de fusion.

7. Utilisation selon l'une quelconque des revendications 1-6 pour la monothérapie ou la thérapie d'association avec au moins un autre agent pharmaceutique, le au moins un autre agent pharmaceutique étant choisi dans le groupe consistant en les dérivés du 2-amino-1,3-propanediol, le chlorhydrate de 2-amino-2[2-(4-octylphényl)éthyl]propane-1,3-diol, la 40-O(2-hydroxyéthyl)-rapamycine, SDZ-RAD, Everolimus, la 6-(3-diméthyl-aminopropionyl) forskoline, la 6-mercaptopurine (6-MP), A-420983, ABX-CBL (CBL-1), Alefacept, l'inhibiteur d'ICAM-1 antisens (ISIS 2302), Enlimomab, BIRR1, Alicaforsen, une immunoglobuline antihymocyte (ATGAM), l'azathioprine, Baohuoside-1, le basiliximab, BMS-279700, BTI-322, la cladribine, CP-690550, le cyclophosphamide (CTX), la cyclosporine (cyclosporine A, cyclosporine), Daclizumab, HAT (anti-Tac humanisé), anti-Tac SMART, anti-CD25, anti-récepteur d'IL2 humanisé, la dexaméthasone (Decadron, Dexone, Dexasone), DIAPEP-277, l'acide dipeptide boronique (DPBA), l'acide docosahexaénoïque (DHA), efalizumab, l'efomycine M, FTY720 (dérivé de myriocine oral), l'acétate de Glatiramer (co-polymère-1), la protéine acide fibrillaire gliale (GFAP), Gusperimus (15-désoxyspergualine), le peptide HLA-B2702, hu1124 (anti-CD11a), hOKT31y (Ala-Ala), Infliximab, l'interféron, ISAtx247, l'isotrétinoïne, L-683,742, le leflunomide (ARAVA), Medi-500 (T10B9), Medi-507, le méthotrexate, la mitoxantrone, le mycophénolate mofetil, OKT4A, Muromonab-CD3, la prednisolone, Psora-4, la rifampicine, Rituximab, S100β, le sirolimus, la rapamycine, le tacrolimus (Prograf; FK-506) ou le triptolide.

8. Procédé d'identification d'un (poly)peptide impliqué dans la régulation de l'homéostasie énergétique et/ou du métabolisme chez un mammifère comprenant les étapes de
(a) mise en contact d'une collection de (poly)peptides avec un polypeptide SF06 comme **caractérisé par** SEQ ID No : 30 ou 32 ou une protéine SF06 codée par un acide nucléique choisi dans le groupe consistant en
(i) une molécule d'acide nucléique codant un polypeptide, le polypeptide ayant une séquence comme illustré dans SEQ ID NO : 30 ou SEQ ID NO : 32, ou une isoforme du polypeptide selon SEQ ID NO : 30 ou SEQ ID NO : 32 ;
(ii) une molécule d'acide nucléique qui comprend ou est la molécule d'acide nucléique selon SEQ ID NO : 30 ou SEQ ID NO : 32 ;
(iii) une molécule d'acide nucléique qui est dégénérée du fait du code génétique par rapport aux séquences d'acide nucléique telles que définies en (i) ou (ii),
(iv) une molécule d'acide nucléique qui s'hybride à 50°C dans une solution contenant 1 x SSC et 0,1 % de SDS à un acide nucléique SF06 de mammifère comme **caractérisé par** SEQ ID NO : 30 ou 32 ou comme défini en (i) à (iii) et/ou une molécule d'acide nucléique qui est complémentaire de celle-ci ;
(v) une molécule d'acide nucléique qui code un polypeptide qui est identique à au moins 85 %, de préférence au moins 90 %, de préférence encore au moins 95 %, de préférence encore au moins 98 % et jusqu'à 99,6 % au SF06 humain comme **caractérisé par** SEQ ID NO : 32 ou à un polypeptide tel que défini en (i) dans des conditions qui permettent la liaison desdits (poly)peptides ;
(b) retrait des (poly)peptides qui ne lient pas et
(c) identification des (poly)peptides qui se lient audit polypeptide SF06 comme **caractérisé par** SEQ ID No : 30 ou 32 ou à ladite protéine SF06 codée par un acide nucléique choisi dans le groupe consistant en
(i) une molécule d'acide nucléique codant un polypeptide, le polypeptide ayant une séquence comme illustré dans SEQ ID NO : 30 ou SEQ ID NO: 32, ou une isoforme du polypeptide selon SEQ ID NO : 30 ou SEQ ID NO : 32 ;
(ii) une molécule d'acide nucléique qui comprend ou est la molécule d'acide nucléique selon SEQ ID NO : 30 ou SEQ ID NO : 32 ;
(iii) une molécule d'acide nucléique qui est dégénérée du fait du code génétique par rapport aux séquences d'acide nucléique telles que définies en (i) ou (ii),
(iv) une molécule d'acide nucléique qui s'hybride à 50°C dans une solution contenant 1 x SSC et 0,1 % de SDS à un acide nucléique SF06 de mammifère comme **caractérisé par** SEQ ID No 30 ou 32 ou comme défini en (i) à (iii) et/ou une molécule d'acide nucléique qui est complémentaire de celle-ci ;
(v) une molécule d'acide nucléique qui code un polypeptide qui est identique à au moins 85 %, de préférence au moins 90 %, de préférence encore au moins 95 %, de préférence encore au moins 98 % et jusqu'à 99,6 % au SF06 humain comme **caractérisé par** SEQ ID No 32 ou à un polypeptide tel que défini en (i) dans des conditions qui permettent la liaison desdits (poly)peptides ;

9. Procédé de criblage pour un agent, qui réalise/module l'activité d'un polypeptide SF06 comme **caractérisé par** SEQ ID No : 30 ou 32, comprenant les étapes de
(a) incubation d'un mélange comprenant
(aa) un polypeptide SF06 comme **caractérisé par** SEQ ID No : 30 ou 32 ; et
(ab) un agent candidat
dans des conditions dans lesquelles ledit polypeptide SF06 ou fragment de celui-ci présente une activité de référence,
(b) détection de l'activité dudit polypeptide SF06 pour déterminer une activité pour l'agent ; et
(c) détermination de la différence entre l'activité pour l'agent et l'activité de référence.
